# EUROPEAN PATENT SPECIFICATION

(11) **EP 1 724 344 B1**
(45) Date of publication and mention of the grant of the patent: **23.02.2011**
(21) Application number: 05710814.4
(22) Date of filing: 25.02.2005
(51) Int. Cl.: C12N 15/09, C12N 1/21, C12P 13/04

(54) **PROCESS FOR PRODUCING AMINO ACID**
VERFAHREN ZUR HERSTELLUNG VON AMINOSÄUREN
PROCEDE DE PRODUCTION D'ACIDE AMINO

(30) Priority: 27.02.2004 JP 2004053361
(43) Date of publication of application: 22.11.2006
(73) Proprietor: Kyowa Hakko Bio Co., Ltd., Tokyo, 100-8185 (JP)
(72) Inventor: HASHIMOTO, Shin-ichi, Kyowa Hakko Kogyo Co., Ltd., Hofu-shi, Yamaguchi 747-8522 (JP); TERAZONO, Kouichi, Kyowa Hakko Kogyo Co., Ltd., Machida-shi, Tokyo 194-8533 (JP); YASUHARA, Akinori, c/o Kyowa Hakko Kogyo Co., Ltd., Ube-shi, Yamaguchi 755-8501 (JP); MATSUSHITA, Kazunobu, Yamaguchi-shi, Yamaguchi 753-0811 (JP)
(74) Representative: Vossius & Partner
(86) International application number: PCT/JP2005/003694
(87) International publication number: WO 2005/083077

(56) References cited:
- JP-A- 2002 017 361
- GOURDON P ET AL: "METABOLIC ANALYSIS OF GLUTAMATE PRODUCTION BY CORYNEBACTERIUM GLUTAMICUM" METABOLIC ENGINEERING, ACADEMIC PRESS,, US, vol. 1, no. 3, July 1999 (1999-07), pages 224-231, XP001145486 ISSN: 1096-7176
- MATSUSHITA K ET AL: "NADH dehydrogenase of Corynebacterium glutamicum. Purification of an NADH dehydrogenase II homolog able to oxidize NADPH." FEMS MICROBIOLOGY LETTERS 13 NOV 2001, vol. 204, no. 2, 13 November 2001 (2001-11-13), pages 271-276, XP002473462 ISSN: 0378-1097
- BERTSOVA Y V ET AL: "Two NADH:ubiquinone oxidoreductases of Azotobacter vinelandii and their role in the respiratory protection" BIOCHIMICA ET BIOPHYSICA ACTA. BIOENERGETICS, AMSTERDAM, NL, vol. 1363, no. 2, 25 February 1998 (1998-02-25), pages 125-133, XP004338630 ISSN: 0005-2728
- BERTSOVA Y V ET AL: "Noncoupled NADH:Ubiquinone oxidoreductase of Azotobacter vinelandii is required for diazotrophic growth at high oxygen concentrations" JOURNAL OF BACTERIOLOGY, WASHINGTON, DC, US, vol. 183, no. 23, December 2001 (2001-12), pages 6869-6874, XP002324518 ISSN: 0021-9193
- DATABASE GENBANK [Online] 03 December 2000 'Corynebacterium glutamicum ndh gene.', XP002987961 Database accession no. AJ238250
- BOTT M. ET AL.: 'The respiratory chain of corynebacterium glutamicum.' J. BIOTECHNOL. vol. 104, 04 September 2003, pages 129 - 153, XP002288114
- NANTAPONG N. ET AL.: 'Effect of NADH dehydrogenase disruption and over-expression on respiration related metabolism in corybacterium glutamicum KY9714.' APPL. MICROBIOL BIOTECHNOL. vol. 66, December 2004, pages 187 - 193, XP002987962
- MOLENAAR D. ET AL.: 'Functions of the menbrane associated and cytoplasmic malate dehydrogenases in the citric acid cycle of corynebacterium glutamicum.' J. BACTERIOL. vol. 182, December 2000, pages 6884 - 6891, XP002182880
- BJORKLOF K. ET AL.: 'Purification of the 45 kDa, menbrane bound NADH dehydrogenase' FEBS LETT. vol. 467, no. 1, 04 February 2000, pages 105 - 110, XP004260932

## Description

### Technical Field

The present invention relates to a process for producing an amino acid.

### Background Art

In recent years, attempts to improve the productivity of substances such as amino acids, etc. by mutating electron transport systems of microorganisms have been reported.

By way of examples, it has been reported that productivity of an amino acid is improved either by amplifying DNA coding for energy production NADH dehydrogenase or by deleting DNA coding for energy non-production NADH dehydrogenase, of Escherichia coli (Japanese Published Unexamined Patent Application No. 17363/2002).

Further, it has been reported that productivity of riboflavin is improved by amplifying DNA coding for cytochrome bc oxidase having high ratio of energy generation efficiency in Bacillus subtilis (WO03/072785).

With respect to the influence of mutation in electron transport systems on the growth of microorganisms, Molenaar reported that destruction of DNA coding for NADH dehydrogenase of Corynebacterium glutamicum did not raise any influence on growth of the microorganism (Journal of Bacteriology, 182, p6884-6891(2000)).

As described above, it is known that there is a possibility that the mutation in electron transport systems of microorganisms raises influence on productivity of substances by microorganisms. However, it is difficult to predict how the influence is raised.

In addition to conventional methods, further development of methods for improving productivity of substances is demanded.

### Disclosure of the Invention

It is an object of the present invention to provide an industrially advantageous process for producing amino acids The present invention relates to the following (1) to (21).
(1) A process for producing an amino acid, which comprises culturing, in a medium, a microorganism obtainable by introducing a DNA coding for energy non-production NADH dehydrogenase, forming and accumulating an amino acid in a culture, and recovering the amino acid from the culture, wherein the DNA coding for energy non-production NADH dehydrogenase is a DNA having a nucleotide sequence selected from the group consisting of nucleotide sequences represented by SEQ ID NOs: 3, 5, 7, 9, 11, 13 and 15, or a DNA which hybridizes, under stringent conditions, with a DNA having a nucleotide sequence complementary to the nucleotide sequence.
(2) The process according to (1), wherein the DNA coding for energy non-production NADH dehydrogenase is a DNA derived from a microorganism selected from the group consisting of microorganisms belonging to the genus Corynebacterium, Escherichia, Pseudomonas, Azotobacter, Salmonella or Lactobacillus, or a DNA which hybridizes, under stringent conditions, with a DNA having a nucleotide sequence complementary to the nucleotide sequence of the DNA.
(3) The process according to (1), wherein the DNA coding for energy non-production NADH dehydrogenase is a DNA derived from a microorganism selected from the group consisting of microorganisms belonging to the species Corynebacterium glutamicum, Corynebacterium diphtheriae, Escherichia coli, Pseudomonas fluorescens, Azotobacter vinelandii, Salmonella typhimurium or Lactobacillus plantarum, or a DNA which hybridizes, under stringent conditions, with a DNA having a nucleotide sequence complementary to the nucleotide sequence of the DNA.
(4) The process according to (1), wherein the DNA coding for energy non-production NADH dehydrogenase is a DNA coding for energy non-production NADH dehydrogenase possessed by the plasmid pCS-CGndh carried by Escherichia coli DH5α/pCS-CGndh (FERM BP-08633) or a DNA which hybridizes, under stringent conditions, with a DNA having a nucleotide sequence complementary to the nucleotide sequence of the DNA and which encodes a polypeptide having the energy non-production NADH dehydrogenase activity.
(5) The process according to (1), wherein the energy non-production NADH dehydrogenase is a polypeptide having an amino acid sequence selected from the group consisting of the amino acids sequences represented by SEQ ID NOs: 4, 6, 8, 10, 12, 14 and 16, or a polypeptide comprising an amino acid sequence wherein one or more amino acid residues are deleted, substituted or added in the amino acid sequence of the polypeptide and having the energy non-production NADH dehydrogenase activity.
(6) The process according to (1), wherein the energy non-production NADH dehydrogenase is a polypeptide encoded by the DNA coding for energy non-production NADH dehydrogenase possessed by the plasmid pCS-CGndh carried by Escherichia coli DH5α/pCS-CGndh (FERM BP-08633) or a polypeptide comprising an amino acid sequence wherein one or more amino acid residues are deleted, substituted or added in the amino acid sequence of the polypeptide and having the energy non-production NADH dehydrogenase activity.
(7) The process according to any one of (1) to (6), wherein the microorganism into which the DNA coding for energy non-production NADH dehydrogenase is introduced is a microorganism selected from the group consisting of microorganisms belonging to the genus Escherichia, Corynebacterium, Brevibacterium, Arthrobacter, Aureobacterium, Cellulomonas, Clavibacter, Curtobacterium, Microbacterium, Pimerobacter or Bacillus.
(8) The process according to any one of (1) to (6), wherein the microorganism into which the DNA coding for energy non-production NADH dehydrogenase is introduced is a microorganism belonging to the genus Escherichia.
(9) The process according to any one of (1) to (6), wherein the microorganism into which the DNA coding for energy non-production NADH dehydrogenase is introduced is a microorganism belonging to the species Escherichia coli, Corynebacterium glutamicum, or Corynebacterium flavum, Corynebacterium lactofermentum, or Corynebacterium efficasis.
(10) The process according to any one of (1) to (6), wherein the microorganism into which the DNA coding for energy non-production NADH dehydrogenase is introduced is a microorganism belonging to the genus Corynebacterium.
(11) The process according to any one of (1) to (10), wherein the amino acid is an amino acid selected from the group consisting of L-glutamic acid, L-glutamine, L-aspartic acid, L-asparagine, L-lysine, L-methionine, L-threonine, L-arginine, L-proline, L-citrulline, L-valine, L-leucine, L-isoleucine, L-serine, L-cysteine, glycine, L-tryptophan, L-tyrosine, L-phenylalanine and L-histidine.
(12) The process according to any one of (1) to (10), wherein the amino acid is an amino acid selected from the group consisting of L-glutamic acid, L-glutamine and L-lysine.
(13) A microorganism which belongs to the genus Corynebacterium, or the species Corynebacterium glutamicum and is obtainable by introducing a DNA coding for energy non-production NADH dehydrogenase, wherein the DNA coding for energy non-production NADH dehydrogenase is a DNA having a nucleotide sequence selected from the group consisting of the nucleotide sequences represented by SEQ ID NOs: 3, 5, 7, 9, 11 and 15, or a DNA which hybridizes, under stringent conditions, with a DNA having a nucleotide sequence complementary to said sequence.
(14) The microorganism according to (13), wherein the DNA coding for energy non-production NADH dehydrogenase is a DNA derived from a microorganism selected from the group consisting of microorganisms belonging to the genus Corynebacterium, Escherichia, Pseudomonas, Azotobacter, Salmonella or Lactobacillus, or a DNA which hybridizes, under stringent conditions, with a DNA having a nucleotide sequence complementary to the nucleotide sequence of the DNA.
(15) The microorganism according to (13), wherein the DNA coding for energy non-production NADH dehydrogenase is a DNA derived from a microorganism selected from the group consisting of microorganisms belonging to the species Corynebacterium glutamicum, Corynebacterium diphtheriae, Escherichia coli, Pseudomonas fluorescens, Azotobacter vinelandii, Salmonella typhimurium or Lactobacillus plantarum, or a DNA which hybridizes, under stringent conditions, with a DNA having a nucleotide sequence complementary to the nucleotide sequence of the DNA.
(16) The microorganism according to (13), wherein the DNA coding for energy non-production NADH dehydrogenase is a DNA coding for energy non-production NADH dehydrogenase possessed by the plasmid pCS-CGndh carried by Escherichia coli DH5α/pCS-CGndh (FERM BP-08633) or a DNA which hybridizes, under stringent conditions, with a DNA having a nucleotide sequence complementary to the nucleotide sequence of the DNA and which encodes a polypeptide having the energy non-production NADH dehydrogenase activity.
(17) The microorganism according to (13), wherein the energy non-production NADH dehydrogenase is a polypeptide having an amino acid sequence selected from the group consisting of the amino acid sequences represented by SEQ ID NOs: 4, 6, 8, 10, 12, 14 and 16, or a polypeptide comprising an amino acid sequence wherein one or more amino acid residues are deleted, substituted or added in the amino acid sequence of the polypeptide and having the energy non-production NADH dehydrogenase activity.
(18) The microorganism according to (13), wherein the energy non-production NADH dehydrogenase is a polypeptide encoded by a DNA possessed by the plasmid pCS-CGndh carried by Escherichia coli DH5 α/pCS-CGndh (FERM BP-08633) or a polypeptide comprising an amino acid sequence in which one or more amino acid residues are deleted, substituted or added in the amino acid sequence of the polypeptide and having the energy non-production NADH dehydrogenase activity.
(19) Corynebacterium glutamicum ATCC 14752/pCS-CGndh or Corynebacterium glutamicum FERM BP-1069 / pCS-CGndh.
(20) Escherichia coli DH5 α/pCS-CGndh (FERM BP-08633).
(21) The plasmid pCS-CGndh carried by Escherichia coli DH5 α /pCS-CGndh (FERM BP-08633).

### Best Mode for Carrying Out the Invention

Energy non-production NADH dehydrogenase (hereinafter referred to also as NDH polypeptide) used in the present invention may be any polypeptide having an activity of NADH dehydrogenase constituting an NADH dehydrogenase complex, by which the number of proton molecules discharged per electron is zero (hereinafter the activity is referred to as an energy non-production NADH dehydrogenase activity) among NADH dehydrogenase complexes functioning as a proton pump in electron transport systems of aerobic bacteria.

Examples of the NDH polypeptide include known NDH polypeptides derived from microorganisms belonging to the genus Corynebacterium, Escherichia, Pseudomonas, Azotobacter, Salmonella or Lactobacillus, and the like.

Microorganisms belonging to the genus Corynebacterium include microorganisms belonging to Corynebacterium glutamicum or Corynebacterium diphtheriae, and the like.

Microorganisms belonging to the genus Escherichia include microorganisms belonging to Escherichia coli, and the like.

Microorganisms belonging to the genus Pseudomonas include microorganisms belonging to Pseudomonas fluorescens, and the like.

Microorganisms belonging to the genus Azotobacter include microorganisms belonging to Azotobacter vinelandii.

Microorganisms belonging to the genus Salmonella include microorganisms belonging to Salmonella typhimurium.

Microorganisms belonging to the genus Lactobacillus include microorganisms belonging to Lactobacillus plantarum.

The NDH polypeptides derived from these microorganisms include known NDH polypeptides such as a polypeptide derived from microorganisms belonging to the genus Corynebacterium and having an amino acid sequence represented by SEQ ID NO: 4 or 6, a polypeptide derived from microorganisms belonging to the genus Escherichia and having an amino acid sequence represented by SEQ ID NO: 8, a polypeptide derived from microorganisms belonging to the genus Pseudomonas and having an amino acid sequence represented by SEQ ID NO: 10, a polypeptide derived from microorganisms belonging to the genus Azotobacter and having an amino acid sequence represented by SEQ ID NO: 12, a polypeptide derived from microorganisms belonging to the genus Salmonella and having an amino acid sequence represented by SEQ ID NO: 14 and a polypeptide derived from microorganisms belonging to the genus Lactobacillus and having an amino acid sequence represented by SEQ ID NO: 16.

A polypeptide encoded by a DNA (ndh) coding for Corynebacterium glutamicum-derived energy non-production NADH dehydrogenase possessed by plasmid pCS-CGndh carried by Escherichia coli DHSa/pCS-CGndh (FERM BP-08633)(hereinafter abbreviated as NDH polypeptide A) can also be mentioned as an NDH polypeptide.

The NDH polypeptide used in the present invention may be a polypeptide comprising an amino acid sequence in which one or more amino acid residues are deleted, substituted or added in the amino acid sequence of NDH polypeptide A or known NDH polypeptides so long as it has the energy non-production NADH dehydrogenase activity.

The polypeptide comprising the amino acid sequence in which one or more amino acid residues are deleted, substituted or added in the amino acid sequence of NDH polypeptide A or known NDH polypeptides can be obtained by introducing site-specific mutations in the DNA coding for NDH polypeptide A or known NDH polypeptides using a site-specific mutation introducing method described in Molecular Cloning, A Laboratory Manual, Third Edition, Cold Spring Harbor Laboratory Press (2001) (hereinafter abbreviated as Molecular Cloning 3rd ed.), Current Protocols in Molecular Biology, John Wiley & Sons (1987-1997) (hereinafter abbreviated as Current Protocols in Molecular Biology), Nucleic Acids Research, 10, 6487 (1982), Proc. Natl. Acad. Sci. USA, 79, 6409 (1982), Gene, 34, 315 (1985), Nucleic Acids Research, 13, 4431 (1985), Proc. Natl Acad. Sci. USA, 82, 488 (1985) and the like.

The number of amino acid residues to be deleted, substituted or added is not particularly limited. It is the number of amino acid residues which can be deleted, substituted or added by a known method such as the site-specific mutation method. The number is from 1 to several tens, preferably from 1 to 20, more preferably from 1 to 10, further preferably from 1 to 5.

That one or more amino acid residues are deleted, substituted or added in an amino acid sequence of NDH polypeptide A or known NDH polypeptides means that one or more amino acid residues are deleted, substituted or added in any one or more sites of one and the same amino acid sequence. Deletion, substitution or addition may take place simultaneously.

An amino acid to be substituted or added may be of a natural type or a non-natural type.

Examples of a natural-type amino acid include L-alanine, L-asparagine, L-aspartic acid, L-glutamine, L-glutamic acid, glycine., L-histidine, L-isoleucine, L-leucine, L-lysine, L-arginine, L-methionine, L-phenylalanine, L-proline, L-serine, L-threonine, L-tryptophan, L-tyrosine, L-valine, L-cysteine and the like.

Examples of amino acids which can be mutually substituted are listed below. Amino acids which are included in the same group can be mutually substituted.
Group A: leucine, isoleucine, norleucine, valine, norvaline, alanine, 2-aminobutanoic acid, methionine, O-methylserine, t-butylglycine, t-butylalanine and cyclohexylalanine
Group B: aspartic acid, glutamic acid, isoaspartic acid, isoglutamic acid, 2-aminoadipic acid and 2-aminosuberic acid
Group C: asparagine and glutamine
Group D: lysine, arginine, ornithine, 2,4-diaminobutanoic acid and 2,3-diaminopropionic acid
Group E: proline, 3-hydroxyproline and 4-hydroxyproline
Group F: serine, threonine and homoserine
Group G: phenylalanine, tyrosine

For a polypeptide having an amino acid sequence in which one or more amino acid residues are deleted, substituted or added in NDH polypeptide A or NDH polypeptides known to have the energy non-production NADH dehydrogenase activity, it is advisable that the peptide has homology to a polypeptide before deletion, substitution or addition by at least 60%, usually at least 80%, especially at least 95%.

The homology of amino acid sequences or nucleotide sequences can be determined using the BLAST algorithm by Karlin and Altschul [Pro. Natl. Acad. Sci. USA, 90, 5873 (1993)] or FASTA [Methods Enzymol., 183, 63 (1990)]. On the basis of this BLAST algorithm, a program called BLASTN or BLASTX has been developed [J. Mol. Biol., 215, 403 (1990)].

When nucleotide sequences are analyzed with BLASTN based on BLAST, parameters are, for example, score=100 and wordlength=12. When amino acid sequences are analyzed with BLASTX based on BLAST, parameters are, for example, score=50 and wordlength=3. When BLAST and Gapped BLAST programs are used, the default parameters of each program are used. Specific methods of these analyses are known (http://www.ncbi.nlm.nih.gov.).

The energy non-production NADH dehydrogenase activity of the NDH polypeptide can be measured by measuring a decrease in absorbance at 275 nm or 340 nm in a reaction solution containing energy non-production NADH dehydrogenase, ubiquinone-1 and NADH according to, for example, the description in FEMS Microbiology Letters, 204, 271 (2001).

A DNA coding for the NDH polypeptide may be any DNA coding for a polypeptide having the energy non-production NADH dehydrogenase activity.

Examples of the DNA coding for the NDH polypeptide include a DNA coding for NDH polypeptide A or coding for Corynebacterium glutamicum-derived NADH dehydrogenase possessed by plasmid pCS-CGndh carried by Escherichia coli DH5α/pCS-CGndh (FERM BP-08633) and DNAs coding for known NDH polypeptides, such as DNAs cording for polypeptides having amino acid sequences represented by SEQ ID NOs: 4, 6, 8, 10, 12, 14 and 16 and having nucleotide sequences represented by SEQ ID NOs: 3, 5, 7, 9 11, 13 and 15.

The DNA coding for the NDH polypeptide which is used in the present invention may be a DNA which hybridizes, under stringent conditions, with a DNA having a nucleotide sequence complementary to the nucleotide sequence of NDH polypeptide A or other known NDH polypeptides and which encodes a polypeptide having the energy non-production NADH dehydrogenase activity.

The DNA which hybridizes, under stringent conditions, with a DNA having a nucleotide sequence complementary to the nucleotide sequence of NDH polypeptide A or known NDH polypeptides means a DNA which is obtainable by a colony hybridization method, a plaque hybridization method, a southern blot hybridization method or the like using as a probe a part or the whole of the DNA having a nucleotide sequence complementary to a nucleotide sequence of a DNA coding for NDH polypeptide A or other known NDH polypeptides.

Specifically, a DNA can be mentioned which can be identified by performing hybridization at 65°C in the presence of from 0.7 to 1.0 mol/l of sodium chloride using a filter having fixed thereon a colony-derived or plaque-derived DNA and then washing the filter at 65°C using an SSC solution (the SSC solution at a 1-fold concentration comprises 150 mmol/l sodium chloride and 15 mmol/l sodium citrate) at a 0.1- to 2-fold concentration.

Hybridization can be performed by a method described in Molecular Cloning, 3rd ed., Current Protocols in Molecular Biology, DNA Cloning 1: Core Techniques, A Practical Approach, Second Edition, Oxford University (1995) or the like.

Examples of the DNA which hybridizes, under stringent conditions, with the DNA having the nucleotide sequence complementary to the nucleotide sequence of the DNA coding for NDH polypeptide A or other known NDH polypeptides include a DNA having homology of 75% or more, preferably 80% or more, further preferably 95% or more, to the nucleotide sequence of the DNA coding for NDH polypeptide A or other known NDH polypeptides when conducting calculation using the foregoing BLAST or FASTA.

The DNA coding for the NDH polypeptide can be prepared in the following manner from microorganisms which are aerobic bacteria having energy non-production NADH dehydrogenase in electron transport systems. Examples of microorganisms having energy non-production NADH dehydrogenase in electron transport systems include so-called coryneform bacteria which are microorganisms belonging to the genus Corynebacterium (for example, Corynebacterium glutamicum), Brevibacterium, Arthrobacter, Aureobacterium, Cellulomonas, Clavibacter, Curtobacterium, Microbacterium and Pimerobacter, microorganisms belonging to the genus Escherichia (for example, Escherichia coli), microorganisms belonging to the genus Pseudomonas (for example, Pseudomonas fluorescens), microorganisms belonging to the genus Azotobacter (for example, Azotobacter vinelandii), microorganisms belonging to the genus Salmonella (for example, Salmonella typhimurium), microorganisms belonging to the genus Lactobacillus (for example, Lactobacillus plantarum), and the like.

The above microorganisms are cultured by the known method [for example, the method described in Mol. Microbiol. , 20, 833 (1996)]. After the culturing, a chromosomal DNA of the microorganisms is isolated and purified by a known method (for example, the method described in Current Protocols in Molecular Biology).

The desired DNA can be obtained by the PCR method [PCR Protocols, Academic Press (1990)] using a DNA synthesized on the basis of the nucleotide sequence of the DNA coding for known NDH polypeptides as a primer and the chromosomal DNA of the microorganisms isolated and purified as a template.

Examples of the primer include DNAs having nucleotide sequences represented by SEQ ID NOs: 1 and 2, which are designed on the basis of the nucleotide sequence, represented by SEQ ID NO: 3, of ndh of a microorganism belonging to Corynebacterium glutamicum.

The desired DNA can also be prepared by the following method.

A DNA library is produced with the chromosomal DNA isolated and purified according to the method described in Molecular Cloning, 3rd ed., Current Protocols in Molecular Biology, DNA cloning 1: Core Techniques, A Practical Approach, Second Edition, Oxford University Press (1955) and the like.

As a cloning vector for producing the DNA library, a phage vector, a plasmid vector and the like can be used so long as they are autonomously replicable in Escherichia coli K12 strain. Specific examples thereof include ZAP Express [manufactured by Stratagene, Strategies, 5 , 58 (1992)], λzap II (manufactured by Stratagene), λgt10, λgt11 [DNA Cloning, A Practical Approach, 1, 49 (1985)], λ TriplEx (manufactured by Clontech), λExCell (manufactured by Amersham Pharmacia Biotech), pBluescript II KS(-), pBluescript II SK(+) [manufactured by Stratagene, Nucleic Acids Research, 17, 9494 (1989)], pUC18 [Gene, 33, 103 (1985)], and the like.

The vector having a DNA incorporated therein is introduced into microorganisms belonging to Escherichia coli.

As the microorganisms belonging to Escherichia coli, any microorganisms belonging to Escherichia coli can be used. Specific examples thereof include Escherichia coli XL1-Blue MRF' [manufactured by Stratagene, Strategies, 5, 81 (1992)], Escherichia coli C600 [Genetics, 39, 440 (1954)], Escherichia coli Y1088 [Science, 222, 778 (1983)], Escherichia coli Y1090 [Science, 222, 778 (1983)], Escherichia coli NM522 [J. Mol. Biol., 166, 1 (1983)], Escherichia coli K802 [J. Mol. Biol., 16, 118 (1966)], Escherichia coli JM109 [Gene, 38, 275 (1985)], Escherichia coli DH5α [J. Mol. Biol., 166, 557 (1983)], and the like.

A desired clone can be obtained from the resulting DNA library by a colony hybridization method, a plaque hybridization method, a southern hybridization method or the like described in an experimentation document such as Molecular Cloning, 3rd ed., Current Protocols in Molecular Biology, DNA cloning 1: Core Techniques, A practical Approach, Second Edition, Oxford University (1995).

Examples of a DNA probe used in the hybridization include a DNA having a nucleotide sequence complementary to a nucleotide sequence of a DNA coding for known NDH polypeptides or a part thereof, a DNA synthesized on the basis of the nucleotide sequence of the DNA coding for known NDH polypeptides, a DNA fragment obtained by PCR or the like with a DNA primer designed using a known nucleotide sequence, and the like.

For example, a DNA fragment can be mentioned which is obtained from a microorganism belonging to Corynebacterium glutamicum using as a primer a DNA having nucleotide sequences represented by SEQ ID NO: 1 or 2 designed on the basis of the nucleotide sequence, represented by SEQ ID NO: 3, of ndh of the microorganism belonging to Corynebacterium glutamicum, the DNA being chemically synthesized with an 8905 type DNA synthesizer manufactured by Perceptive Biosystems or the like.

The obtained DNA is introduced into a vector either as such or after cleavage with an appropriate restriction endonuclease or the like, and a nucleotide sequence of the DNA is determined by an ordinary nucleotide sequence analytical method such as a dideoxy method [Proc. Natl. Acad. Sci. USA, 74, 5463 (1977)] using ABI377DNA Sequencer (manufactured by Perkin Elmer) or the like.

Further, a primer is prepared on the basis of the determined nucleotide sequence, and the desired DNA can be obtained by the PCR method [PCR Protocols, Academic Press (1990)] using the primer and the isolated and purified chromosomal DNA as a template.

The desired DNA can also be prepared by chemical synthesis with an 8905 type DNA synthesizer manufactured by Perceptive Biosystems or the like on the basis of the determined nucleotide sequence of the DNA.

As the above-obtained DNA coding for the polypeptide as used in the present invention, for example, a DNA coding for energy non-production NADH dehydrogenase possessed by plasmid pCS-CGndh carried by Escherichia coli DH5α/pCS-CGndh (FERM BP-08633) can be mentioned. This DNA is a DNA coding for NDH polypeptide A.

The microorganism used in the process for producing the amino acid in the present invention can be produced by introducing the DNA coding for the polypeptide as used in the present invention into the host microorganism.

As the method for introducing the DNA coding for the polypeptide as used in the present invention into the host microorganism, a method in which the DNA is inserted downstream of a promoter of an appropriate expression vector to form a recombinant DNA and the recombinant DNA is introduced into the host microorganism,can be mentioned.

The host microorganism is not particularly limited so long as it is an aerobic bacterium. The electron transport system of the microorganism is not necessarily a system using energy non-production NADH dehydrogenase.

Examples of the host microorganism include microorganisms belonging to the genus Escherichia, Corynebacterium, Brevibacterium, Arthrobacter, Aureobacterium, Cellulomonas, Clavibacter, Curtobacterium, Microbacterium, Pimerobacter, Enterobacter, Klebsiella, Serratia, Erwinia, Bacillus, Pseudomonas, Agrobacterium, Anabaena, Chromatium, Rhodobacter, Rhodopseudomonas, Rhodospirillum, Streptomyces. Zymomonas or the like.

Examples of the microorganisms belonging to the genus Escherichia include Escherichia coli XL1-Blue, Escherichia coli XL2-Blue, Escherichia coli DH1, Escherichia coli DH5α, Escherichia coli MC1000, Escherichia coli KY3276, Escherichia coli W1485, Escherichia coli JM109, Escherichia coli HB101, Escherichia coli No. 49, Escherichia coli W3110, Escherichia coli NY49, Escherichia coli MP347 and Escherichia coli NM522.

Examples of the microorganisms belonging to the genus Corynebacterium include microorganisms belonging to Corynebacterium glutamicum (for example, Corynebacterium glutamicum ATCC 13032 and Corynebacterium glutamicum ATCC 13869), Corynebacterium ammoniagenes (for example, Corynebacterium ammoniagenes ATCC 6872 and Corynebacterium ammoniagenes ATCC 21170), Corynebacterium acetoacidophilum (for example, Corynebacterium acetoacidophilum ATCC 13870), and the like.

Examples of the microorganisms belonging to the genus Brevibacterium include microorganisms belonging to Brevibacterium immariophilum, Brevibacterium saccharolyticum, Brevibacterium flavum, Brevibacterium lactofermentum, and the like.

Examples of the microorganisms belonging to the genus Arthrobacter include microorganisms belonging to Arthrobacter citreus, Arthrobacter globiformis, and the like.

Examples of the microorganisms belonging to the genus Aureobacterium include microorganisms belonging to Aureobacterium flavescens, Aureobacter iumsaperdae, Aureobacterium testaceum, and the like.

Examples of the microorganisms belonging to the genus Cellulomonas include microorganisms belonging to Cellulomonas flavigena, Cellulomonas carta, and the like.

Examples of the microorganisms belonging to the genus Clavibacter include microorganisms belonging to Clavibacter michiganensis, Clavibacter rathayi, and the like.

Examples of the microorganisms belonging to the genus Curtobacterium include microorganisms belonging to Curtobacterium albidum, Curtobacter iumcitreum, Curtobacerium luteum, and the like.

Examples of the microorganisms belonging to the genus Microbacterium include microorganisms belonging to Microbacterium ammoniaphilum (for example, Microbacterium ammoniaphilum ATCC 15354), Microbacterium lacticum, Microbacterium imperiale, and the like.

Examples of the microorganisms belonging to the genus Pimerobacter include microorganisms belonging to Pimerobacter simplex, and the like.

Examples of the microorganisms belonging to the genus Enterobacter include microorganisms belonging to Enterobacter agglomerans (for example, Enterobacter agglomerans ATCC 1228), Enterobacter aerogenes, Enterobacter amnigenus, Enterobacter asburiae, Enterobacter cloacae, Enterobacter dissolvens, Enterobacter gergoviae, Enterobacter hormaechei, Enterobacter intermedius, Enterobacter nimipressuralis, Enterobacter sakazakii, Enterobacter taylorae, and the like.

Examples of the microorganisms belonging to the genus Klebsiella include microorganisms belonging to Klebsiella planticola, and the like.

Examples of the microorganisms belonging to the genus Serratia include microorganisms belonging to Serratia ficaria, Serratia fonticola, Serratia liquefaciens, Serratia entomophila, Serratia grimessi, Serratia proteamaculans, Serratia odorifera, Serratia plymuthica, Serratia rubidaea, Serratia marcescens, and the like.

Examples of the microorganisms belonging to the genus Erwinia include microorganisms belonging to Erwinia uredovora, Erwinia carotovora, Erwinia ananas, Erwinia herbicola, Erwinia punctata, Erwinia terreus, Erwinia cacticida, Erwinia chrysanthemi, Erwinia mallotivora, Erwinia persicinus, Erwinia psidii, Erwinia quercina, Erwinia rhapontici, Erwinia rubrifaciens, Erwinia salicis, and the like.

Examples of the microorganisms belonging to the genus Bacillus include microorganisms belonging to Bacillus subtilis, Bacillus megaterium, Bacillus amyloliquefaciens, Bacillus coagulans, Bacillus licheniformis, Bacillus pumilus, and the like.

Examples of the microorganisms belonging to the genus Pseudomonas include microorganisms belonging to Pseudomonas putida, and the like.

Examples of the microorganisms belonging to the genus Agrobacterium include microorganisms belonging to Agrobacterium radiobacter, Agrobacterium rhizogenes, Agrobacterium rubi, and the like.

Examples of the microorganisms belonging to the genus Anabaena include microorganisms belonging to Anabaena cylindrica, Anabaena doliolum, Anabaena flosaquae, and the like.

Examples of the microorganisms belonging to the genus Chromatium include microorganisms belonging to Chromatium buderi, Chromatium tepidum, Chromatium vinosum, Chromatium warmingii, Chromatium fluviatile, and the like.

Examples of the microorganisms belonging to the genus Rhodobacter include microorganisms belonging to Rhodobacter capsulatus, Rhodobacter sphaeroides, and the like.

Examples of the microorganisms belonging to the genus Rhodopseudomonas include microorganisms belonging to Rhodopseudomonas blastica, Rhodopseudomonas marina, Rhodopseudomonas palustris, and the like.

Examples of the microorganisms belonging to the genus Rhodospirillum include Rhodospirillum rubrum, Rhodospirillum salexigens, Rhodospirillum salinarum, and the like.

Examples of the microorganisms belonging to the genus Streptomyces include microorganisms belonging to Streptomyces ambofaciens, Streptomyces aureofaciens, Streptomyces aureus, Streptomyces fungicidicus, Streptomyces griseochromogenes, Streptomyces griseus, Streptomyces lividans, Streptomyces olivogriseus, Streptomyces rameus, Streptomyces tanashiensis, Streptomyces vinaceus, and the like.

Examples of the microorganisms belonging to the genus Zymomonas include microorganisms belonging to Zymomonas mobilis, and the like.

Among the foregoing host microorganisms, the microorganisms belonging to the genus Corynebacterium, Brevibacterium, Arthrobacter, Aureobacterium, Cellulomonas, Clavibacter, Curtobacterium, Microbacterium, Pimerobacter, Escherichia or Bacillus are preferably used. The microorganisms belonging to the genus Corynebacterium or Escherichia are more preferably used. The microorganisms belonging to the genus Corynebacterium are further preferably used.

As the method for introducing a recombinant DNA into a host microorganism, any method capable of introducing a DNA into the host microorganism is available. Examples thereof include a method using a calcium ion [Proc. Natl. Acad. Sci., USA, 69, 2110 (1972)], a protoplast method (Japanese Published Unexamined Patent Application No. 248394/1988), an electroporation method [Nucleic Acids, Res., 16, 6127 (1988)], and the like.

As the expression vector, a vector capable of autonomous replication or incorporation into a chromosome in a host microorganism and containing a promoter in a site where the DNA coding for the polypeptide as used in the present invention can be transcribed is used.

Examples thereof include pBTrp2, pBTacl, pBTac2 (all manufactured by Boehringer Mannheim), pHelix1 (manufactured by Roche Diagnostics), pKK233-2 (manufactured by Amersham Pharmacia Biotech), pSE280 (manufactured by Invitrogen), pGEMEX-1 (manufactured by Promega), pQE-8 (manufactured by Qiagen), pET-3 (manufactured by Novagen), pKYP10 (Japanese Published Unexamined Patent Application No. 110600/1983), pKYP200 [Agric. Biol. Chem., 48, 669 (1984)], pLSA1 [Agric. Biol. Chem., 53, 277 (1989)], pGEL1 [Proc. Natl. Acad. Sci., USA, 82, 4306 (1985)], pBluescript II SK (+), pBluescript II KS(-) (manufactured by Stratagene), pTrS30 [prepared from Escherichia coli JM109/pTrS30 (FERM BP-5407)], pTrS32 [prepared from Escherichia coli JM109/pTrS32 (FERM BP-5408)], pPAC31 (WO 98/12343), pUC19 [Gene, 33, 103 (1985)], pSTV28 (manufactured by Takara Shuzo), pUC118 (manufactured by Takara Shuzo), pPA1 (Japanese Published Unexamined Patent Application No. 233798/1988), pCG116, pCG1 (Japanese Published Unexamined Patent Application No. 277082/1994), pCS299P (WO 00/63388) and the like.

As the promoter, any promoter functioning in the host microorganisms may be used. Examples thereof include promoters derived from microorganisms belonging to Escherichia coli, phages or the like, such as trp promoter (Pₜᵣₚ), lac promoter (P_{lac}), P_{L} promoter, P_{R} promoter and P_{SE} promoter, SPO1 promoter, SP02 promoter, penP Promoter and the like. Further, artificially modified promoters, such as a promoter in which two Pₜᵣₚs are arranged in series, tac promoter, lacT7 promoter and let I promoter.

When the host microorganism is a microorganism belonging to the genus Corynebacterium, P54-6 promoter [Appl. Microbiol. Biotechnol., 53, 674-679 (2000)] is also mentioned. When the host microorganism is a microorganism belonging to the genus Bacillus, xylA promoter [Appl. Microbiol. Biotechnol., 35, 549-599 (1991)] is also mentioned.

It is preferred that the recombinant DNA is capable of autonomous replication in host microorganisms, and at the same time, is a recombinant DNA comprising the foregoing promoter, a ribosome binding sequence, a DNA coding for the polypeptide as used in the present invention and a transcription termination sequence. A promoter-controlling gene may be incorporated therein.

It is preferred that there is an appropriate distance (for example, from 6 to 18 nucleotides) between the Shine-Dalgarno sequence as a ribosome binding sequence and an initiation codon.

Although the transcription termination sequence is not necessarily required, it is preferred that the transcription termination sequence lies immediately downstream of the structural gene.

As the recombinant DNA, for example, plasmid pCS-CGndh carried by Escherichia coli DH5α/pCS-CGndh (FERM BP-08633) can be mentioned.

Examples of the microorganisms of the present invention obtained by the foregoing method include Corynebacterium glutamicum LS-22/pCS-CGndh, Corynebacterium glutamicum ATCC 14752/pCS-CGndh and Corynebacterium glutamicum FERM BP-1069/pCS-CGndh.

The DNA coding for the polypeptide, as used in the present invention, which is introduced into the host microorganism may be present in a recombinant DNA in the microorganism or may be incorporated in a chromosome.

As the method in which the DNA is incorporated into the chromosome, for example, a method using a phage or a transposon described in Escherichia coli and Salmonella typhimurium, 1996, p. 2325, p. 2339, American Society for Microbiology or the like may be used.

An amino acid can be produced by culturing in a medium a microorganism obtained by introducing the DNA coding for the polypeptide as used in the present invention (hereinafter abbreviated as a microorganism of the present invention), forming and accumulating the amino acid in the culture and collecting the amino acid from the culture.

The amino acid is not particularly limited, and examples thereof include amino acids biosynthesized from 2-oxoglutaric acid, such as L-glutamic acid and L-glutamine, amino acids biosynthesized from oxaloacetic acid, such as L-aspartic acid and L-asparagine, amino acids biosynthesized from aspartic acid, such as L-lysine, L-methionine and L-threonine, amino acids biosynthesized from L-glutamic acid, such as L-arginine, L-proline and L-citrulline, amino acids biosynthesized from pyruvic acid, such as L-valine, L-leucine and L-isoleucine, amino acids biosynthesized from 3-phosphoglyceric acid, such as L-serine, L-cysteine and glycine, amino acids biosynthesized from chorismic acid, such as L-tryptophan, L-tyrosine and L-phenylalanine, L-histidine, and the like.

The medium used in culturing may be either a natural medium or a synthetic medium so long as it contains a carbon source, a nitrogen source, inorganic salts and the like which can be assimilated by the microorganisms of the present invention, in which the microorganisms can be grown and a desired amino acid is produced efficiently.

Any carbon source capable of being assimilated by the microorganisms of the present invention may be used. Glucose, fructose, sucrose, molasses containing the same, carbohydrates such as starch and starch hydrolyzate, organic acids such as acetic acid and propionic acid, alcohols such as methanol, ethanol and propanol, and the like can be used.

As the nitrogen source, ammonia, inorganic or organic acid ammonium salts such as ammonium chloride, ammonium sulfate, ammonium acetate and ammonium phosphate, other nitrogen-containing compounds, peptone, meat extract, yeast extract, corn steep liquor, casein hydrolyzate, soybean cake hydrolyzate, various fermented bacteria, digested substances thereof, and the like can be used.

As the inorganic salt, potassium dihydrogenphosphate, dipotassium hydrogenphosphate, magnesium phosphate, magnesium sulfate, sodium chloride, ferrous sulfate, manganese sulfate, copper sulfate, calcium carbonate and the like can be used.

The culturing is conducted under aerobic conditions of shake culturing, or submerged culturing with stirring or the like. The culturing temperature is preferably from 15°C to 50°C, more preferably from 20°C to 45°C. The culturing time is usually from 5 hours to 7 days, preferably from 12 hours to 4 days. During the culturing, pH is maintained at from 3 to 9 as required. The pH is adjusted with an inorganic or organic acid, an alkaline solution, urea, calcium carbonate, ammonia or the like.

During the culturing, an antibiotic such as penicillin, ampicillin or tetracycline may be added to the medium as required.

When culturing a microorganism into which a DNA is introduced with a recombinant DNA using an inducible promoter as the promoter, an inducer may be added to a medium as required. For example, when culturing a microorganism into which a DNA is introduced with a recombinant DNA using lac promoter, isopropyl-β-D-thiogalactopyranoside or the like may be added to a medium, and when culturing a microorganism into which a DNA is introduced with a recombinant DNA using trp promoter, indole acrylic acid or the like may be added to a medium.

An amino acid can be recovered from a culture after completion of the culturing by methods usually employed for isolation of an amino acid, such as a method using activated carbon, a method using an ion exchange resin, a crystallization method and a precipitation method which are employed either singly or in combination.

Examples are described below. However, the present invention is not limited to the following Examples.

### Example 1

(1) Corynebacterium glutamicum LS-22 strain was inoculated in LB medium [medium containing 10 g/L bactotrypton (manufactured by Difco), 5 g/L yeast extract (manufactured by Difco) and 5 g/L sodium chloride], and was cultured overnight at 30°C. After the culturing, a chromosomal DNA of the microorganism was isolated and purified according to the method of Eikmanns et al [Microbiol., 140, 1817 (1994)].

A DNA having the nucleotide sequence represented by SEQ ID NO: 1 or 2 was synthesized on the basis of a nucleotide sequence of ndh of Corynebacterium glutamicum ATCC 13032 represented by SEQ ID NO: 3.

PCR was performed in 40 µL of a reaction solution containing 2.5 units of PfuDNA polymerase (manufactured by Stratagene) and dNTPs (dATP, dGTP, dCTP and dTTP) in amounts of 200 µmol/L each using the above chromosomal DNA (0.1 µg) as a template.

After completion of the reaction, 4 µL of the resulting reaction solution was subjected to agarose gel electrophoresis to confirm that a 1.9 kb fragment corresponding to ndh of known Corynebacterium glutamicum was amplified. Then, the remaining reaction solution and an equal amount of a TE [solution containing 10 mmol/L Tris-hydrochloride (pH 8.0) and 1 mmol/L ethylenediaminetetraacetic acid] saturated phenol/chloroform (1 vol/l vol) solution were mixed. A 2-fold amount of cold ethanol was added to the upper layer obtained by centrifugal separation of the solution, and the mixture was allowed to stand at -80°C for 30 minutes. The solution was centrifuged to obtain a DNA, and the DNA was dissolved in 20 µL of TE.

5 µl of the DNA-containing solution and 0.06 µg of pGEM^{R}-T Easy vector (manufactured by Promega) were reacted at 16°C for 16 hours using a ligation kit, pGEM^{R}-T Easy vector system (manufactured by Promega) to ligate a ndh-containing DNA fragment with pGEM^{R}-T Easy vector.

Escherichia coli DH5α strain was transformed by an electroporation method [Nucleic Acid Res., 16, 6127-6145 (1988)] using the reaction solution after the ligation reaction.

The resulting transformant was spread on an LB agar medium containing 100 µg/mL ampicillin, and cultured overnight at 30°C. A plasmid was extracted from colonies grown in the agar medium in a usual manner, and its structure was analyzed with a restriction endonuclease to confirm that the plasmid was a plasmid in which the DNA fragment containing ndh of Corynebacterium glutamicum was inserted in the pGEM^{R}-T Easy vector. This plasmid was designated pT-CGndh.

1 µg of plasmid pT-CGndh was cleaved with restriction endonucleases KpnI and SalI, and a solution containing the resulting DNA fragment was subjected to agarose gel electrophoresis to separate the DNA fragment of approximately 2 kb.

0.2 µg of pCS299P (WO 00/63388) as an expression vector for coryneform bacteria was cleaved with restriction endonucleases KpnI and SalI, and a solution containing the resulting DNA fragment was subjected to agarose gel electrophoresis to separate the DNA fragment of approximately 5.4 kb.

The above-obtained ndh-containing DNA fragment of approximately 2 kb and the cleaved fragment of pCS299P (approximately 5.4 kb) were reacted at 16°C for 16 hours using a ligation kit (manufactured by Takara Shuzo) for ligation.

Corynebacterium glutamicum LS-22 strain was transformed with the reaction solution after the ligation reaction by an electroporation method [Nucleic Acid Res., 16, 6127-6145 (1988)].

The resulting transformant was spread on an LB agar medium containing 25 µg/mL of kanamycin, and cultured at 30°C for 1 day. A plasmid was extracted from colonies grown in the agar medium in a usual manner, and its structure was analyzed with a restriction endonuclease to confirm that the plasmid was a plasmid in which the ndh-containing DNA fragment was inserted in pCS299P. This plasmid was designated pCS-CGndh.

Escherichia coli DH5α/pCS-CGndh containing plasmid pCS-CGndh was deposited as FERM BP-08633 on February 19, 2004 in International Patent Organism Depositary, National Institute of Advanced Industrial Science and Technology, AIST Tsukuba Central 6, 1-1-1 Higashi, Tsukuba Ibaraki, 305-8566, Japan.

(2) Each of Corynebacterium glutamicum LS-22 strain and Corynebacterium glutamicum LS-22/pCS-CGdh strain obtained by introducing pCS-CGndh into Corynebacterium glutamicum LS-22 strain was inoculated into 5 mL of LB medium in a test tube, and cultured overnight at 30°C while being shaken.

One mL of the culture was added to a 500-mL conical flask containing 100 mL of MG medium [medium containing 10 g/L glucose, 3g/L potassium dihydrogenphosphate, 3 g/L dipotassium hydrogenphosphate, 2 g/L ammonium chloride, 2 g/L urea, 0.5 g/L magnesium sulfate 7-hydrate, 10 mg/L iron sulfate 10-hydrage, 1 mg/L manganese sulfate 7-hydrate, 30 mg/L biotin, 1 mg/L thiamine hydrochloride, 20 mg/L cysteine hydrochloride, 0.5 g/L casamino acid and 1 mL/L metal mix (solution containing 990 mg/L iron sulfate 7-hydrate, 880 mg/L zinc sulfate 7-hydrate, 393 mg/L copper sulfate 5-hydrate, 72 mg/L manganese chloride 4-hydrate, 88 mg/L sodium tetraborate 10-hydrate and 37 mg/L ammonium paramolybdate 4-hydrate)], and was cultured at 30°C and 220 rpm for 80 hours while being shaken. After the culturing, a concentration of glutamic acid in a culture supernatant was measured by HPLC.

In the HPLC analysis, the culture supernatant was subjected to column AQ-312 (manufactured by YMC) (mobile phase: solution of pH 2.4 containing 2.94 g/L sodium citrate, 1.42 g/L sodium sulfate, 17 mL/L n-propanol and 3 g/L sodium laurylsulfate), and was mixed with a reaction solution (solution containing 18.5 g/L boric acid, 11 g/L NaOH, 0.6 g/L orthophthalaldehyde 2 ml/L mercaptoethanol and 3 mL/L Brige-35). The mixture was subjected to fluorescence analysis with an excitation wavelength of 345 nm and an absorption wavelength of 455 nm.

As the result, Corynebacterium glutamicum LS-22 strain accumulated 1.5 g/L glutamic acid in the culture, while Corynebacterium glutamicum LS-22/pCS-CGndh strain accumulated 2.3 g/L glutamic acid.

### Example 2

pCS-CGndh was introduced into Corynebacterium glutamicum ATCC 14752 strain as in Example 1 using the electroporation method to obtain Corynebacterium glutamicum ATCC 14752/pCS-CGndh strain.

Each of Corynebacterium glutamicum ATCC 14752 strain and Corynebacterium glutamicum ATCC 14752/pCS-CGndh strain was inoculated in a test tube containing 5 mL of GS medium [medium of pH 7.2 containing 70 g/L glucose, 10 g/L corn steep liquor, 10 g/L meat extract, 10 g/L yeast extract, 5 g/L ammonium sulfate, 0.5 g/L potassium dihydrogenphosphate, 1.5 g/L dipotassium hydrogenphosphate, 0.5 g/L magnesium sulfate 7-hydrate, 10 mg/L iron sulfate 10-hydrate, 10 mg/L manganese sulfate 7-hydrate, 0.8 mg/L copper sulfate 5-hydrate, 8.3 g/L urea, 5 µg/L biotin and 1 mg/L thiamine hydrochloride), and was cultured at 30°C for 24 hours while being shaken

2.5 mL of this culture was added to a conical flask containing 25 ml of GP medium [medium of pH 7.2 containing 116 g/L glucose, 4 g/L fructose, 50 g/L ammonium chloride, 10 mg/L nicotinic acid, 0.7 g/L potassium dihydrogenphosphate, 0.7 g/L dipotassium hydrogenphosphate, 0.5 g/L magnesium sulfate 7-hydrate, 20 mg/L iron sulfate 10-hydrate, 20 mg/L manganese sulfate 7-hydrate, 0.8 mg/L copper sulfate 5-hydrate, 5 g/L urea, 0.5 µg/L biotin, 1 mg/L thiamine hydrochloride and 50 g/L calcium carbonate), and was cultured at 30°C and 220 rpm for 72 hours while being shaken.

After the culturing, the amount of glutamine accumulated in the culture supernatant was measured under the HPCL conditions described in Example 1.

Consequently, the amount of glutamine accumulated in Corynebacterium glutamicum ATCC 14752 strain was 32.2 g/L, while the amount of glutamine accumulated in Corynebacterium glutamicum ATCC 14752/pCS-CGndh strain was 33.3 g/L.

### Example 3

pCS-CGndh was introduced into Corynebacterium glutamicum FERM BP-1069 strain as in Example 1 using the electroporation method to obtain Corynebacterium glutamicum FERM BP-1069/pCS-CGndh strain.

Each of Corynebacterium glutamicum FERM BP-1069 strain and Corynebacterium glutamicum FERM BP-1069/pCS-CGndh strain was inoculated in a test tube containing 5 mL of LS medium [medium of pH 7.2 containing 50 g/L sucrose, 30 g/L corn steep liquor, 20 g/L meat extract, 20 g/L casamino acid, 8 g/L ammonium sulfate, 2 g/L potassium dihydrogenphosphate, 0.5 g/L magnesium sulfate 7-hydrate, 3 g/L urea, 20 g/L peptone, 10 mg/L iron sulfate 10-hydrate, 10 mg/L zinc sulfate 7-hydrate, 20 mg/L nicotinic acid, 10 mg/L calcium pantothenate, 0.1 mg/L biotin, 1 mg/L thiamine hydrochloride and 10 g/L calcium carbonate], and was cultured at 30°C for 24 hours while being shaken.

0.5 mL of this culture was added to a test tube containing 5 mL of LP medium (medium of pH 7.0 containing 100 g/L molasses (as a sugar content), 45 g/L ammonium sulfate, 3 g/L urea, 0.5 g/L potassium dihydrogenphosphate, 0.5 g/L magnesium sulfate 7-hydrate, 0.3 mg/L biotin and 30 g/L calcium carbonate], and was cultured at 30°C and 220 rpm for 72 hours while being shaken. After completion of the culturing, a concentration of lysine in a culture supernatant was measured by HPLC.

In the HPLC analysis, the culture supernatant was subjected to column ODS-80TS (manufactured by TOSOH) (mobile phase: solution of pH 6.0 containing 2.94 g/L sodium citrate, 1.42 g/L sodium sulfate, 300 mL/L acetonitrile and 3 g/L sodium laurylsulfate), and was mixed with a reaction solution (solution containing 18.5 g/L boric acid, 11 g/L sodium hydroxide, 0.6 g/L orthophthalaldehyde, 2 ml/L mercaptoethanol and 3 mL/L Brige-35). The mixture was subjected to fluorescence analysis with an excitation wavelength of 345 nm and an absorption wavelength of 455 nm.

As a result, the amount of lysine accumulated in Corynebacterium glutamicum FERM BP-1069 strain was 24.8 g/L, while the amount of lysine accumulated in Corynebacterium glutamicum FERM BP-1069/pCS-CGndh strain was 28.6 g/L.

### Industrial Applicability

According to the present invention, an industrially advantageous process for producing an amino acid can be provided.

### Sequence Listing Free Text

SEQ ID NO: 1 - description of an artificial sequence: synthetic DNA
SEQ ID NO: 2 - description of an artificial sequence: synthetic DNA

## Claims

1. A process for producing an amino acid, which comprises culturing, in a medium, a microorganism into which a DNA coding for energy non-production NADH dehydrogenase is introduced, forming and accumulating an amino acid in a culture, and recovering the amino acid from the culture, wherein the DNA coding for energy non-production NADH dehydrogenase is a DNA having a nucleotide sequence selected from the group consisting of nucleotide sequences represented by SEQ ID NOs: 3, 5, 7, 9, 11, 13 and 15, or a DNA which hybridizes, under stringent conditions, with a DNA having a nucleotide sequence complementary to the nucleotide sequence.

2. A microorganism which belongs to
(a) the genus Corynebacterium; or
(b) the species Corynebacterium glutamicum;
and into which a DNA coding for energy non-production NADH dehydrogenase is introduced, wherein the DNA coding for energy non-production NADH dehydrogenase is a DNA having a nucleotide sequence selected from the group consisting of nucleotide sequences represented by SEQ ID NOs: 3, 5, 7, 9, 11, 13 and 15, or a DNA which hybridizes, under stringent conditions, with a DNA having a nucleotide sequence complementary to the nucleotide sequence.

3. The process according to claim 1 or the microorganism according to claim 2, wherein the DNA coding for energy non-production NADH dehydrogenase is a DNA derived from a microorganism selected from the group consisting of microorganisms belonging to
(a) the genus Corynebacterium, Escherichia, Pseudomonas, Azotobacter, Salmonella or Lactobacillus; or
(b) the species Corynebacterium glutamicum, Corynebacterium diphtheriae, Escherichia coli, Pseudomonas fluorescens, Azotobacter vinelandii, Salmonella typhimurium or Lactobacillus plantarum;
or a DNA which hybridizes, under stringent conditions, with a DNA having a nucleotide sequence complementary to the nucleotide sequence of the DNA.

4. The process according to claim 1 or the microorganism according to claim 2, wherein the DNA coding for energy non-production NADH dehydrogenase is a DNA coding for energy non-production NADH dehydrogenase possessed by a plasmid pCS-CGndh carried by Escherichia coli DH5α/pCS-CGndh (FERM BP-08633) or a DNA which hybridizes, under stringent conditions, with a DNA having a nucleotide sequence complementary to the nucleotide sequence of the DNA and which encodes a polypeptide having the energy non-production NADH dehydrogenase activity.

5. The process according to claim 1 or the microorganism according to claim 2, wherein the energy non-production NADH dehydrogenase is a polypeptide having an amino acid sequence selected from the group consisting of amino acids sequences represented by SEQ ID NOs: 4, 6, 8, 10, 12, 14 and 16, or a polypeptide comprising an amino acid sequence wherein one or more amino acid residues are deleted, substituted or added in the amino acid sequence of the polypeptide and having the energy non-production NADH dehydrogenase activity.

6. The process according to claim 1 or the microorganism according to claim 2, wherein the energy non-production NADH dehydrogenase is a polypeptide encoded by a DNA possessed by a plasmid pCS-CGndh carried by Escherichia coli DH5α/pCS-CGndh (FERM BP-08633) or a polypeptide comprising an amino acid sequence wherein one or more amino acid residues are deleted, substituted or added in the amino acid sequence of the polypeptide and having the energy non-production NADH dehydrogenase activity.

7. The process according to any one of claims 1 or 3 to 6, wherein the microorganism into which the DNA coding for energy non-production NADH dehydrogenase is introduced is a microorganism selected from the group consisting of microorganisms belonging to
(a) the genus Escherichia, Corynebacterium, Brevibacterium, Arthrobacter, Aureobacterium, Cellulomonas, Clavibacter, Curtobacterium, Microbacterium, Pimerobacter or Bacillus; or
(b) the species Escherichia coli, Corynebacterium glutamicum, Corynebacterium flavum, Corynebacterium lactofermentum, or Corynebacterium efficasis.

8. The process according to any one of claims 1 or 3 to 7, wherein the produced amino acid is an amino acid selected from the group consisting of L-glutamic acid, L-glutamine, L-aspartic acid, L-asparagine, L-lysine, L-methionine, L-threonine, L-arginine, L-proline, L-citrulline, L-valine, L-leucine, L-isoleucine, L-serine, L-cysteine, glycine, L-tryptophan, L- tyrosine, L-phenylalanine and L-histidine.

9. Corynebactecium glutamicum ATCC 14752/pCS-CGndh or Corynebacterium glutamicum FERM BP-1069 / pCS-CGndh.

10. Escherichia coli DHSα/pCS-CGndh (FERM BP-08633).

11. Plasmid pCS-CGndh carried by Escherichia coli DHSα/pCS-CGndh (FERM BP-08633).

## Patentansprüche

1. Verfahren zur Herstellung einer Aminosäure, umfassend die Züchtung, in einem Medium, eines Mikroorganismus, in welchen eine DNA eingeführt ist, die eine Energie-Nichtproduktions-NADH-Dehydrogenase codiert, die Erzeugung und Anreicherung einer Aminosäure in einer Kultur und die Gewinnung der Aminosäure aus der Kultur, wobei die DNA, die eine Energie-Nichtproduktions-NADH-Dehydrogenase codiert, eine DNA ist, die eine Nucleotidsequenz hat, welche ausgewählt ist aus der Gruppe bestehend aus Nucleotidsequenzen, wie dargestellt durch SEQ ID NOs: 3, 5, 7, 9, 11, 13 und 15, oder eine DNA, die unter stringenten Bedingungen mit einer DNA hybridisiert, welche eine zu der Nucleotidsequenz komplementäre Nucleotidsequenz hat.

2. Mikroorganismus, der zur
(a) Gattung Corynebacterium; oder
(b) Art Corynebacterium glutamicum gehört;
und in welchen eine DNA eingeführt ist, die eine Energie-Nichtproduktions-NADH-Dehydrogenase codiert, wobei die DNA, die eine Energie-Nichtproduktions-NADH-Dehydrogenase codiert, eine DNA ist, die eine Nucleotidsequenz hat, welche ausgewählt ist aus der Gruppe bestehend aus Nucleotidsequenzen, wie dargestellt durch SEQ ID NOs: 3, 5, 7, 9, 11, 13 und 15, oder eine DNA, die unter stringenten Bedingungen mit einer DNA hybridisiert, welche eine zu der Nucleotidsequenz komplementäre Nucleotidsequenz hat.

3. Verfahren gemäß Anspruch 1 oder Mikroorganismus gemäß Anspruch 2, wobei die DNA, die für eine Energie-Nichtproduktions-NADH-Dehydrogenase codiert, eine DNA ist, die aus einem Mikroorganismus abgeleitet ist, der ausgewählt ist aus der Gruppe bestehend aus Mikroorganismen, die zu
(a) der Gattung Corynebacterium, Escherichia, Pseudomonas, Azotobacter, Salmonella oder Lactobacillus; oder
(b) der Art Corynebacterium alutamicum, Corynebacterium diphteriae, Escherichia coli, Pseudomonas fluorescens, Azotobacter vinelandii, Salmonella typhimurium oder Lactobacillus plantarum gehören;
oder eine DNA, die unter stringenten Bedingungen mit einer DNA hybridisiert, welche eine zu der Nucleotidsequenz der DNA komplementäre Nucleotidsequenz hat.

4. Verfahren gemäß Anspruch 1 oder Mikroorganismus gemäß Anspruch 2, wobei die DNA, die eine Energie-Nichtproduktions-NADH-Dehydrogenase codiert, eine DNA ist, die eine Energie-Nichtproduktions-NADH-Dehydrogenase in einem Plasmid pCS-CGndh, das von Escherichia coli DH5α/pCS-GCndh (FERM BP-08633) getragen wird, codiert, oder eine DNA, die unter stringenten Bedingungen mit einer DNA hybridisiert, welche eine zu der Nucleotidsequenz der DNA komplementäre Nucleotidsequenz hat und welche ein Polynucleotid, welches die Energie-Nichtproduktions-NADH-Dehydrogeriase-Aktivität hat, codiert.

5. Verfahren gemäß Anspruch 1 oder Mikroorganismus gemäß Anspruch 2, wobei die Energie-Nichtproduktions-NADH-Dehydrogenase ein Polypeptid ist, das eine Aminosäuresequenz hat, welche ausgewählt ist aus der Gruppe bestehend aus Aminosäuresequenzen, wie dargestellt durch SEQ ID NOs: 4, 6, 8, 10, 12, 14 und 16, oder ein Polypeptid, das eine Aminosäuresequenz umfasst, in der eine oder mehrere Aminosäurereste in der Aminosäuresequenz des Polypeptids deletiert, substituiert oder hinzugefügt sind, und das Energie-Nichtproduktions-NADH-Dehydrogenase-Aktivität hat.

6. Verfahren gemäß Anspruch 1 oder Mikroorganismus gemäß Anspruch 2, wobei die Energie-Nichtproduktions-NADH-Dehydrogenase ein Polypeptid ist, welches durch eine DNA codiert wird, die in einem Plasmid pCS-CGndh enthalten ist, das von Escherichia coli DH5α/pCS-CGndh (FERM BP-08633) getragen wird, oder ein Polypeptid, das eine Aminosäuresequenz umfasst, in der eine oder mehrere Aminosäurereste in der Aminosäuresequenz des Polypeptids deletiert, substituiert oder hinzugefügt sind, und das Energie-Nichtproduktions-NADH-Dehydrogenase-Aktivität hat.

7. Verfahren gemäß einem der Ansprüche 1 oder 3 bis 6, wobei der Mikroorganismus, in welchen die DNA, die eine Energie-Nichtproduktions-NADH-Dehydrogenase codiert, eingeführt ist, ein Mikroorganismus ist, der ausgewählt ist aus der Gruppe bestehend aus Mikroorganismen, die zu
(a) der Gattung Escherichia, Corynebacterium, Brevibacterium, Arthrobacter, Aureobacterium, Cellulomonas, Clavibacter, Curtobacterium, Microbacterium, Pimerobacter oder Bacillus; oder
(b) der Art Escherichia coli, Corynebacterium glutamicum, Corynebacterium flavum, Corynebacterium lactofermentum oder Corynebacterium efficasis gehören.

8. Verfahren gemäß einem der Ansprüche 1 oder 3 bis 7, wobei die produzierte Aminosäure eine Aminosäure ist, ausgewählt aus der Gruppe bestehend aus L-Glutaminsäure, L-Glutamin, L-Asparaginsäure, L-Asparagin, L-Lysin, L-Methionin, L-Threonin, L-Arginin, L-Prolin, L-Citrullin, L-Valin, L-Leucin, L-Isoleucin, L-Serin, L-Cystein, Glycin, L-Tryptophan, L-Tyrosin, L-Phenylalanin und L-Histidin.

9. Corynebacterium glutamicum ATCC 14752/pCS-CGndh oder Corynebacterium glutamicum FERM BP-1069 / pCS-CGndh.

10. Escherichia coli DH5α/pCS-CGndh (FERM BP-08633).

11. Plasmid pCS-CGndh, das von Escherichia coli DH5α/pCS-CGndh (FERM BP-08633) getragen wird.

## Revendications

1. Procédé de production d'un acide aminé, qui consiste à mettre en culture, dans un milieu, un micro-organisme dans lequel un ADN codant pour une NADH déshydrogénase non productrice d'énergie est introduit, à former et à accumuler un acide aminé dans une culture, et à récupérer l'acide aminé à partir de la culture, où l'ADN codant pour une NADH déshydrogénase non productrice d'énergie est un ADN ayant une séquence nucléotidique choisie dans le groupe consistant en séquences nucléotidiques représentées par les SEQ ID NO: 3, 5, 7, 9, 11, 13 et 15, ou un ADN qui s'hybride, dans des conditions de stringence, avec un ADN ayant une séquence nucléotidique complémentaire de la séquence nucléotidique.

2. Micro-organisme qui appartient
(a) au genre *Corynebacterium ;* ou
(b) à l'espèce *Corynebacterium glutamicum* ;
et dans lequel un ADN codant pour une NADH déshydrogénase non productrice d'énergie est introduit, où l'ADN codant pour la NADH déshydrogénase non productrice d'énergie est un ADN ayant une séquence nucléotidique choisie dans le groupe consistant en séquences nucléotidiques représentées par les SEQ ID NO: 3, 5, 7, 9, 11, 13 et 15, ou un ADN qui s'hybride, dans des conditions de stringence, avec un ADN ayant une séquence nucléotidique complémentaire de la séquence nucléotidique.

3. Procédé selon la revendication 1, ou micro-organisme selon la revendication 2, où l'ADN codant pour une NADH déshydrogénase non productrice d'énergie est un ADN dérivé d'un micro-organisme choisi dans le groupe consistant en micro-organismes appartenant
(a) aux genres *Corynebacterium, Escherichia, Pseudomonas, Azotobacter, Salmonella* ou *Lactobacillus ;* ou
(b) aux espèces *Corynebacterium glutamicum, Corynebacterium diphtheriae, Escherichia coli, Pseudomonas fluorescens, Azotobacter vinelandii, Salmonella typhimurium ou Lactobacillus plantarum* ;
ou un ADN qui s'hybride, dans des conditions de stringence, avec un ADN ayant une séquence nucléotidique complémentaire de la séquence nucléotidique de l'ADN.

4. Procédé selon la revendication 1 ou micro-organisme selon la revendication 2, où l'ADN codant pour une NADH déshydrogénase non productrice d'énergie est un ADN codant pour une NADH déshydrogénase non productrice d'énergie que possède un plasmide pCS-CGndh porté par *Escherichia coli* DHSα/pCS-CGndh (FERM BP-08633) ou un ADN qui s'hybride, dans des conditions de stringence, avec un ADN ayant une séquence nucléotidique complémentaire de la séquence nucléotidique de l'ADN et qui code pour un polypeptide ayant l'activité de NADH déshydrogénase non productrice d'énergie.

5. Procédé selon la revendication 1 ou micro-organisme selon la revendication 2, où la NADH déshydrogénase non productrice d'énergie est un polypeptide ayant une séquence d'acides aminés choisie parmi le groupe consistant en séquences d'acides aminés représentées par les SEQ ID NO: 4, 6, 8, 10, 12, 14 et 16, ou un polypeptide comprenant une séquence d'acides aminés où un ou plusieurs résidu d'acides aminés sont supprimés, substitués ou ajoutés dans la séquence d'acides aminés du polypeptide et ayant l'activité de NADH déshydrogénase non productrice d'énergie.

6. Procédé selon la revendication 1 ou micro-organisme selon la revendication 2, où la NADH déshydrogénase non productrice d'énergie est un polypeptide codé par un ADN que possède un plasmide pCS-CGndh porté par *Escherichia coli* DHSα/pCS-CGndh (FERM BP-08633) ou un polypeptide comprenant une séquence d'acides aminés où un ou plusieurs résidus d'acides aminés sont supprimés, substitués ou ajoutés dans la séquence d'acides aminés du polypeptide et ayant l'activité de NADH déshydrogénase non productrice d'énergie.

7. Procédé selon l'une quelconque des revendications 1 ou 3 à 6, où le micro-organisme dans lequel l'ADN codant pour une NADH déshydrogénase non productrice d'énergie est introduit est un micro-organisme choisi dans le groupe consistant en micro-organismes appartenant
(a) aux genres *Escherichia, Corynebacterium, Brevibacterium, Arthrobacter, Aureobacterium, Cellulomonas, Clavibacter, Curtobacterium, Microbacterium, Pimerobacter* ou *Bacillus ;* ou
(b) aux espèces *Escherichia coli, Corynebacterium glutamicum, Corynebacterium flavum, Corynebacterium lactofermentum,* ou *Corynebacterium efficasis.*

8. Procédé selon l'une quelconque des revendications 1 ou 3 à 7, où l'acide aminé produit est un acide aminé choisi dans le groupe consistant en acide L-glutamique, L-glutamine, acide L-aspartique, L-asparagine, L-lysine, L-méthionine, L-thréonine, L-arginine, L-proline, L-citrulline, L-valine, L-leucine, L-isoleucine, L-sérine, L-cystéine, glycine, L-tryptophane, L-thyrosine, L-phénylalanine et L-histidine.

9. *Corynebacterium glutamicum* ATCC 14752/pCS-CGndh ou *Corynebacterium glutamicum* FERM BP-1069 / pCS-CGndh.

10. *Escherichia coli* DH5α/pCS-CGndh (FERM BP-08633).

11. Plasmide pCS-CGndh porté par *Escherichia coli* DH5α/pCS-CGndh (FERM BP-08633).


### SEQUENCE LISTING

<110> KYOWA HAKKO KOGYO CO., LTD
<120> Method for producing amino acid
<130> 1657
<160> 16
<170> PatentIn Ver. 3.1
<210> 1
   <211> 30
   <212> DNA
   <213> Artificial
<220>
   <223> Synthetic DNA
<400> 1
   ctgcttgccc tgcaggtgca ccagcaaacg 30
<210> 2
   <211> 30
   <212> DNA
   <213> Artificial
<220>
   <223> Synthetic DNA
<400> 2
   cgagctgcgc gacaaccagg aattcagcgg 30
<210> 3
<2n> 1404
   <212> DNA
   <213> Corynebacterium glutamicum ATCC13032
<220>
   <221> CDS
   <222>
<400> 3
<210> 4
   <211> 467
   <212> PRT
   <213> Corynebacterium glutamicum ATCC13032
<400> 4
   Met Ser Val Asn Pro Thr Arg Pro Glu Gly Gly Arg His His Val Val
   1 5 10 15
   Val Ile Gly Ser Gly Phe Gly Gly Leu Phe Ala Ala Lys Asn Leu Ala
   20 25 30
   Lys Ala Asp Val Asp Val Thr Leu Ile Asp Arg Thr Asn His His Leu
   35 40 45
   Phe Gln Pro Leu Leu Tyr Gln Val Ala Thr Gly Ile Leu Ser Ser Gly
   50 55 60
   Glu Ile Ala Pro Ser Thr Arg Gln Ile Leu Gly Ser Gln Glu Asn Val
   65 70 75 80
   Asn Val Ile Lys Gly Glu Val Thr Asp Ile Asn Val Glu Ser Gln Thr
   85 90 95
   Val Thr Ala Ser Leu Gly Glu Phe Thr Arg Val Phe Glu Tyr Asp Ser
   100 105 110
   Leu Val Val Gly Ala Gly Ala Gly Gln Ser Tyr Phe Gly Asn Asp His
   115 120 125
   Phe Ala Glu Phe Ala Pro Gly Met Lys Ser Ile Asp Asp Ala Leu Glu
   130 135 140
   Ile Arg Ala Arg Ile Ile Gly Ala Phe Glu Arg Ala Glu Ile Cys Glu
   145 150 155 160
   Asp Pro Ala Glu Arg Glu Arg Leu Leu Thr Phe Val Val Val Gly Ala
   165 170 175
   Gly Pro Thr Gly Val Glu Leu Ala Gly Gln Leu Ala Glu Met Ala His
   180 185 190
   Arg Thr Leu Ala Gly Glu Tyr Lys Asn Phe Asn Thr Asn Ser Ala Lys
   195 200 205
   Ile Ile Leu Leu Asp Gly Ala Pro Gln Val Leu Pro Pro Phe Gly Lys
   210 215 220
   Arg Leu Gly Arg Asn Ala Gln Arg Thr Leu Glu Lys Met Gly Val Asn
   225 230 235 240
   Val Arg Leu Asn Ala Met Val Thr Asn Val Asp Ala Thr Ser Val Thr
   245 250 255
   Tyr Lys Thr Lys Asp Gly Glu Glu His Thr Ile Glu Ser Phe Cys Lys
   260 265 270
   Ile Trp Ser Ala Gly Val Ala Ala Ser Pro Leu Gly Lys Leu Val Ala
   275 280 285
   Glu Gln Thr Gly Val Glu Thr Asp Arg Ala Gly Arg Val Met Val Asn
   290 295 300
   Asp Asp Leu Ser Val Gly Asp Gln Lys Asn Val Phe Val Val Gly Asp
   305 310 315 320
   Met Met Asn Tyr Asn Asn Leu Pro Gly Val Ala Gln Val Ala Ile Gln
   325 330 335
   Ser Gly Glu Tyr Val Ala Glu Gln Ile Glu Ala Glu Val Glu Gly Arg
   340 345 350
   Ser Asn Thr Glu Arg Glu Ala Phe Asp Tyr Phe Asp Lys Gly Ser Met
   355 360 365
   Ala Thr Ile Ser Arg Phe Ser Ala Val Val Lys Met Gly Lys Val Glu
   370 375 380
   Val Thr Gly Phe Ile Gly Trp Val Leu Trp Leu Ala Val His Ile Met
   385 390 395 400
   Phe Leu Val Gly Phe Arg Asn Arg Phe Val Ser Ala Ile Ser Trp Gly
   405 410 415
   Leu Asn Ala Leu Ser Arg Lys Arg Trp Asn Leu Ala Thr Thr Arg Gln
   420 425 430
   Gln Leu His Ser Arg Thr Thr Leu Ser Lys Phe Ala His Glu Leu Glu
   435 440 445
   Glu Ala Ser Ser Asp Leu Pro Ile Glu Leu Arg Asp Asn Gln Arg Phe
   450 455 460
   Ser Gly Lys
   465
<210> 5
   <211> 1362
   <212> DNA
   <213> Corynebacterium diphtheriae
<220>
   <221> CDS
   <222>
<400> 5
   atg act aac acc cca ttt cgc cca gaa ggt gga cgc cac cac gtt gta 48
   Met Thr Asn Thr Pro Phe Arg Pro Glu Gly Gly Arg His His Val Val
   1 5 10 15
   gtt att ggc tcc ggc ttc ggt gga cta ttc gca gtt caa aac ctc aaa 96
   Val Ile Gly Ser Gly Phe Gly Gly Leu Phe Ala Val Gln Asn Leu Lys
   20 25 30
   gat gca gat gtc gat atc acc ctc atc gac cgg aca aac cac cac ctt 144
   Asp Ala Asp Val Asp Ile Thr Leu Ile Asp Arg Thr Asn His His Leu
   35 40 45
   ttc cag ccg ttg ctt tac caa gta gca acc ggt atc ttg tcg tct ggt 192
   Phe Gln Pro Leu Leu Tyr Gln Val Ala Thr Gly Ile Leu Ser Ser Gly
   50 55 60
   gaa atc gca cca caa acg cgt caa gtt ctt gca cag caa aat aat gtg 240
   Glu Ile Ala Pro Gln Thr Arg Gln Val Leu Ala Gln Gln Asn Asn Val
   65 70 75 80
   cac gtt ctt aag gct gaa gtc acc gac att gac acc gaa tcg aag acg 288
   His Val Leu Lys Ala Glu Val Thr Asp Ile Asp Thr Glu Ser Lys Thr
   85 90 95
   gtc gtc gca gac ttg gat gat tat tct aaa aca att gaa tac gat tcc 336
   Val Val Ala Asp Leu Asp Asp Tyr Ser Lys Thr Ile Glu Tyr Asp Ser
   100 105 110
   ctg atc gtc gcc gct ggt gca ggt cag tct tac ttc gga aat gat cac 384
   Leu Ile Val Ala Ala Gly Ala Gly Gln Ser Tyr Phe Gly Asn Asp His
   115 120 125
   ttc gcg gaa ttc gcg ccg ggt atg aaa aca atc gat gat gca ctc gaa 432
   Phe Ala Glu Phe Ala Pro Gly Met Lys Thr Ile Asp Asp Ala Leu Glu
   130 135 140
   ctg cgt gcg cgc atc atc ggc gct ttc gaa cgc gca gaa atg tgc gaa 480
   Leu Arg Ala Arg Ile Ile Gly Ala Phe Glu Arg Ala Glu Met Cys Glu
   145 150 155 160
   gat ccc aaa gaa cgt gaa cgc ctc ttg act ttt gtt atc gtt ggc gca 528
   Asp Pro Lys Glu Arg Glu Arg Leu Leu Thr Phe Val Ile Val Gly Ala
   165 170 175
   gga cca aca ggc gta gaa ctt gca ggt cag ctg gcc gaa atg gca cac 576
   Gly Pro Thr Gly Val Glu Leu Ala Gly Gln Leu Ala Glu Met Ala His
   180 185 190
   cgc acg ttg tct gga gag tac acg cag ttc acg cct tcc aac gcg aag 624
   Arg Thr Leu Ser Gly Glu Tyr Thr Gln Phe Thr Pro Ser Asn Ala Lys
   195 200 205
   atc atc ctg ctt gac ggc gct cct cag gtg ctt cca ccg ttc ggc aag 672
   Ile Ile Leu Leu Asp Gly Ala Pro Gln Val Leu Pro Pro Phe Gly Lys
   210 215 220
   cgt ttg ggt cgt act gca cag cgt gaa tta gaa aag att ggt gta acg 720
   Arg Leu Gly Arg Thr Ala Gln Arg Glu Leu Glu Lys Ile Gly Val Thr
   225 230 235 240
   gtc aag ctg aac gct atc gtt acc ggc gta gac gaa aac tca gtg aca 768
   Val Lys Leu Asn Ala Ile Val Thr Gly Val Asp Glu Asn Ser Val Thr
   245 250 255
   tat aag tcc acc gtt gat gat tct ttg cac acg atc gat tcc ttc tgc 816
   Tyr Lys Ser Thr Val Asp Asp Ser Leu His Thr Ile Asp Ser Phe Cys
   260 265 270
   aag atc tgg tcg gca ggc gta gcc gct tcc cca cta ggc aaa cta gtt 864
   Lys Ile Trp Ser Ala Gly Val Ala Ala Ser Pro Leu Gly Lys Leu Val
   275 280 285
   gca gag cag ctt ggt gtc gag gtt gat cgt gca gga cgc gtc cca gtc 912
   Ala Glu Gln Leu Gly Val Glu Val Asp Arg Ala Gly Arg Val Pro Val
   290 295 300
   aac gaa gat ctt tct gtt ggc gac gat aag aac gtc ttt gtt att ggc 960
   Asn Glu Asp Leu Ser Val Gly Asp Asp Lys Asn Val Phe Val Ile Gly
   305 310 315 320
   gat atg atg tcg ctc aac agg ctt ccg gga gtc gca cag gta gca atc
   1008
   Asp Met Met Ser Leu Asn Arg Leu Pro Gly Val Ala Gln Val Ala Ile
   325 330 335
   caa ggc ggt gaa tac gtt gct gag cag att gct gct ggg gtc gag gga
   1056
   Gln Gly Gly Glu Tyr Val Ala Glu Gln Ile Ala Ala Gly Val Glu Gly
   340 345 350
   cgt tcc tcg tct gaa cgc cca gcc ttt gaa tac tac gac aag ggt tcg
   1104
   Arg Ser Ser Ser Glu Arg Pro Ala Phe Glu Tyr Tyr Asp Lys Gly Ser
   355 360 365
   atg gct acg gtg tcg cgc ttt aac gcc gtt gtg aag ctt gga aaa gtt
   1152
   Met Ala Thr Val Ser Arg Phe Asn Ala Val Val Lys Leu Gly Lys Val
   370 375 380
   gaa gtt acg gga ttt att ggc tgg gtc atg tgg ctt ctc gtc cac ttg
   1200
   Glu Val Thr Gly Phe Ile Gly Trp Val Met Trp Leu Leu Val His Leu
   385 390 395 400
   atg ttc ttg gtc ggc ttc cgc aac cga gca act gcg gct ttc tct tgg
   1248
   Met Phe Leu Val Gly Phe Arg Asn Arg Ala Thr Ala Ala Phe Ser Trp
   405 410 415
   ggc atc aat gcg ctt tca cgt aag cgt tgg aac ctc gcc acc act cgt
   1296
   Gly Ile Asn Ala Leu Ser Arg Lys Arg Trp Asn Leu Ala Thr Thr Arg
   420 425 430
   cag cag ctt cac ggc cgt act ggt ttg caa aaa ctt act gcg ctc gtc
   1344
   Gln Gln Leu His Gly Arg Thr Gly Leu Gln Lys Leu Thr Ala Leu Val
   435 440 445
   gat acc gcc gaa aag aag 1362
   Asp Thr Ala Glu Lys Lys
   450
<210> 6
   <211> 454
   <212> PRT
   <213> Corynebacterium diphtheriae
<400> 6
   Met Thr Asn Thr Pro Phe Arg Pro Glu Gly Gly Arg His His Val Val
   1 5 10 15
   Val Ile Gly Ser Gly Phe Gly Gly Leu Phe Ala Val Gln Asn Leu Lys
   20 25 30
   Asp Ala Asp Val Asp Ile Thr Leu Ile Asp Arg Thr Asn His His Leu
   35 40 45
   Phe Gln Pro Leu Leu Tyr Gln Val Ala Thr Gly Ile Leu Ser Ser Gly
   50 55 60
   Glu Ile Ala Pro Gln Thr Arg Gln Val Leu Ala Gln Gln Asn Asn Val
   65 70 75 80
   His Val Leu Lys Ala Glu Val Thr Asp Ile Asp Thr Glu Ser Lys Thr
   85 90 95
   Val Val Ala Asp Leu Asp Asp Tyr Ser Lys Thr Ile Glu Tyr Asp Ser
   100 105 110
   Leu Ile Val Ala Ala Gly Ala Gly Gln Ser Tyr Phe Gly Asn Asp His
   115 120 125
   Phe Ala Glu Phe Ala Pro Gly Met Lys Thr Ile Asp Asp Ala Leu Glu
   130 135 140
   Leu Arg Ala Arg Ile Ile Gly Ala Phe Glu Arg Ala Glu Met Cys Glu
   145 150 155 160
   Asp Pro Lys Glu Arg Glu Arg Leu Leu Thr Phe Val Ile Val Gly Ala
   165 170 175
   Gly Pro Thr Gly Val Glu Leu Ala Gly Gln Leu Ala Glu Met Ala His
   180 185 190
   Arg Thr Leu Ser Gly Glu Tyr Thr Gln Phe Thr Pro Ser Asn Ala Lys
   195 200 205
   Ile Ile Leu Leu Asp Gly Ala Pro Gln Val Leu Pro Pro Phe Gly Lys
   210 215 220
   Arg Leu Gly Arg Thr Ala Gln Arg Glu Leu Glu Lys Ile Gly Val Thr
   225 230 235 240
   Val Lys Leu Asn Ala Ile Val Thr Gly Val Asp Glu Asn Ser Val Thr
   245 250 255
   Tyr Lys Ser Thr Val Asp Asp Ser Leu His Thr Ile Asp Ser Phe Cys
   260 265 270
   Lys Ile Trp Ser Ala Gly Val Ala Ala Ser Pro Leu Gly Lys Leu Val
   275 280 285
   Ala Glu Gln Leu Gly Val Glu Val Asp Arg Ala Gly Arg Val Pro Val
   290 295 300
   Asn Glu Asp Leu Ser Val Gly Asp Asp Lys Asn Val Phe Val Ile Gly
   305 310 315 320
   Asp Met Met Ser Leu Asn Arg Leu Pro Gly Val Ala Gln Val Ala Ile
   325 330 335
   Gln Gly Gly Glu Tyr Val Ala Glu Gln Ile Ala Ala Gly Val Glu Gly
   340 345 350
   Arg Ser Ser Ser Glu Arg Pro Ala Phe Glu Tyr Tyr Asp Lys Gly Ser
   355 360 365
   Met Ala Thr Val Ser Arg Phe Asn Ala Val Val Lys Leu Gly Lys Val
   370 375 380
   Glu Val Thr Gly Phe Ile Gly Trp Val Met Trp Leu Leu Val His Leu
   385 390 395 400
   Met Phe Leu Val Gly Phe Arg Asn Arg Ala Thr Ala Ala Phe Ser Trp
   405 410 415
   Gly Ile Asn Ala Leu Ser Arg Lys Arg Trp Asn Leu Ala Thr Thr Arg
   420 425 430
   Gln Gln Leu His Gly Arg Thr Gly Leu Gln Lys Leu Thr Ala Leu Val
   435 440 445
   Asp Thr Ala Glu Lys Lys
   450
<210> 7
   <211> 1302
   <212> DNA
   <213> Escherichia coli
<220>
   <221> CDS
   <222>
<400> 7
   ttg act acg cca ttg aaa aag att gtg att gtc ggc ggc ggt gct ggt      48
   Met Thr Thr Pro Leu Lys Lys Ile Val Ile Val Gly Gly Gly Ala Gly
   1               5                   10                  15
   ggg ctg gaa atg gca aca cag ctg ggg cat aag ctg gga cgc aag aaa      96
   Gly Leu Glu Met Ala Thr Gln Leu Gly His Lys Leu Gly Arg Lys Lys
               20                  25                  30
   aaa gcc aaa att acg ctg gtc gat cgt aac cac agc cac ctg tgg aaa     144
   Lys Ala Lys Ile Thr Leu Val Asp Arg Asn His Ser His Leu Trp Lys
           35                  40                  45
   ccg ctg ctg cac gaa gtg gcg act ggc tcg ctt gat gaa ggc gtc gat     192
   Pro Leu Leu His Glu Val Ala Thr Gly Ser Leu Asp Glu Gly Val Asp
       50                  55                  60
   gcg ttg agc tat ctg gcc cat gcg cgc aat cat ggt ttc cag ttc cag     240
   Ala Leu Ser Tyr Leu Ala His Ala Arg Asn His Gly Phe Gln Phe Gln
   65                  70                  75                  80
   ctg ggt tcc gtc att gat att gat cgt gaa gcg aaa aca atc act att     288
   Leu Gly Ser Val Ile Asp Ile Asp Arg Glu Ala Lys Thr Ile Thr Ile
                   85                  90                  95
   gca gaa ctg cgc gac gag aaa ggt gaa ctg ctg gtt ccg gaa cgt aaa     336
   Ala Glu Leu Arg Asp Glu Lys Gly Glu Leu Leu Val Pro Glu Arg Lys
               100                 105                 110
   atc gcc tat gac acc ctg gta atg gcg ctg ggt agc acc tct aac gat     384
   Ile Ala Tyr Asp Thr Leu Val Met Ala Leu Gly Ser Thr Ser Asn Asp
           115                 120                 125
   ttc aat acg cca ggt gtc aaa gag aac tgc att ttc ctc gat aac ccg     432
   Phe Asn Thr Pro Gly Val Lys Glu Asn Cys Ile Phe Leu Asp Asn Pro
       130                 135                 140
   cac cag gcg cgt cgc ttc cac cag gag atg ctg aat ttg ttc ctg aaa     480
   His Gln Ala Arg Arg Phe His Gln Glu Met Leu Asn Leu Phe Leu Lys
   145                 150                 155                 160
   tac tcc gcc aac ctg ggc gcg aat ggc aaa gtg aac att gcg att gtc 528
   Tyr Ser Ala Asn Leu Gly Ala Asn Gly Lys Val Asn Ile Ala Ile Val
   165 170 175
   ggc ggc ggc gcg acg ggt gta gaa ctc tcc gct gaa ttg cac aac gcg 576
   Gly Gly Gly Ala Thr Gly Val Glu Leu Ser Ala Glu Leu His Asn Ala
   180 185 190
   gtc aag caa ctg cac agc tac ggt tac aaa ggc ctg acc aac gaa gcc 624
   Val Lys Gln Leu His Ser Tyr Gly Tyr Lys Gly Leu Thr Asn Glu Ala
   195 200 205
   ctg aac gta acg ctg gta gaa gcg gga gaa cgt att ttg cct gcg tta 672
   Leu Asn Val Thr Leu Val Glu Ala Gly Glu Arg Ile Leu Pro Ala Leu
   210 215 220
   ccg cca cgt atc tct gct gcg gcc cac aac gag cta acg aaa ctt ggc 720
   Pro Pro Arg Ile Ser Ala Ala Ala His Asn Glu Leu Thr Lys Leu Gly
   225 230 235 240
   gtt cgc gtg ctg acg caa acc atg gtc acc agt gct gat gaa ggc ggc 768
   Val Arg Val Leu Thr Gln Thr Met Val Thr Ser Ala Asp Glu Gly Gly
   245 250 255
   ctg cac act aaa gat ggc gaa tat att gag gct gat ctg atg gta tgg 816
   Leu His Thr Lys Asp Gly Glu Tyr Ile Glu Ala Asp Leu Met Val Trp
   260 265 270
   gca gcc ggg atc aaa gcg cca gac ttc ctg aaa gat atc ggt ggt ctt 864
   Ala Ala Gly Ile Lys Ala Pro Asp Phe Leu Lys Asp Ile Gly Gly Leu
   275 280 285
   gaa act aac cgt atc aac cag ctg gtg gtg gaa ccg acg ctg caa acc 912
   Glu Thr Asn Arg Ile Asn Gln Leu Val Val Glu Pro Thr Leu Gln Thr
   290 295 300
   acc cgc gat cca gac att tac gct att ggc gac tgc gcg tca tgc ccg 960
   Thr Arg Asp Pro Asp Ile Tyr Ala Ile Gly Asp Cys Ala Ser Cys Pro
   305 310 315 320
   cgt ccg gaa ggg ggc ttt gtt ccg ccg cgt gct cag gct gca cac cag
   1008
   Arg Pro Glu Gly Gly Phe Val Pro Pro Arg Ala Gln Ala Ala His Gln
   325 330 335
   atg gcg act tgc gca atg aac aac att ctg gcg cag atg aac ggt aag
   1056
   Met Ala Thr Cys Ala Met Asn Asn Ile Leu Ala Gln Met Asn Gly Lys
   340 345 350
   ccg ctg aaa aat tat cag tat aaa gat cat ggt tcg ctg gta tcg ctg 1104
   Pro Leu Lys Asn Tyr Gln Tyr Lys Asp His Gly Ser Leu Val Ser Leu
   355 360 365
   tcg aac ttc tcc acc gtc ggt agc ctg atg ggt aac ctg acg cgc ggc 1152
   Ser Asn Phe Ser Thr Val Gly Ser Leu Met Gly Asn Leu Thr Arg Gly
   370 375 380
   tca atg atg att gaa gga cga att gcg cgc ttt gta tat atc tcg cta 1200
   Ser Met Met Ile Glu Gly Arg Ile Ala Arg Phe Val Tyr Ile Ser Leu
   385 390 395 400
   tac cga atg cat cag att gcg ctg cat ggt tac ttt aaa acc gga tta 1248
   Tyr Arg Met His Gln Ile Ala Leu His Gly Tyr Phe Lys Thr Gly Leu
   405 410 415
   atg atg ctg gtg ggg agt att aac cgc gtt atc cgt ccg cgt ttg aag 1296
   Met Met Leu Val Gly Ser Ile Asn Arg Val Ile Arg Pro Arg Leu Lys
   420 425 430
   ttg cat 1302
   Leu His
<210> 8
   <211> 434
   <212> PRT
   <213> Escherichia coli
<400> 8
   Met Thr Thr Pro Leu Lys Lys Ile Val Ile Val Gly Gly Gly Ala Gly
   1 5 10 15
   Gly Leu Glu Met Ala Thr Gln Leu Gly His Lys Leu Gly Arg Lys Lys
   20 25 30
   Lys Ala Lys Ile Thr Leu Val Asp Arg Asn His Ser His Leu Trp Lys
   35 40 45
   Pro Leu Leu His Glu Val Ala Thr Gly Ser Leu Asp Glu Gly Val Asp
   50 55 60
   Ala Leu Ser Tyr Leu Ala His Ala Arg Asn His Gly Phe Gln Phe Gln
   65 70 75 80
   Leu Gly Ser Val Ile Asp Ile Asp Arg Glu Ala Lys Thr Ile Thr Ile
   85 90 95
   Ala Glu Leu Arg Asp Glu Lys Gly Glu Leu Leu Val Pro Glu Arg Lys
   100 105 110
   Ile Ala Tyr Asp Thr Leu Val Met Ala Leu Gly Ser Thr Ser Asn Asp
   115 120 125
   Phe Asn Thr Pro Gly Val Lys Glu Asn Cys Ile Phe Leu Asp Asn Pro
   130 135 140
   His Gln Ala Arg Arg Phe His Gln Glu Met Leu Asn Leu Phe Leu Lys
   145 150 155 160
   Tyr Ser Ala Asn Leu Gly Ala Asn Gly Lys Val Asn Ile Ala Ile Val
   165 170 175
   Gly Gly Gly Ala Thr Gly Val Glu Leu Ser Ala Glu Leu His Asn Ala
   180 185 190
   Val Lys Gln Leu His Ser Tyr Gly Tyr Lys Gly Leu Thr Asn Glu Ala
   195 200 205
   Leu Asn Val Thr Leu Val Glu Ala Gly Glu Arg Ile Leu Pro Ala Leu
   210 215 220
   Pro Pro Arg Ile Ser Ala Ala Ala His Asn Glu Leu Thr Lys Leu Gly
   225 230 235 240
   Val Arg Val Leu Thr Gln Thr Met Val Thr Ser Ala Asp Glu Gly Gly
   245 250 255
   Leu His Thr Lys Asp Gly Glu Tyr Ile Glu Ala Asp Leu Met Val Trp
   260 265 270
   Ala Ala Gly Ile Lys Ala Pro Asp Phe Leu Lys Asp Ile Gly Gly Leu
   275 280 285
   Glu Thr Asn Arg Ile Asn Gln Leu Val Val Glu Pro Thr Leu Gln Thr
   290 295 300
   Thr Arg Asp Pro Asp Ile Tyr Ala Ile Gly Asp Cys Ala Ser Cys Pro
   305 310 315 320
   Arg Pro Glu Gly Gly Phe Val Pro Pro Arg Ala Gln Ala Ala His Gln
   325 330 335
   Met Ala Thr Cys Ala Met Asn Asn Ile Leu Ala Gln Met Asn Gly Lys
   340 345 350
   Pro Leu Lys Asn Tyr Gln Tyr Lys Asp His Gly Ser Leu Val Ser Leu
   355 360 365
   Ser Asn Phe Ser Thr Val Gly Ser Leu Met Gly Asn Leu Thr Arg Gly
   370 375 380
   Ser Met Met Ile Glu Gly Arg Ile Ala Arg Phe Val Tyr Ile Ser Leu
   385 390 395 400
   Tyr Arg Met His Gln Ile Ala Leu His Gly Tyr Phe Lys Thr Gly Leu
   405 410 415
   Met Met Leu Val Gly Ser Ile Asn Arg Val Ile Arg Pro Arg Leu Lys
   420 425 430
   Leu His
<210> 9
<211> 1296
   <212> DNA
   <213> Pseudomonas fluorescens
<220>
   <221> CDS
   <222>
<400> 9
   atg act cat cgt att gtc atc gtt ggc ggc ggc gcc ggc ggt ctg gag 48
   Met Thr His Arg Ile Val Ile Val Gly Gly Gly Ala Gly Gly Leu Glu
   1 5 10 15
   ttg gct acc cgt ctg ggt aag act ctg ggc aag cgt ggc acg gcc agt 96
   Leu Ala Thr Arg Leu Gly Lys Thr Leu Gly Lys Arg Gly Thr Ala Ser
   20 25 30
   gtg atg ctg gtc gac gcg aac ctg acc cac atc tgg aaa ccg cta ctg 144
   Val Met Leu Val Asp Ala Asn Leu Thr His Ile Trp Lys Pro Leu Leu
   35 40 45
   cac gaa gtg gcc gcc ggc tcc ttg aac tcc tcc gaa gac gaa ctc aac 192
   His Glu Val Ala Ala Gly Ser Leu Asn Ser Ser Glu Asp Glu Leu Asn
   50 55 60
   tat gtc gcc cag gca aaa tgg aac cac ttc gag ttc cag ctc ggg cgc 240
   Tyr Val Ala Gln Ala Lys Trp Asn His Phe Glu Phe Gln Leu Gly Arg
   65 70 75 80
   atg agc ggc ctg gat cgc gag cgc aag aga atc caa ctg gcc gcc acc 288
   Met Ser Gly Leu Asp Arg Glu Arg Lys Arg Ile Gln Leu Ala Ala Thr
   85 90 95
   tat gac gag acc ggc gtc gag ctg ttg ccg gcc cgg gaa ctg ggc tac 336
   Tyr Asp Glu Thr Gly Val Glu Leu Leu Pro Ala Arg Glu Leu Gly Tyr
   100 105 110
   gac acc ctg gtg att gcc gtc ggc agc acc acc aat gac ttc ggc acc 384
   Asp Thr Leu Val Ile Ala Val Gly Ser Thr Thr Asn Asp Phe Gly Thr
   115 120 125
   gaa ggc gcg gcg cag cac tgc ctg ttc ctc gac acc cgc aaa cag gcc 432
   Glu Gly Ala Ala Gln His Cys Leu Phe Leu Asp Thr Arg Lys Gln Ala
   130 135 140
   gag cgc ttc cat cag caa ttg ctg cac cac tat ctg cgc gcc cac gcc 480
   Glu Arg Phe His Gln Gln Leu Leu His His Tyr Leu Arg Ala His Ala
   145 150 155 160
   ggg cag acc gat atc gtc gag cgc atc agc gtc gcc att gtc ggc gcc 528
   Gly Gln Thr Asp Ile Val Glu Arg Ile Ser Val Ala Ile Val Gly Ala
   165 170 175
   ggt gcg acc ggg gtc gaa ctg gcc gcc gag ctg cat aat gcc gcc cac 576
   Gly Ala Thr Gly Val Glu Leu Ala Ala Glu Leu His Asn Ala Ala His
   180 185 190
   gaa ctg cac gcc tac ggc ctg gac cgg atc aaa ccg gag aac atg cac 624
   Glu Leu His Ala Tyr Gly Leu Asp Arg Ile Lys Pro Glu Asn Met His
   195 200 205
   atc acc ctg atc gag gcc ggg cca cgc gtc ttg ccg gcc ctg ccg gag 672
   Ile Thr Leu Ile Glu Ala Gly Pro Arg Val Leu Pro Ala Leu Pro Glu
   210 215 220
   cgt atc ggc ggg ccg gtg cac aag acc ctg gaa aaa ctc ggg gtc aac 720
   Arg Ile Gly Gly Pro Val His Lys Thr Leu Glu Lys Leu Gly Val Asn
   225 230 235 240
   gtc atg acc aac gcc gcc gtc agc cag gtg acc gcc gac agc ctg att 768
   Val Met Thr Asn Ala Ala Val Ser Gln Val Thr Ala Asp Ser Leu Ile
   245 250 255
   acc gcg gac ggc aaa gtg atc gac gcg agc ctg aaa gtc tgg gcc gcc 816
   Thr Ala Asp Gly Lys Val Ile Asp Ala Ser Leu Lys Val Trp Ala Ala
   260 265 270
   ggg att cgc gcc ccg gac ttc ctc aag gac atc gac ggg ctg gag acc 864
   Gly Ile Arg Ala Pro Asp Phe Leu Lys Asp Ile Asp Gly Leu Glu Thr
   275 280 285
   aac cgg atc aac cag ttg cac gtg ctg ccc act ttg cag acc acc cgc 912
   Asn Arg Ile Asn Gln Leu His Val Leu Pro Thr Leu Gln Thr Thr Arg
   290 295 300
   gac gag aac atc ttc gcc ttc ggc gac tgc gcc gcc tgc ccg caa ccc 960
   Asp Glu Asn Ile Phe Ala Phe Gly Asp Cys Ala Ala Cys Pro Gln Pro
   305 310 315 320
   ggc agc gag cgc aac gtc cca cct cgc gcc cag gcc gca cac cag caa
   1008
   Gly Ser Glu Arg Asn Val Pro Pro Arg Ala Gln Ala Ala His Gln Gln
   325 330 335
   gcc tca ctg ctg gcc aaa tcc ttg aag ctg cgg atc gag ggc aag gcc
   1056
   Ala Ser Leu Leu Ala Lys Ser Leu Lys Leu Arg Ile Glu Gly Lys Ala
   340 345 350
   ctg ccg gaa tac aaa tac acc gac tac ggc tcg ctg atc tcg ctg tcg
   1104
   Leu Pro Glu Tyr Lys Tyr Thr Asp Tyr Gly Ser Leu Ile Ser Leu Ser
   355 360 365
   cgg ttc tcg gca gtg ggc aac ctg atg ggt aac ctg acc ggc agc gtg
   1152
   Arg Phe Ser Ala Val Gly Asn Leu Met Gly Asn Leu Thr Gly Ser Val
   370 375 380
   atg ctc gaa ggc tgg ctg gcg cgg atg ttc tat gtg tcg ctg tac cgc
   1200
   Met Leu Glu Gly Trp Leu Ala Arg Met Phe Tyr Val Ser Leu Tyr Arg
   385 390 395 400
   atg cac cag atg gcg ctg tac ggc atg ttc cgc acg gcc atg ttg atg
   1248
   Met His Gln Met Ala Leu Tyr Gly Met Phe Arg Thr Ala Met Leu Met
   405 410 415
   ctg ggt agc aag atc ggg cgt ggg acc gag cct cgg ctg aag ctg cac
   1296
   Leu Gly Ser Lys Ile Gly Arg Gly Thr Glu Pro Arg Leu Lys Leu His
   420 425 430
<210> 10
   <211> 432
   <212> PRT
   <213> Pseudomonas fluorescens
<400> 10
   Met Thr His Arg Ile Val Ile Val Gly Gly Gly Ala Gly Gly Leu Glu
   1 5 10 15
   Leu Ala Thr Arg Leu Gly Lys Thr Leu Gly Lys Arg Gly Thr Ala Ser
   20 25 30
   Val Met Leu Val Asp Ala Asn Leu Thr His Ile Trp Lys Pro Leu Leu
   35 40 45
   His Glu Val Ala Ala Gly Ser Leu Asn Ser Ser Glu Asp Glu Leu Asn
   50 55 60
   Tyr Val Ala Gln Ala Lys Trp Asn His Phe Glu Phe Gln Leu Gly Arg
   65 70 75 80
   Met Ser Gly Leu Asp Arg Glu Arg Lys Arg Ile Gln Leu Ala Ala Thr
   85 90 95
   Tyr Asp Glu Thr Gly Val Glu Leu Leu Pro Ala Arg Glu Leu Gly Tyr
   100 105 110
   Asp Thr Leu Val Ile Ala Val Gly Ser Thr Thr Asn Asp Phe Gly Thr
   115 120 125
   Glu Gly Ala Ala Gln His Cys Leu Phe Leu Asp Thr Arg Lys Gln Ala
   130 135 140
   Glu Arg Phe His Gln Gln Leu Leu His His Tyr Leu Arg Ala His Ala
   145 150 155 160
   Gly Gln Thr Asp Ile Val Glu Arg Ile Ser Val Ala Ile Val Gly Ala
   165 170 175
   Gly Ala Thr Gly Val Glu Leu Ala Ala Glu Leu His Asn Ala Ala His
   180 185 190
   Glu Leu His Ala Tyr Gly Leu Asp Arg Ile Lys Pro Glu Asn Met His
   195 200 205
   Ile Thr Leu Ile Glu Ala Gly Pro Arg Val Leu Pro Ala Leu Pro Glu
   210 215 220
   Arg Ile Gly Gly Pro Val His Lys Thr Leu Glu Lys Leu Gly Val Asn
   225 230 235 240
   Val Met Thr Asn Ala Ala Val Ser Gln Val Thr Ala Asp Ser Leu Ile
   245 250 255
   Thr Ala Asp Gly Lys Val Ile Asp Ala Ser Leu Lys Val Trp Ala Ala
   260 265 270
   Gly Ile Arg Ala Pro Asp Phe Leu Lys Asp Ile Asp Gly Leu Glu Thr
   275 280 285
   Asn Arg Ile Asn Gln Leu His Val Leu Pro Thr Leu Gln Thr Thr Arg
   290 295 300
   Asp Glu Asn Ile Phe Ala Phe Gly Asp Cys Ala Ala Cys Pro Gln Pro
   305 310 315 320
   Gly Ser Glu Arg Asn Val Pro Pro Arg Ala Gln Ala Ala His Gln Gln
   325 330 335
   Ala Ser Leu Leu Ala Lys Ser Leu Lys Leu Arg Ile Glu Gly Lys Ala
   340 345 350
   Leu Pro Glu Tyr Lys Tyr Thr Asp Tyr Gly Ser Leu Ile Ser Leu Ser
   355 360 365
   Arg Phe Ser Ala Val Gly Asn Leu Met Gly Asn Leu Thr Gly Ser Val
   370 375 380
   Met Leu Glu Gly Trp Leu Ala Arg Met Phe Tyr Val Ser Leu Tyr Arg
   385 390 395 400
   Met His Gln Met Ala Leu Tyr Gly Met Phe Arg Thr Ala Met Leu Met
   405 410 415
   Leu Gly Ser Lys Ile Gly Arg Gly Thr Glu Pro Arg Leu Lys Leu His
   420 425 430
<210> 11
   <211> 1296
   <212> DNA
   <213> Azotobacter vinelandii
<220>
   <221> CDS
   <222>
<400> 11
   atg act cat cgt atc gta atc gtc ggc ggt ggc gct ggc ggc gtg gaa    48
   Met Thr His Arg Ile Val Ile Val Gly Gly Gly Ala Gly Gly Val Glu
    1               5                  10                  15
   ctc gct acc cgc ctc ggc aag acc atg ggc agg aac ttc cag gcg aag    96
   Leu Ala Thr Arg Leu Gly Lys Thr Met Gly Arg Asn Phe Gln Ala Lys
               20                  25                  30
   atc acc ctg gtc gac gcc aac atg acc cac ctg tgg aaa ccg ctg ctg   144
   Ile Thr Leu Val Asp Ala Asn Met Thr His Leu Trp Lys Pro Leu Leu
   35 40 45
   cac gaa gtc gcc gcc ggc tcg ctg aac tcg acc ggc gac gaa ctg aac 192
   His Glu Val Ala Ala Gly Ser Leu Asn Ser Thr Gly Asp Glu Leu Asn
   50 55 60
   tat gtg gcc cag gcc aaa tgg aac aac ttc gag ttc cag tac ggc cgc 240
   Tyr Val Ala Gln Ala Lys Trp Asn Asn Phe Glu Phe Gln Tyr Gly Arg
   65 70 75 80
   atg tgc ggt ctg gac cgg gcc aac aag cgt atc cgc ctg gcg gcc cag 288
   Met Cys Gly Leu Asp Arg Ala Asn Lys Arg Ile Arg Leu Ala Ala Gln
   85 90 95
   ccg gcc cag gaa gat cgc gcg ccc ctg ccc gag cgc gaa ctg gaa tac 336
   Pro Ala Gln Glu Asp Arg Ala Pro Leu Pro Glu Arg Glu Leu Glu Tyr
   100 105 110
   gac acc ctg gtc ctt tcc gtc ggc agc acc acc aat gac ttc ggc acc 384
   Asp Thr Leu Val Leu Ser Val Gly Ser Thr Thr Asn Asp Phe Gly Thr
   115 120 125
   ccg ggc gcc gcc gag aac tgt att ttc ctg gaa ggc cgc gac cag gcc 432
   Pro Gly Ala Ala Glu Asn Cys Ile Phe Leu Glu Gly Arg Asp Gln Ala
   130 135 140
   gag cgt ttc cgc cgt ccg ctg ctc agc cac tac ttg cgt gcc cac gcc 480
   Glu Arg Phe Arg Arg Pro Leu Leu Ser His Tyr Leu Arg Ala His Ala
   145 150 155 160
   agc aat gac gac ggc cat cag gtc aag gtc gcc atc gtc ggc gcc ggt 528
   Ser Asn Asp Asp Gly His Gln Val Lys Val Ala Ile Val Gly Ala Gly
   165 170 175
   gcc acc ggg gtc gaa ctg gcc gca gaa ctg cgc cac gcc tcc aag gaa 576
   Ala Thr Gly Val Glu Leu Ala Ala Glu Leu Arg His Ala Ser Lys Glu
   180 185 190
   ctg gtc gcc tat ggg ctg gag cgc att ccg ccg gag aac ctc agc atc 624
   Leu Val Ala Tyr Gly Leu Glu Arg Ile Pro Pro Glu Asn Leu Ser Ile
   195 200 205
   acg ctg atc gaa tcc agc ccg cgt gta ctc gcc gcc ctg ccc gaa cgc 672
   Thr Leu Ile Glu Ser Ser Pro Arg Val Leu Ala Ala Leu Pro Glu Arg
   210 215 220
   atc agc cgc tcc gcg cac gcc acc ctg gaa agc ctg ggc gtt cgc gtg 720
   Ile Ser Arg Ser Ala His Ala Thr Leu Glu Ser Leu Gly Val Arg Val
   225 230 235 240
   ctc gtc agc acc gcc gtc agc gag gtc acc gcg gaa ggc gtg aag acg     768
   Leu Val Ser Thr Ala Val Ser Glu Val Thr Ala Glu Gly Val Lys Thr
                   245                 250                 255
   aag gac gac cag ttc atc ccc gcc gac ctc atg gtc tgg gcc gca ggc     816
   Lys Asp Asp Gln Phe Ile Pro Ala Asp Leu Met Val Trp Ala Ala Gly
               260                 265                 270
   gtc cgc gcg ccc gcc ttc ctc aag gag ctg gat ggt ctg gaa acc aat     864
   Val Arg Ala Pro Ala Phe Leu Lys Glu Leu Asp Gly Leu Glu Thr Asn
           275                 280                 285
   cgc atc aac cag ttg cag gtc cgc cag acc ctg cag act act ctg gac     912
   Arg Ile Asn Gln Leu Gln Val Arg Gln Thr Leu Gln Thr Thr Leu Asp
       290                 295                 300
   gac gat atc ttc gcc ttc ggc gat tgt gcc tcc tgc ccg cag ccg ggt     960
   Asp Asp Ile Phe Ala Phe Gly Asp Cys Ala Ser Cys Pro Gln Pro Gly
   305                 310                 315                 320
   acc gac cgc ccc gtt ccg ccg cgc gcc cag gcc gct cac cag cag gcc
   1008
   Thr Asp Arg Pro Val Pro Pro Arg Ala Gln Ala Ala His Gln Gln Ala
                   325                 330                 335
   agc ctg ctg gcc aag tcg ctc cac cgc aag ctg cag gaa gac agc ctc
   1056
   Ser Leu Leu Ala Lys Ser Leu His Arg Lys Leu Gln Glu Asp Ser Leu
               340                 345                 350
   tgc tgg agt atc gct aca gcg aac cac ggc tcg ctg atc tcc ctc tcg
   1104
   Cys Trp Ser Ile Ala Thr Ala Asn His Gly Ser Leu Ile Ser Leu Ser
           355                 360                 365
   agc ttc tcg gcg atc ggt aac ctg atg ggc aac ctg acc ggc aac gtg
   1152
   Ser Phe Ser Ala Ile Gly Asn Leu Met Gly Asn Leu Thr Gly Asn Val
       370                 375                 380
   acc ttg gaa ggc tgg ctg gcc cgc aag ttc tac att tcc ctg tac cgc
   1200
   Thr Leu Glu Gly Trp Leu Ala Arg Lys Phe Tyr Ile Ser Leu Tyr Arg
   385                 390                 395                 400
   atg cac cag atg gcg ctc tac ggt acc ttc cgc acc ctg atg atg atg
   1248
   Met His Gln Met Ala Leu Tyr Gly Thr Phe Arg Thr Leu Met Met Met
                   405                 410                 415
   ctg ggc gac cgc ttc cgc agc agc acc gaa ccc cgc ctc aag ctt cac 1296
   Leu Gly Asp Arg Phe Arg Ser Ser Thr Glu Pro Arg Leu Lys Leu His
   420 425 430
<210> 12
   <211> 432
   <212> PRT
   <213> Azotobacter vinelandii
<400> 12
   Met Thr His Arg Ile Val Ile Val Gly Gly Gly Ala Gly Gly Val Glu
    1               5                  10                  15
   Leu Ala Thr Arg Leu Gly Lys Thr Met Gly Arg Asn Phe Gln Ala Lys
               20                  25                  30
   Ile Thr Leu Val Asp Ala Asn Met Thr His Leu Trp Lys Pro Leu Leu
           35                  40                  45
   His Glu Val Ala Ala Gly Ser Leu Asn Ser Thr Gly Asp Glu Leu Asn
       50                  55                  60
   Tyr Val Ala Gln Ala Lys Trp Asn Asn Phe Glu Phe Gln Tyr Gly Arg
   65                  70                  75                  80
   Met Cys Gly Leu Asp Arg Ala Asn Lys Arg Ile Arg Leu Ala Ala Gln
                   85                  90                  95
   Pro Ala Gln Glu Asp Arg Ala Pro Leu Pro Glu Arg Glu Leu Glu Tyr
               100                 105                 110
   Asp Thr Leu Val Leu Ser Val Gly Ser Thr Thr Asn Asp Phe Gly Thr
           115                 120                 125
   Pro Gly Ala Ala Glu Asn Cys Ile Phe Leu Glu Gly Arg Asp Gln Ala
       130                 135                 140
   Glu Arg Phe Arg Arg Pro Leu Leu Ser His Tyr Leu Arg Ala His Ala
   145                 150                 155                 160
   Ser Asn Asp Asp Gly His Gln Val Lys Val Ala Ile Val Gly Ala Gly
                   165                 170                 175
   Ala Thr Gly Val Glu Leu Ala Ala Glu Leu Arg His Ala Ser Lys Glu
               180                 185                 190
   Leu Val Ala Tyr Gly Leu Glu Arg Ile Pro Pro Glu Asn Leu Ser Ile
           195                 200                 205
   Thr Leu Ile Glu Ser Ser Pro Arg Val Leu Ala Ala Leu Pro Glu Arg
   210 215 220
   Ile Ser Arg Ser Ala His Ala Thr Leu Glu Ser Leu Gly Val Arg Val
   225 230 235 240
   Leu Val Ser Thr Ala Val Ser Glu Val Thr Ala Glu Gly Val Lys Thr
   245 250 255
   Lys Asp Asp Gln Phe Ile Pro Ala Asp Leu Met Val Trp Ala Ala Gly
   260 265 270
   Val Arg Ala Pro Ala Phe Leu Lys Glu Leu Asp Gly Leu Glu Thr Asn
   275 280 285
   Arg Ile Asn Gln Leu Gln Val Arg Gln Thr Leu Gln Thr Thr Leu Asp
   290 295 300
   Asp Asp Ile Phe Ala Phe Gly Asp Cys Ala Ser Cys Pro Gln Pro Gly
   305 310 315 320
   Thr Asp Arg Pro Val Pro Pro Arg Ala Gln Ala Ala His Gln Gln Ala
   325 330 335
   Ser Leu Leu Ala Lys Ser Leu His Arg Lys Leu Gln Glu Asp Ser Leu
   340 345 350
   Cys Trp Ser Ile Ala Thr Ala Asn His Gly Ser Leu Ile Ser Leu Ser
   355 360 365
   Ser Phe Ser Ala Ile Gly Asn Leu Met Gly Asn Leu Thr Gly Asn Val
   370 375 380
   Thr Leu Glu Gly Trp Leu Ala Arg Lys Phe Tyr Ile Ser Leu Tyr Arg
   385 390 395 400
   Met His Gln Met Ala Leu Tyr Gly Thr Phe Arg Thr Leu Met Met Met
   405 410 415
   Leu Gly Asp Arg Phe Arg Ser Ser Thr Glu Pro Arg Leu Lys Leu His
   420 425 430
<210> 13
   <211> 1302
   <212> DNA
   <213> Salmonella typhimurium LT2
<220>
   <221> CDS
   <222>
<400> 13
   ttg act aca cca tta aaa aag atc gtg att gtc ggc ggc ggc gct ggc 48
   Met Thr Thr Pro Leu Lys Lys Ile Val Ile Val Gly Gly Gly Ala Gly
   1 5 10 15
   ggg ctg gaa atg gcg acg cag tta ggc cat aaa ctg ggg cgc aag aaa 96
   Gly Leu Glu Met Ala Thr Gln Leu Gly His Lys Leu Gly Arg Lys Lys
   20 25 30
   aaa gcg aaa atc acg ctg gta gac aga aat cac agc cat ctg tgg aaa 144
   Lys Ala Lys Ile Thr Leu Val Asp Arg Asn His Ser His Leu Trp Lys
   35 40 45
   cca ttg ctg cac gaa gtg gcg act ggc tct ctg gac gaa ggc gtg gat 192
   Pro Leu Leu His Glu Val Ala Thr Gly Ser Leu Asp Glu Gly Val Asp
   50 55 60
   gcg ctg agc tat ctg gct cat gcg cgt aat cat ggt ttc cag ttc cag 240
   Ala Leu Ser Tyr Leu Ala His Ala Arg Asn His Gly Phe Gln Phe Gln
   65 70 75 80
   ctg ggg tcg gtg atg gat atc gat cgc gaa gcg aaa acc atc acc att 288
   Leu Gly Ser Val Met Asp Ile Asp Arg Glu Ala Lys Thr Ile Thr Ile
   85 90 95
   gcc gag ttg cgt gat gaa aag ggc gaa ctg ctg gtg ccg gag cgc aaa 336
   Ala Glu Leu Arg Asp Glu Lys Gly Glu Leu Leu Val Pro Glu Arg Lys
   100 105 110
   atc gcg tat gac acg ctg gtg atg gcg ctg ggc agc acc tct aat gat 384
   Ile Ala Tyr Asp Thr Leu Val Met Ala Leu Gly Ser Thr Ser Asn Asp
   115 120 125
   ttc aac acg ccg ggc gtg aaa gag cac tgt atc ttc ctc gat aac ccg 432
   Phe Asn Thr Pro Gly Val Lys Glu His Cys Ile Phe Leu Asp Asn Pro
   130 135 140
   cat cag gcg cgc cgt ttt cac cag gag atg ctg aac ctg ttc ctc aag 480
   His Gln Ala Arg Arg Phe His Gln Glu Met Leu Asn Leu Phe Leu Lys
   145 150 155 160
   tat tcg gct aat ctg ggc gcg aac ggt aag gtc aat atc gcc atc gtt 528
   Tyr Ser Ala Asn Leu Gly Ala Asn Gly Lys Val Asn Ile Ala Ile Val
   165 170 175
   ggc ggc ggt gcg acg ggg gtt gag ctg tcg gct gaa ctg cat aat gcg     576
   Gly Gly Gly Ala Thr Gly Val Glu Leu Ser Ala Glu Leu His Asn Ala
               180                 185                 190
   gta aaa cag ctg cat agc tat ggt tat aag ggg ctg acc aac gac gcg     624
   Val Lys Gln Leu His Ser Tyr Gly Tyr Lys Gly Leu Thr Asn Asp Ala
           195                 200                 205
   ctg aac gtg acg ttg gtt gag gct ggc gag cgt att ctg cct gcg ttg     672
   Leu Asn Val Thr Leu Val Glu Ala Gly Glu Arg Ile Leu Pro Ala Leu
       210                 215                 220
   ccg ccg cgt atc tcc agc gcc gcg cat aat gaa ctg acc aaa ctg ggc     720
   Pro Pro Arg Ile Ser Ser Ala Ala His Asn Glu Leu Thr Lys Leu Gly
   225                 230                 235                 240
   gtt cgc gtg ctg acg caa act atg gtc acc agc gcc gac gaa ggc ggt     768
   Val Arg Val Leu Thr Gln Thr Met Val Thr Ser Ala Asp Glu Gly Gly
                   245                 250                 255
   ctg cat acg aaa gaa ggt gaa tat att cag gcc gat ctg atg gtg tgg     816
   Leu His Thr Lys Glu Gly Glu Tyr Ile Gln Ala Asp Leu Met Val Trp
               260                 265                 270
   gcg gcg ggt atc aaa gcg cca gat ttt atg aaa gag att ggt ggt ctg     864
   Ala Ala Gly Ile Lys Ala Pro Asp Phe Met Lys Glu Ile Gly Gly Leu
           275                 280                 285
   gaa acg aac cgc att aac caa ctg gtg gtg gaa ccg acg ctg caa acc     912
   Glu Thr Asn Arg Ile Asn Gln Leu Val Val Glu Pro Thr Leu Gln Thr
       290                 295                 300
   acg cgc gat cct gat att tat gcg att ggc gac tgc gct tcc tgc gca     960
   Thr Arg Asp Pro Asp Ile Tyr Ala Ile Gly Asp Cys Ala Ser Cys Ala
   305                 310                 315                 320
   cgt ccg gag gga ggt ttt gtg ccg cct cgc gct cag gcg gcg cat cag
   1008
   Arg Pro Glu Gly Gly Phe Val Pro Pro Arg Ala Gln Ala Ala His Gln
                   325                 330                 335
   atg gct acc tgc gcg atg aaa aac att ctg gcg cag atg aat ggc aaa
   1056
   Met Ala Thr Cys Ala Met Lys Asn Ile Leu Ala Gln Met Asn Gly Lys
               340                 345                 350
   ccg ctg aaa gct tac cag tat aaa gat cat gga tcg ctg gtc tct ctg
   1104
   Pro Leu Lys Ala Tyr Gln Tyr Lys Asp His Gly Ser Leu Val Ser Leu
           355                 360                 365
   tcc aac ttc tct acc gtg ggt agt ctg atg ggg aac ctg acc cgc ggt
   1152
   Ser Asn Phe Ser Thr Val Gly Ser Leu Met Gly Asn Leu Thr Arg Gly
   370 375 380
   tca atg atg att gaa gga cgt atc gcc cgc ttc gtg tat atc tcg cta
   1200
   Ser Met Met Ile Glu Gly Arg Ile Ala Arg Phe Val Tyr Ile Ser Leu
   385 390 395 400
   tac cgt atg cac cag ata gcg ctg cat gga tac ttt aaa acc ggc ctg
   1248
   Tyr Arg Met His Gln Ile Ala Leu His Gly Tyr Phe Lys Thr Gly Leu
   405 410 415
   atg atg ctg gtg ggc agt att aac cgc gtc att cgt ccg cgc ctg aaa
   1296
   Met Met Leu Val Gly Ser Ile Asn Arg Val Ile Arg Pro Arg Leu Lys
   420 425 430
   ctg cat 1302
   Leu His
<210> 14
   <211> 434
   <212> PRT
   <213> Salmonella typhimurium LT2
<400> 14
<210> 15
   <211> 1908
   <212> DNA
   <213> Lactobacillus plantarum WCFS1
<220>
   <221> CDS
   <222>
<400> 15
   atg gca aag aaa aat att gtc gtt gtc ggt gcg ggg ttt gct ggt gtt 48
   Met Ala Lys Lys Asn Ile Val Val Val Gly Ala Gly Phe Ala Gly Val
   1 5 10 15
   tac gca acc aag aaa ctg tct aag cat ttc aaa aaa aat gca gac gtc 96
   Tyr Ala Thr Lys Lys Leu Ser Lys His Phe Lys Lys Asn Ala Asp Val
   20 25 30
   gag att acg ttg att gac cgg cat tca tac ttc acg tat atg act gaa 144
   Glu Ile Thr Leu Ile Asp Arg His Ser Tyr Phe Thr Tyr Met Thr Glu
   35 40 45
   tta cat gaa gtt gct acc gaa cgg gtg gaa cct gag cat atc caa tat 192
   Leu His Glu Val Ala Thr Glu Arg Val Glu Pro Glu His Ile Gln Tyr
   50 55 60
   gat ttg caa cgg ttg ttc gca cgg cga aaa aac gtt cgt ctc gtg acc 240
   Asp Leu Gln Arg Leu Phe Ala Arg Arg Lys Asn Val Arg Leu Val Thr
   65 70 75 80
   gat acc gtg acg ggc atc gac aaa aag gca caa aca gtt act acc gaa 288
   Asp Thr Val Thr Gly Ile Asp Lys Lys Ala Gln Thr Val Thr Thr Glu
   85 90 95
   cac gga agt tat caa tat gat caa ctt tta att agt ttg ggt ggg gaa 336
   His Gly Ser Tyr Gln Tyr Asp Gln Leu Leu Ile Ser Leu Gly Gly Glu
               100                 105                 110
   tcc aat gac ttt ggg act ccc ggt gtt aag gaa cac ggc ttc gaa ttg      384
   Ser Asn Asp Phe Gly Thr Pro Gly Val Lys Glu His Gly Phe Glu Leu
           115                 120                 125
   tgg tcc ttc gaa caa gcg atg gca ttg cgc gct cac tta tct gca att      432
   Trp Ser Phe Glu Gln Ala Met Ala Leu Arg Ala His Leu Ser Ala Ile
       130                 135                 140
   att cgg cgg ggg gcg gcg gag ctc gac cct gct aag cgc aaa gcc atg      480
   Ile Arg Arg Gly Ala Ala Glu Leu Asp Pro Ala Lys Arg Lys Ala Met
   145                 150                 155                 160
   ttg acc ttt aca gtc tgt ggt tct ggt ttt act ggt tct gaa ctg att      528
   Leu Thr Phe Thr Val Cys Gly Ser Gly Phe Thr Gly Ser Glu Leu Ile
                   165                 170                 175
   ggt gaa tta atc gaa tat cgt gat gtt ttg gct cga gac aac aag ctc      576
   Gly Glu Leu Ile Glu Tyr Arg Asp Val Leu Ala Arg Asp Asn Lys Leu
               180                 185                 190
   gat cca agt gaa atc acg ctc caa ttg gtc gaa gca gcg ccg act att      624
   Asp Pro Ser Glu Ile Thr Leu Gln Leu Val Glu Ala Ala Pro Thr Ile
           195                 200                 205
   att aac atg ctc aac cgg acg caa gcc ggt aag gcc gct aag tac atg      672
   Ile Asn Met Leu Asn Arg Thr Gln Ala Gly Lys Ala Ala Lys Tyr Met
       210                 215                 220
   gaa aaa cat ggt gtc aaa atc atg acg aac tcc atg att acc gaa gtc      720
   Glu Lys His Gly Val Lys Ile Met Thr Asn Ser Met Ile Thr Glu Val
   225                 230                 235                 240
   tgt gaa gac cat gtt aac tta aaa ggc aag gat cca att cca acc tac      768
   Cys Glu Asp His Val Asn Leu Lys Gly Lys Asp Pro Ile Pro Thr Tyr
                   245                 250                 255
   acg tta atc tgg aca gcc ggt gtt cgt gct aat agt atc gtt aaa aag      816
   Thr Leu Ile Trp Thr Ala Gly Val Arg Ala Asn Ser Ile Val Lys Lys
               260                 265                 270
   ttc ggc att gaa act aac ccc cgc ggt ggt cgc ttg atg gcc aat gaa      864
   Phe Gly Ile Glu Thr Asn Pro Arg Gly Gly Arg Leu Met Ala Asn Glu
           275                 280                 285
   ttc atg caa gct aag gat tgt aac aat atc ttc tta gcc ggt gat tca      912
   Phe Met Gln Ala Lys Asp Cys Asn Asn Ile Phe Leu Ala Gly Asp Ser
       290                 295                 300
   acc agc tac caa gaa cct gac caa cca cgg cca gtc cca caa atc gtt 960
   Thr Ser Tyr Gln Glu Pro Asp Gln Pro Arg Pro Val Pro Gln Ile Val
   305 310 315 320
   caa ggg gct gaa gaa acc gca gct aag gcc gtc gaa ggt att att aag
   1008
   Gln Gly Ala Glu Glu Thr Ala Ala Lys Ala Val Glu Gly Ile Ile Lys
   325 330 335
   aac gtt gac cag act gac gtt acg atc aag cca ttt aag ggc gct tat
   1056
   Asn Val Asp Gln Thr Asp Val Thr Ile Lys Pro Phe Lys Gly Ala Tyr
   340 345 350
   caa gca tcc gtc gac tca att ggt tcc aaa tat gcc gtt gca caa gtt
   1104
   Gln Ala Ser Val Asp Ser Ile Gly Ser Lys Tyr Ala Val Ala Gln Val
   355 360 365
   tta gag aag tgg aac gtc tct ggt ttt att gcc gtg ctt tta aaa cac
   1152
   Leu Glu Lys Trp Asn Val Ser Gly Phe Ile Ala Val Leu Leu Lys His
   370 375 380
   gcc atc aac tgg atg tac tac gtt cag att ttc tca ggt tac tac cta
   1200
   Ala Ile Asn Trp Met Tyr Tyr Val Gln Ile Phe Ser Gly Tyr Tyr Leu
   385 390 395 400
   ttc cag tac ttc atg cac gaa ttc ttc cgg act cgt aat aac cgt aac
   1248
   Phe Gln Tyr Phe Met His Glu Phe Phe Arg Thr Arg Asn Asn Arg Asn
   405 410 415
   gtc ttc cgc ggt tgg gtc tca cgg gct ggt aac gta ctc tgg agt gtg
   1296
   Val Phe Arg Gly Trp Val Ser Arg Ala Gly Asn Val Leu Trp Ser Val
   420 425 430
   cca ctg cgg ttc ttc tat ggt gcc atg tgg tta tgg gac tgc tgg act
   1344
   Pro Leu Arg Phe Phe Tyr Gly Ala Met Trp Leu Trp Asp Cys Trp Thr
   435 440 445
   aaa gtt cag gga tct gaa tcc tgg ttc act gac aag tta cgg tta cca
   1392
   Lys Val Gln Gly Ser Glu Ser Trp Phe Thr Asp Lys Leu Arg Leu Pro
   450 455 460
   ttc gaa tgg att acc gtg gcc gca acc agt ggt gcc tct caa gca act
   1440
   Phe Glu Trp Ile Thr Val Ala Ala Thr Ser Gly Ala Ser Gln Ala Thr
   465 470 475 480
   aaa gcc gct gca acc agt ggt gct tct gaa gct gcc acg tca acc gtt
   1488
   Lys Ala Ala Ala Thr Ser Gly Ala Ser Glu Ala Ala Thr Ser Thr Val
   485 490 495
   aaa gcc gct aag ggt gtc ttc agt ctt tca tac atg tac ggt aaa gaa
   1536
   Lys Ala Ala Lys Gly Val Phe Ser Leu Ser Tyr Met Tyr Gly Lys Glu
   500 505 510
   ccc ctg atg gtc ttt gac aaa atg cca cat tgg ttc gaa tca att acc
   1584
   Pro Leu Met Val Phe Asp Lys Met Pro His Trp Phe Glu Ser Ile Thr
   515 520 525
   aag gtc ttc att ccg aac atg caa atg gcc ctc ttc ttc cag aaa ttc
   1632
   Lys Val Phe Ile Pro Asn Met Gln Met Ala Leu Phe Phe Gln Lys Phe
   530 535 540
   atg act tgt gtt gaa atc gtc att gca tta tgt att ttc ttc gga ctc
   1680
   Met Thr Cys Val Glu Ile Val Ile Ala Leu Cys Ile Phe Phe Gly Leu
   545 550 555 560
   ttt act tgg ttt gct aac gca gtc acc att ggc tta gtt gtc gtc ttc
   1728
   Phe Thr Trp Phe Ala Asn Ala Val Thr Ile Gly Leu Val Val Val Phe
   565 570 575
   tgc tta tct ggc atg ttc tac tgg gtc aac att tgg atg atc ttt gtt
   1776
   Cys Leu Ser Gly Met Phe Tyr Trp Val Asn Ile Trp Met Ile Phe Val
   580 585 590
   gcc ctc gcc tta atg aac ggt tcc gga cgg aca ttt ggg tta gac tac
   1824
   Ala Leu Ala Leu Met Asn Gly Ser Gly Arg Thr Phe Gly Leu Asp Tyr
   595 600 605
   tgg gtc gtt ccg tgg atg caa aaa cac ctt gga cac tgg tgg tac ggc
   1872
   Trp Val Val Pro Trp Met Gln Lys His Leu Gly His Trp Trp Tyr Gly
   610 615 620
   aac gtt cgt tct cat tac gac ggt gtt aaa acc cgc 1908
   Asn Val Arg Ser His Tyr Asp Gly Val Lys Thr Arg
   625 630 635
<210> 16
   <211> 636
   <212> PRT
   <213> Lactobacillus plantarum WCFS1
<400> 16
   Met Ala Lys Lys Asn Ile Val Val Val Gly Ala Gly Phe Ala Gly Val
   1 5 10 15
   Tyr Ala Thr Lys Lys Leu Ser Lys His Phe Lys Lys Asn Ala Asp Val
   20 25 30
   Glu Ile Thr Leu Ile Asp Arg His Ser Tyr Phe Thr Tyr Met Thr Glu
   35 40 45
   Leu His Glu Val Ala Thr Glu Arg Val Glu Pro Glu His Ile Gln Tyr
   50 55 60
   Asp Leu Gln Arg Leu Phe Ala Arg Arg Lys Asn Val Arg Leu Val Thr
   65 70 75 80
   Asp Thr Val Thr Gly Ile Asp Lys Lys Ala Gln Thr Val Thr Thr Glu
   85 90 95
   His Gly Ser Tyr Gln Tyr Asp Gln Leu Leu Ile Ser Leu Gly Gly Glu
   100 105 110
   Ser Asn Asp Phe Gly Thr Pro Gly Val Lys Glu His Gly Phe Glu Leu
   115 120 125
   Trp Ser Phe Glu Gln Ala Met Ala Leu Arg Ala His Leu Ser Ala Ile
   130 135 140
   Ile Arg Arg Gly Ala Ala Glu Leu Asp Pro Ala Lys Arg Lys Ala Met
   145 150 155 160
   Leu Thr Phe Thr Val Cys Gly Ser Gly Phe Thr Gly Ser Glu Leu Ile
   165 170 175
   Gly Glu Leu Ile Glu Tyr Arg Asp Val Leu Ala Arg Asp Asn Lys Leu
   180 185 190
   Asp Pro Ser Glu Ile Thr Leu Gln Leu Val Glu Ala Ala Pro Thr Ile
   195 200 205
   Ile Asn Met Leu Asn Arg Thr Gln Ala Gly Lys Ala Ala Lys Tyr Met
   210 215 220
   Glu Lys His Gly Val Lys Ile Met Thr Asn Ser Met Ile Thr Glu Val
   225 230 235 240
   Cys Glu Asp His Val Asn Leu Lys Gly Lys Asp Pro Ile Pro Thr Tyr
   245 250 255
   Thr Leu Ile Trp Thr Ala Gly Val Arg Ala Asn Ser Ile Val Lys Lys
   260 265 270
   Phe Gly Ile Glu Thr Asn Pro Arg Gly Gly Arg Leu Met Ala Asn Glu
   275 280 285
   Phe Met Gln Ala Lys Asp Cys Asn Asn Ile Phe Leu Ala Gly Asp Ser
   290 295 300
   Thr Ser Tyr Gln Glu Pro Asp Gln Pro Arg Pro Val Pro Gln Ile Val
   305 310 315 320
   Gln Gly Ala Glu Glu Thr Ala Ala Lys Ala Val Glu Gly Ile Ile Lys
   325 330 335
   Asn Val Asp Gln Thr Asp Val Thr Ile Lys Pro Phe Lys Gly Ala Tyr
   340 345 350
   Gln Ala Ser Val Asp Ser Ile Gly Ser Lys Tyr Ala Val Ala Gln Val
   355 360 365
   Leu Glu Lys Trp Asn Val Ser Gly Phe Ile Ala Val Leu Leu Lys His
   370 375 380
   Ala Ile Asn Trp Met Tyr Tyr Val Gln Ile Phe Ser Gly Tyr Tyr Leu
   385 390 395 400
   Phe Gln Tyr Phe Met His Glu Phe Phe Arg Thr Arg Asn Asn Arg Asn
   405 410 415
   Val Phe Arg Gly Trp Val Ser Arg Ala Gly Asn Val Leu Trp Ser Val
   420 425 430
   Pro Leu Arg Phe Phe Tyr Gly Ala Met Trp Leu Trp Asp Cys Trp Thr
   435 440 445
   Lys Val Gln Gly Ser Glu Ser Trp Phe Thr Asp Lys Leu Arg Leu Pro
   450 455 460
   Phe Glu Trp Ile Thr Val Ala Ala Thr Ser Gly Ala Ser Gln Ala Thr
   465 470 475 480
   Lys Ala Ala Ala Thr Ser Gly Ala Ser Glu Ala Ala Thr Ser Thr Val
   485 490 495
   Lys Ala Ala Lys Gly Val Phe Ser Leu Ser Tyr Met Tyr Gly Lys Glu
   500 505 510
   Pro Leu Met Val Phe Asp Lys Met Pro His Trp Phe Glu Ser Ile Thr
   515 520 525
   Lys Val Phe Ile Pro Asn Met Gln Met Ala Leu Phe Phe Gln Lys Phe
   530 535 540
   Met Thr Cys Val Glu Ile Val Ile Ala Leu Cys Ile Phe Phe Gly Leu
   545 550 555 560
   Phe Thr Trp Phe Ala Asn Ala Val Thr Ile Gly Leu Val Val Val Phe
   565 570 575
   Cys Leu Ser Gly Met Phe Tyr Trp Val Asn Ile Trp Met Ile Phe Val
   580 585 590
   Ala Leu Ala Leu Met Asn Gly Ser Gly Arg Thr Phe Gly Leu Asp Tyr
   595 600 605
   Trp Val Val Pro Trp Met Gln Lys His Leu Gly His Trp Trp Tyr Gly
   610 615 620
   Asn Val Arg Ser His Tyr Asp Gly Val Lys Thr Arg
   625 630 635

### SEQUENCE LISTING

<110> KYOWA HAKKO KOGYO CO., LTD
<120> Method for producing amino acid
<130> 1657
<160> 16
<170> PatentIn Ver. 3.1
<210> 1
   <211> 30
   <212> DNA
   <213> Artificial
<220>
   <223> Synthetic DNA
<400> 1
   ctgcttgccc tgcaggtgca ccagcaaacg 30
<210> 2
   <211> 30
   <212> DNA
   <213> Artificial
<220>
   <223> Synthetic DNA
<400> 2
   cgagctgcgc gacaaccagg aattcagcgg 30
<210> 3
   <211> 1404
   <212> DNA
   <213> Corynebacterium glutamicum ATCC13032
<220>
   <221> CDS
   <222>
<400> 3
   atg tca gtt aac cca acc cgc ccc gaa ggc ggc cgt cac cac gtc gtc    48
   Met Ser Val Asn Pro Thr Arg Pro Glu Gly Gly Arg His His Val Val
     1               5                  10                  15
   gtc atc ggt tct ggt ttt ggt ggc ctt ttt gct gcc aag aac ctg gcc    96
   Val Ile Gly Ser Gly Phe Gly Gly Leu Phe Ala Ala Lys Asn Leu Ala
                20                  25                  30
   aag gca gac gtc gat gtc act ctg att gac cgc acc aac cac cac ctc    144
   Lys Ala Asp Val Asp Val Thr Leu Ile Asp Arg Thr Asn His His Leu
               35                  40                  45
   ttc cag cca ctg ctg tac caa gtg gca acc ggt atc ctc tcc tcc ggt    192
   Phe Gln Pro Leu Leu Tyr Gln Val Ala Thr Gly Ile Leu Ser Ser Gly
           50                  55                  60
   gaa atc gca cct tcc act cga cag atc ctg ggc tcc cag gaa aac gtc    240
   Glu Ile Ala Pro Ser Thr Arg Gln Ile Leu Gly Ser Gln Glu Asn Val
   65                  70                  75                  80
   aac gtc atc aag ggc gaa gtc acc gac atc aac gtc gag tcc cag act    288
   Asn Val Ile Lys Gly Glu Val Thr Asp Ile Asn Val Glu Ser Gln Thr
                   85                  90                  95
   gtg acc gcc tcc ctg ggc gag ttc acc cgc gtt ttt gag tac gat tcc    336
   Val Thr Ala Ser Leu Gly Glu Phe Thr Arg Val Phe Glu Tyr Asp Ser
               100                 105                 110
   ttg gtc gtt ggt gct ggc gca ggt cag tcc tac ttc ggc aat gat cac    384
   Leu Val Val Gly Ala Gly Ala Gly Gln Ser Tyr Phe Gly Asn Asp His
           115                 120                 125
   ttc gct gag ttc gca cct ggc atg aag tcc atc gac gat gca ctg gag    432
   Phe Ala Glu Phe Ala Pro Gly Met Lys Ser Ile Asp Asp Ala Leu Glu
       130                 135                 140
   att cgt gca cgc atc atc ggt gct ttc gag cgc gct gag atc tgc gag    480
   Ile Arg Ala Arg Ile Ile Gly Ala Phe Glu Arg Ala Glu Ile Cys Glu
   145                 150                 155                 160
   gat cca gct gag cgc gaa cgc ctg ctc acc ttc gtc gtt gtt ggc gct    528
   Asp Pro Ala Glu Arg Glu Arg Leu Leu Thr Phe Val Val Val Gly Ala
                   165                 170                 175
   ggc cca acc ggt gtt gag ctt gct ggc cag ttg gct gag atg gct cac    576
   Gly Pro Thr Gly Val Glu Leu Ala Gly Gln Leu Ala Glu Met Ala His
               180                 185                 190
   cgc acc ctt gct ggt gag tac aag aac ttc aac acc aac tcc gca aag    624
   Arg Thr Leu Ala Gly Glu Tyr Lys Asn Phe Asn Thr Asn Ser Ala Lys
           195                 200                 205
   atc atc ctg ctt gat ggt gct cca cag gtt ctt cct cca ttc ggt aag    672
   Ile Ile Leu Leu Asp Gly Ala Pro Gln Val Leu Pro Pro Phe Gly Lys
       210                 215                 220
   cgc cta ggc cgc aac gca cag cgc acc ctg gaa aag atg ggt gtc aac    720
   Arg Leu Gly Arg Asn Ala Gln Arg Thr Leu Glu Lys Met Gly Val Asn
   225 230 235 240
   gtt cgc ctg aac gct atg gtc acc aac gtt gac gct acc tcg gtc acc 768
   Val Arg Leu Asn Ala Met Val Thr Asn Val Asp Ala Thr Ser Val Thr
   245 250 255
   tac aag acc aag gac ggc gaa gag cac acc atc gaa tct ttc tgc aag 816
   Tyr Lys Thr Lys Asp Gly Glu Glu His Thr Ile Glu Ser Phe Cys Lys
   260 265 270
   att tgg tcc gct ggt gtt gcg gca tcc cca ctg ggc aag ctc gtc gca 864
   Ile Trp Ser Ala Gly Val Ala Ala Ser Pro Leu Gly Lys Leu Val Ala
   275 280 285
   gag cag acc ggt gtt gag acc gac cgc gca ggc cgc gtc atg gtt aac 912
   Glu Gln Thr Gly Val Glu Thr Asp Arg Ala Gly Arg Val Met Val Asn
   290 295 300
   gat gac ctg tct gtt ggc gat cag aag aac gtc ttc gtt gtt ggc gac 960
   Asp Asp Leu Ser Val Gly Asp Gln Lys Asn Val Phe Val Val Gly Asp
   305 310 315 320
   atg atg aac tac aac aac ctc cct ggt gtt gct cag gta gca atc cag
   1008
   Met Met Asn Tyr Asn Asn Leu Pro Gly Val Ala Gln Val Ala Ile Gln
   325 330 335
   agt ggt gag tac gtt gct gag cag atc gaa gct gag gtt gaa ggc cgc
   1056
   Ser Gly Glu Tyr Val Ala Glu Gln Ile Glu Ala Glu Val Glu Gly Arg
   340 345 350
   tcc aac acc gag cgc gaa gct ttc gat tac ttc gac aag ggc tcc atg
   1104
   Ser Asn Thr Glu Arg Glu Ala Phe Asp Tyr Phe Asp Lys Gly Ser Met
   355 360 365
   gct acc att tcc cgc ttc tcc gca gtg gtg aag atg ggc aag gtt gag
   1152
   Ala Thr Ile Ser Arg Phe Ser Ala Val Val Lys Met Gly Lys Val Glu
   370 375 380
   gtc acc ggc ttc atc ggt tgg gtt ctg tgg ttg gct gtt cac atc atg
   1200
   Val Thr Gly Phe Ile Gly Trp Val Leu Trp Leu Ala Val His Ile Met
   385 390 395 400
   ttc ctg gtt ggc ttc cgc aac cgt ttc gtc tcc gca atc agc tgg ggc
   1248
   Phe Leu Val Gly Phe Arg Asn Arg Phe Val Ser Ala Ile Ser Trp Gly
   405 410 415
   ctg aac gca ctg tcc cgc aag cgt tgg aac ctg gca acc acc cgc cag
   1296
   Leu Asn Ala Leu Ser Arg Lys Arg Trp Asn Leu Ala Thr Thr Arg Gln
   420 425 430
   cag ctc cac tca cgc acc acg ctg tcc aag ttc gct cac gag ctt gag
   1344
   Gln Leu His Ser Arg Thr Thr Leu Ser Lys Phe Ala His Glu Leu Glu
   435 440 445
   gaa gca tct tct gat ctt cca atc gag ctg cgc gac aac cag cgt ttc
   1392
   Glu Ala Ser Ser Asp Leu Pro Ile Glu Leu Arg Asp Asn Gln Arg Phe
   450 455 460
   agc gga aag taa
   1404
   Ser Gly Lys
   465
<210> 4
   <211> 467
   <212> PRT
   <213> Corynebacterium glutamicum ATCC13032
<400> 4
   Met Ser Val Asn Pro Thr Arg Pro Glu Gly Gly Arg His His Val Val
   1 5 10 15
   Val Ile Gly Ser Gly Phe Gly Gly Leu Phe Ala Ala Lys Asn Leu Ala
   20 25 30
   Lys Ala Asp Val Asp Val Thr Leu Ile Asp Arg Thr Asn His His Leu
   35 40 45
   Phe Gln Pro Leu Leu Tyr Gln Val Ala Thr Gly Ile Leu Ser Ser Gly
   50 55 60
   Glu Ile Ala Pro Ser Thr Arg Gln Ile Leu Gly Ser Gln Glu Asn Val
   65 70 75 80
   Asn Val Ile Lys Gly Glu Val Thr Asp Ile Asn Val Glu Ser Gln Thr
   85 90 95
   Val Thr Ala Ser Leu Gly Glu Phe Thr Arg Val Phe Glu Tyr Asp Ser
   100 105 110
   Leu Val Val Gly Ala Gly Ala Gly Gln Ser Tyr Phe Gly Asn Asp His
           115                 120                 125
   Phe Ala Glu Phe Ala Pro Gly Met Lys Ser Ile Asp Asp Ala Leu Glu
       130                 135                 140
   Ile Arg Ala Arg Ile Ile Gly Ala Phe Glu Arg Ala Glu Ile Cys Glu
   145                 150                 155                 160
   Asp Pro Ala Glu Arg Glu Arg Leu Leu Thr Phe Val Val Val Gly Ala
                   165                 170                 175
   Gly Pro Thr Gly Val Glu Leu Ala Gly Gln Leu Ala Glu Met Ala His
               180                 185                 190
   Arg Thr Leu Ala Gly Glu Tyr Lys Asn Phe Asn Thr Asn Ser Ala Lys
           195                 200                 205
   Ile Ile Leu Leu Asp Gly Ala Pro Gln Val Leu Pro Pro Phe Gly Lys
       210                 215                 220
   Arg Leu Gly Arg Asn Ala Gln Arg Thr Leu Glu Lys Met Gly Val Asn
   225                 230                 235                 240
   Val Arg Leu Asn Ala Met Val Thr Asn Val Asp Ala Thr Ser Val Thr
                   245                 250                 255
   Tyr Lys Thr Lys Asp Gly Glu Glu His Thr Ile Glu Ser Phe Cys Lys
               260                 265                 270
   Ile Trp Ser Ala Gly Val Ala Ala Ser Pro Leu Gly Lys Leu Val Ala
           275                 280                 285
   Glu Gln Thr Gly Val Glu Thr Asp Arg Ala Gly Arg Val Met Val Asn
       290                 295                 300
   Asp Asp Leu Ser Val Gly Asp Gln Lys Asn Val Phe Val Val Gly Asp
   305                 310                 315                 320
   Met Met Asn Tyr Asn Asn Leu Pro Gly Val Ala Gln Val Ala Ile Gln
                   325                 330                 335
   Ser Gly Glu Tyr Val Ala Glu Gln Ile Glu Ala Glu Val Glu Gly Arg
               340                 345                 350
   Ser Asn Thr Glu Arg Glu Ala Phe Asp Tyr Phe Asp Lys Gly Ser Met
           355                 360                 365
   Ala Thr Ile Ser Arg Phe Ser Ala Val Val Lys Met Gly Lys Val Glu
   370 375 380
   Val Thr Gly Phe Ile Gly Trp Val Leu Trp Leu Ala Val His Ile Met
   385 390 395 400
   Phe Leu Val Gly Phe Arg Asn Arg Phe Val Ser Ala Ile Ser Trp Gly
   405 410 415
   Leu Asn Ala Leu Ser Arg Lys Arg Trp Asn Leu Ala Thr Thr Arg Gln
   420 425 430
   Gln Leu His Ser Arg Thr Thr Leu Ser Lys Phe Ala His Glu Leu Glu
   435 440 445
   Glu Ala Ser Ser Asp Leu Pro Ile Glu Leu Arg Asp Asn Gln Arg Phe
   450 455 460
   Ser Gly Lys
   465
<210> 5
   <211> 1362
   <212> DNA
   <213> Corynebacterium diphtheriae
<220>
   <221> CDS
   <222>
<400> 5
   atg act aac acc cca ttt cgc cca gaa ggt gga cgc cac cac gtt gta      48
   Met Thr Asn Thr Pro Phe Arg Pro Glu Gly Gly Arg His His Val Val
    1               5                  10                  15
   gtt att ggc tcc ggc ttc ggt gga cta ttc gca gtt caa aac ctc aaa      96
   Val Ile Gly Ser Gly Phe Gly Gly Leu Phe Ala Val Gln Asn Leu Lys
               20                  25                  30
   gat gca gat gtc gat atc acc ctc atc gac cgg aca aac cac cac ctt     144
   Asp Ala Asp Val Asp Ile Thr Leu Ile Asp Arg Thr Asn His His Leu
           35                  40                  45
   ttc cag ccg ttg ctt tac caa gta gca acc ggt atc ttg tcg tct ggt     192
   Phe Gln Pro Leu Leu Tyr Gln Val Ala Thr Gly Ile Leu Ser Ser Gly
       50                  55                  60
   gaa atc gca cca caa acg cgt caa gtt ctt gca cag caa aat aat gtg     240
   Glu Ile Ala Pro Gln Thr Arg Gln Val Leu Ala Gln Gln Asn Asn Val
   65                  70                  75                  80
   cac gtt ctt aag gct gaa gtc acc gac att gac acc gaa tcg aag acg    288
   His Val Leu Lys Ala Glu Val Thr Asp Ile Asp Thr Glu Ser Lys Thr
                    85                  90                  95
   gtc gtc gca gac ttg gat gat tat tct aaa aca att gaa tac gat tcc    336
   Val Val Ala Asp Leu Asp Asp Tyr Ser Lys Thr Ile Glu Tyr Asp Ser
               100                 105                 110
   ctg atc gtc gcc gct ggt gca ggt cag tct tac ttc gga aat gat cac    384
   Leu Ile Val Ala Ala Gly Ala Gly Gln Ser Tyr Phe Gly Asn Asp His
           115                 120                 125
   ttc gcg gaa ttc gcg ccg ggt atg aaa aca atc gat gat gca ctc gaa    432
   Phe Ala Glu Phe Ala Pro Gly Met Lys Thr Ile Asp Asp Ala Leu Glu
       130                 135                 140
   ctg cgt gcg cgc atc atc ggc gct ttc gaa cgc gca gaa atg tgc gaa    480
   Leu Arg Ala Arg Ile Ile Gly Ala Phe Glu Arg Ala Glu Met Cys Glu
   145                 150                 155                 160
   gat ccc aaa gaa cgt gaa cgc ctc ttg act ttt gtt atc gtt ggc gca    528
   Asp Pro Lys Glu Arg Glu Arg Leu Leu Thr Phe Val Ile Val Gly Ala
                   165                 170                 175
   gga cca aca ggc gta gaa ctt gca ggt cag ctg gcc gaa atg gca cac    576
   Gly Pro Thr Gly Val Glu Leu Ala Gly Gln Leu Ala Glu Met Ala His
               180                 185                 190
   cgc acg ttg tct gga gag tac acg cag ttc acg cct tcc aac gcg aag    624
   Arg Thr Leu Ser Gly Glu Tyr Thr Gln Phe Thr Pro Ser Asn Ala Lys
           195                 200                 205
   atc atc ctg ctt gac ggc gct cct cag gtg ctt cca ccg ttc ggc aag    672
   Ile Ile Leu Leu Asp Gly Ala Pro Gln Val Leu Pro Pro Phe Gly Lys
       210                 215                 220
   cgt ttg ggt cgt act gca cag cgt gaa tta gaa aag att ggt gta acg    720
   Arg Leu Gly Arg Thr Ala Gln Arg Glu Leu Glu Lys Ile Gly Val Thr
   225                 230                 235                 240
   gtc aag ctg aac gct atc gtt acc ggc gta gac gaa aac tca gtg aca    768
   Val Lys Leu Asn Ala Ile Val Thr Gly Val Asp Glu Asn Ser Val Thr
                   245                 250                 255
   tat aag tcc acc gtt gat gat tct ttg cac acg atc gat tcc ttc tgc    816
   Tyr Lys Ser Thr Val Asp Asp Ser Leu His Thr Ile Asp Ser Phe Cys
               260                 265                 270
   aag atc tgg tcg gca ggc gta gcc gct tcc cca cta ggc aaa cta gtt     864
   Lys Ile Trp Ser Ala Gly Val Ala Ala Ser Pro Leu Gly Lys Leu Val
           275                 280                 285
   gca gag cag ctt ggt gtc gag gtt gat cgt gca gga cgc gtc cca gtc     912
   Ala Glu Gln Leu Gly Val Glu Val Asp Arg Ala Gly Arg Val Pro Val
       290                 295                 300
   aac gaa gat ctt tct gtt ggc gac gat aag aac gtc ttt gtt att ggc     960
   Asn Glu Asp Leu Ser Val Gly Asp Asp Lys Asn Val Phe Val Ile Gly
   305                 310                 315                 320
   gat atg atg tcg ctc aac agg ctt ccg gga gtc gca cag gta gca atc
   1008
   Asp Met Met Ser Leu Asn Arg Leu Pro Gly Val Ala Gln Val Ala Ile
                   325                 330                 335
   caa ggc ggt gaa tac gtt gct gag cag att gct gct ggg gtc gag gga
   1056
   Gln Gly Gly Glu Tyr Val Ala Glu Gln Ile Ala Ala Gly Val Glu Gly
               340                 345                 350
   cgt tcc tcg tct gaa cgc cca gcc ttt gaa tac tac gac aag ggt tcg
   1104
   Arg Ser Ser Ser Glu Arg Pro Ala Phe Glu Tyr Tyr Asp Lys Gly Ser
           355                 360                 365
   atg gct acg gtg tcg cgc ttt aac gcc gtt gtg aag ctt gga aaa gtt
   1152
   Met Ala Thr Val Ser Arg Phe Asn Ala Val Val Lys Leu Gly Lys Val
       370                 375                 380
   gaa gtt acg gga ttt att ggc tgg gtc atg tgg ctt ctc gtc cac ttg
   1200
   Glu Val Thr Gly Phe Ile Gly Trp Val Met Trp Leu Leu Val His Leu
   385                 390                 395                 400
   atg ttc ttg gtc ggc ttc cgc aac cga gca act gcg gct ttc tct tgg
   1248
   Met Phe Leu Val Gly Phe Arg Asn Arg Ala Thr Ala Ala Phe Ser Trp
                   405                 410                 415
   ggc atc aat gcg ctt tca cgt aag cgt tgg aac ctc gcc acc act cgt
   1296
   Gly Ile Asn Ala Leu Ser Arg Lys Arg Trp Asn Leu Ala Thr Thr Arg
               420                 425                 430
   cag cag ctt cac ggc cgt act ggt ttg caa aaa ctt act gcg ctc gtc
   1344
   Gln Gln Leu His Gly Arg Thr Gly Leu Gln Lys Leu Thr Ala Leu Val
   435 440 445
   gat acc gcc gaa aag aag 1362
   Asp Thr Ala Glu Lys Lys
   450
<210> 6
   <211> 454
   <212> PRT
   <213> Corynebacterium diphtheriae
<400> 6
   Met Thr Asn Thr Pro Phe Arg Pro Glu Gly Gly Arg His His Val Val
   1 5 10 15
   Val Ile Gly Ser Gly Phe Gly Gly Leu Phe Ala Val Gln Asn Leu Lys
   20 25 30
   Asp Ala Asp Val Asp Ile Thr Leu Ile Asp Arg Thr Asn His His Leu
   35 40 45
   Phe Gln Pro Leu Leu Tyr Gln Val Ala Thr Gly Ile Leu Ser Ser Gly
   50 55 60
   Glu Ile Ala Pro Gln Thr Arg Gln Val Leu Ala Gln Gln Asn Asn Val
   65 70 75 80
   His Val Leu Lys Ala Glu Val Thr Asp Ile Asp Thr Glu Ser Lys Thr
   85 90 95
   Val Val Ala Asp Leu Asp Asp Tyr Ser Lys Thr Ile Glu Tyr Asp Ser
   100 105 110
   Leu Ile Val Ala Ala Gly Ala Gly Gln Ser Tyr Phe Gly Asn Asp His
   115 120 125
   Phe Ala Glu Phe Ala Pro Gly Met Lys Thr Ile Asp Asp Ala Leu Glu
   130 135 140
   Leu Arg Ala Arg Ile Ile Gly Ala Phe Glu Arg Ala Glu Met Cys Glu
   145 150 155 160
   Asp Pro Lys Glu Arg Glu Arg Leu Leu Thr Phe Val Ile Val Gly Ala
   165 170 175
   Gly Pro Thr Gly Val Glu Leu Ala Gly Gln Leu Ala Glu Met Ala His
   180 185 190
   Arg Thr Leu Ser Gly Glu Tyr Thr Gln Phe Thr Pro Ser Asn Ala Lys
   195 200 205
   Ile Ile Leu Leu Asp Gly Ala Pro Gln Val Leu Pro Pro Phe Gly Lys
   210 215 220
   Arg Leu Gly Arg Thr Ala Gln Arg Glu Leu Glu Lys Ile Gly Val Thr
   225 230 235 240
   Val Lys Leu Asn Ala Ile Val Thr Gly Val Asp Glu Asn Ser Val Thr
   245 250 255
   Tyr Lys Ser Thr Val Asp Asp Ser Leu His Thr Ile Asp Ser Phe Cys
   260 265 270
   Lys Ile Trp Ser Ala Gly Val Ala Ala Ser Pro Leu Gly Lys Leu Val
   275 280 285
   Ala Glu Gln Leu Gly Val Glu Val Asp Arg Ala Gly Arg Val Pro Val
   290 295 300
   Asn Glu Asp Leu Ser Val Gly Asp Asp Lys Asn Val Phe Val Ile Gly
   305 310 315 320
   Asp Met Met Ser Leu Asn Arg Leu Pro Gly Val Ala Gln Val Ala Ile
   325 330 335
   Gln Gly Gly Glu Tyr Val Ala Glu Gln Ile Ala Ala Gly Val Glu Gly
   340 345 350
   Arg Ser Ser Ser Glu Arg Pro Ala Phe Glu Tyr Tyr Asp Lys Gly Ser
   355 360 365
   Met Ala Thr Val Ser Arg Phe Asn Ala Val Val Lys Leu Gly Lys Val
   370 375 380
   Glu Val Thr Gly Phe Ile Gly Trp Val Met Trp Leu Leu Val His Leu
   385 390 395 400
   Met Phe Leu Val Gly Phe Arg Asn Arg Ala Thr Ala Ala Phe Ser Trp
   405 410 415
   Gly Ile Asn Ala Leu Ser Arg Lys Arg Trp Asn Leu Ala Thr Thr Arg
   420 425 430
   Gln Gln Leu His Gly Arg Thr Gly Leu Gln Lys Leu Thr Ala Leu Val
   435 440 445
   Asp Thr Ala Glu Lys Lys
   450
<210> 7
   <211> 1302
   <212> DNA
   <213> Escherichia coli
<220>
   <221> CDS
   <222>
<400> 7
   ttg act acg cca ttg aaa aag att gtg att gtc ggc ggc ggt gct ggt      48
   Met Thr Thr Pro Leu Lys Lys Ile Val Ile Val Gly Gly Gly Ala Gly
    1               5                  10                  15
   ggg ctg gaa atg gca aca cag ctg ggg cat aag ctg gga cgc aag aaa      96
   Gly Leu Glu Met Ala Thr Gln Leu Gly His Lys Leu Gly Arg Lys Lys
               20                  25                  30
   aaa gcc aaa att acg ctg gtc gat cgt aac cac agc cac ctg tgg aaa     144
   Lys Ala Lys Ile Thr Leu Val Asp Arg Asn His Ser His Leu Trp Lys
           35                  40                  45
   ccg ctg ctg cac gaa gtg gcg act ggc tcg ctt gat gaa ggc gtc gat     192
   Pro Leu Leu His Glu Val Ala Thr Gly Ser Leu Asp Glu Gly Val Asp
       50                  55                  60
   gcg ttg agc tat ctg gcc cat gcg cgc aat cat ggt ttc cag ttc cag     240
   Ala Leu Ser Tyr Leu Ala His Ala Arg Asn His Gly Phe Gln Phe Gln
    65                  70                  75                  80
   ctg ggt tcc gtc att gat att gat cgt gaa gcg aaa aca atc act att     288
   Leu Gly Ser Val Ile Asp Ile Asp Arg Glu Ala Lys Thr Ile Thr Ile
                   85                  90                  95
   gca gaa ctg cgc gac gag aaa ggt gaa ctg ctg gtt ccg gaa cgt aaa     336
   Ala Glu Leu Arg Asp Glu Lys Gly Glu Leu Leu Val Pro Glu Arg Lys
               100                 105                 110
   atc gcc tat gac acc ctg gta atg gcg ctg ggt agc acc tct aac gat     384
   Ile Ala Tyr Asp Thr Leu Val Met Ala Leu Gly Ser Thr Ser Asn Asp
           115                 120                 125
   ttc aat acg cca ggt gtc aaa gag aac tgc att ttc ctc gat aac ccg     432
   Phe Asn Thr Pro Gly Val Lys Glu Asn Cys Ile Phe Leu Asp Asn Pro
       130                 135                 140
   cac cag gcg cgt cgc ttc cac cag gag atg ctg aat ttg ttc ctg aaa     480
   His Gln Ala Arg Arg Phe His Gln Glu Met Leu Asn Leu Phe Leu Lys
   145                 150                 155                 160
   tac tcc gcc aac ctg ggc gcg aat ggc aaa gtg aac att gcg att gtc     528
   Tyr Ser Ala Asn Leu Gly Ala Asn Gly Lys Val Asn Ile Ala Ile Val
                   165                 170                 175
   ggc ggc ggc gcg acg ggt gta gaa ctc tcc gct gaa ttg cac aac gcg     576
   Gly Gly Gly Ala Thr Gly Val Glu Leu Ser Ala Glu Leu His Asn Ala
               180                 185                 190
   gtc aag caa ctg cac agc tac ggt tac aaa ggc ctg acc aac gaa gcc     624
   Val Lys Gln Leu His Ser Tyr Gly Tyr Lys Gly Leu Thr Asn Glu Ala
           195                 200                 205
   ctg aac gta acg ctg gta gaa gcg gga gaa cgt att ttg cct gcg tta     672
   Leu Asn Val Thr Leu Val Glu Ala Gly Glu Arg Ile Leu Pro Ala Leu
       210                 215                 220
   ccg cca cgt atc tct gct gcg gcc cac aac gag cta acg aaa ctt ggc     720
   Pro Pro Arg Ile Ser Ala Ala Ala His Asn Glu Leu Thr Lys Leu Gly
   225                 230                 235                 240
   gtt cgc gtg ctg acg caa acc atg gtc acc agt gct gat gaa ggc ggc     768
   Val Arg Val Leu Thr Gln Thr Met Val Thr Ser Ala Asp Glu Gly Gly
                   245                 250                 255
   ctg cac act aaa gat ggc gaa tat att gag gct gat ctg atg gta tgg     816
   Leu His Thr Lys Asp Gly Glu Tyr Ile Glu Ala Asp Leu Met Val Trp
               260                 265                 270
   gca gcc ggg atc aaa gcg cca gac ttc ctg aaa gat atc ggt ggt ctt     864
   Ala Ala Gly Ile Lys Ala Pro Asp Phe Leu Lys Asp Ile Gly Gly Leu
           275                 280                 285
   gaa act aac cgt atc aac cag ctg gtg gtg gaa ccg acg ctg caa acc     912
   Glu Thr Asn Arg Ile Asn Gln Leu Val Val Glu Pro Thr Leu Gln Thr
       290                 295                 300
   acc cgc gat cca gac att tac gct att ggc gac tgc gcg tca tgc ccg     960
   Thr Arg Asp Pro Asp Ile Tyr Ala Ile Gly Asp Cys Ala Ser Cys Pro
   305                 310                 315                 320
   cgt ccg gaa ggg ggc ttt gtt ccg ccg cgt gct cag gct gca cac cag
   1008
   Arg Pro Glu Gly Gly Phe Val Pro Pro Arg Ala Gln Ala Ala His Gln
                   325                 330                 335
   atg gcg act tgc gca atg aac aac att ctg gcg cag atg aac ggt aag
   1056
   Met Ala Thr Cys Ala Met Asn Asn Ile Leu Ala Gln Met Asn Gly Lys
               340                 345                 350
   ccg ctg aaa aat tat cag tat aaa gat cat ggt tcg ctg gta tcg ctg 1104
   Pro Leu Lys Asn Tyr Gln Tyr Lys Asp His Gly Ser Leu Val Ser Leu
   355 360 365
   tcg aac ttc tcc acc gtc ggt agc ctg atg ggt aac ctg acg cgc ggc 1152
   Ser Asn Phe Ser Thr Val Gly Ser Leu Met Gly Asn Leu Thr Arg Gly
   370 375 380
   tca atg atg att gaa gga cga att gcg cgc ttt gta tat atc tcg cta 1200
   Ser Met Met Ile Glu Gly Arg Ile Ala Arg Phe Val Tyr Ile Ser Leu
   385 390 395 400
   tac cga atg cat cag att gcg ctg cat ggt tac ttt aaa acc gga tta 1248
   Tyr Arg Met His Gln Ile Ala Leu His Gly Tyr Phe Lys Thr Gly Leu
   405 410 415
   atg atg ctg gtg ggg agt att aac cgc gtt atc cgt ccg cgt ttg aag 1296
   Met Met Leu Val Gly Ser Ile Asn Arg Val Ile Arg Pro Arg Leu Lys
   420 425 430
   ttg cat 1302
   Leu His
<210> 8
   <211> 434
   <212> PRT
   <213> Escherichia coli
<400> 8
   Met Thr Thr Pro Leu Lys Lys Ile Val Ile Val Gly Gly Gly Ala Gly
   1               5                   10                  15
   Gly Leu Glu Met Ala Thr Gln Leu Gly His Lys Leu Gly Arg Lys Lys
               20                  25                  30
   Lys Ala Lys Ile Thr Leu Val Asp Arg Asn His Ser His Leu Trp Lys
           35                  40                  45
   Pro Leu Leu His Glu Val Ala Thr Gly Ser Leu Asp Glu Gly Val Asp
       50                  55                  60
   Ala Leu Ser Tyr Leu Ala His Ala Arg Asn His Gly Phe Gln Phe Gln
   65                  70                  75                  80
   Leu Gly Ser Val Ile Asp Ile Asp Arg Glu Ala Lys Thr Ile Thr Ile
   85 90 95
   Ala Glu Leu Arg Asp Glu Lys Gly Glu Leu Leu Val Pro Glu Arg Lys
   100 105 110
   Ile Ala Tyr Asp Thr Leu Val Met Ala Leu Gly Ser Thr Ser Asn Asp
   115 120 125
   Phe Asn Thr Pro Gly Val Lys Glu Asn Cys Ile Phe Leu Asp Asn Pro
   130 135 140
   His Gln Ala Arg Arg Phe His Gln Glu Met Leu Asn Leu Phe Leu Lys
   145 150 155 160
   Tyr Ser Ala Asn Leu Gly Ala Asn Gly Lys Val Asn Ile Ala Ile Val
   165 170 175
   Gly Gly Gly Ala Thr Gly Val Glu Leu Ser Ala Glu Leu His Asn Ala
   180 185 190
   Val Lys Gln Leu His Ser Tyr Gly Tyr Lys Gly Leu Thr Asn Glu Ala
   195 200 205
   Leu Asn Val Thr Leu Val Glu Ala Gly Glu Arg Ile Leu Pro Ala Leu
   210 215 220
   Pro Pro Arg Ile Ser Ala Ala Ala His Asn Glu Leu Thr Lys Leu Gly
   225 230 235 240
   Val Arg Val Leu Thr Gln Thr Met Val Thr Ser Ala Asp Glu Gly Gly
   245 250 255
   Leu His Thr Lys Asp Gly Glu Tyr Ile Glu Ala Asp Leu Met Val Trp
   260 265 270
   Ala Ala Gly Ile Lys Ala Pro Asp Phe Leu Lys Asp Ile Gly Gly Leu
   275 280 285
   Glu Thr Asn Arg Ile Asn Gln Leu Val Val Glu Pro Thr Leu Gln Thr
   290 295 300
   Thr Arg Asp Pro Asp Ile Tyr Ala Ile Gly Asp Cys Ala Ser Cys Pro
   305 310 315 320
   Arg Pro Glu Gly Gly Phe Val Pro Pro Arg Ala Gln Ala Ala His Gln
   325 330 335
   Met Ala Thr Cys Ala Met Asn Asn Ile Leu Ala Gln Met Asn Gly Lys
   340 345 350
   Pro Leu Lys Asn Tyr Gln Tyr Lys Asp His Gly Ser Leu Val Ser Leu
   355 360 365
   Ser Asn Phe Ser Thr Val Gly Ser Leu Met Gly Asn Leu Thr Arg Gly
   370 375 380
   Ser Met Met Ile Glu Gly Arg Ile Ala Arg Phe Val Tyr Ile Ser Leu
   385 390 395 400
   Tyr Arg Met His Gln Ile Ala Leu His Gly Tyr Phe Lys Thr Gly Leu
   405 410 415
   Met Met Leu Val Gly Ser Ile Asn Arg Val Ile Arg Pro Arg Leu Lys
   420 425 430
   Leu His
<210> 9
   <211> 1296
   <212> DNA
   <213> Pseudomonas fluorescens
<220>
   <221> CDS
   <222>
<400> 9
   atg act cat cgt att gtc atc gtt ggc ggc ggc gcc ggc ggt ctg gag     48
   Met Thr His Arg Ile Val Ile Val Gly Gly Gly Ala Gly Gly Leu Glu
    1               5                  10                  15
   ttg gct acc cgt ctg ggt aag act ctg ggc aag cgt ggc acg gcc agt     96
   Leu Ala Thr Arg Leu Gly Lys Thr Leu Gly Lys Arg Gly Thr Ala Ser
               20                  25                  30
   gtg atg ctg gtc gac gcg aac ctg acc cac atc tgg aaa ccg cta ctg    144
   Val Met Leu Val Asp Ala Asn Leu Thr His Ile Trp Lys Pro Leu Leu
           35                  40                  45
   cac gaa gtg gcc gcc ggc tcc ttg aac tcc tcc gaa gac gaa ctc aac    192
   His Glu Val Ala Ala Gly Ser Leu Asn Ser Ser Glu Asp Glu Leu Asn
       50                  55                  60
   tat gtc gcc cag gca aaa tgg aac cac ttc gag ttc cag ctc ggg cgc    240
   Tyr Val Ala Gln Ala Lys Trp Asn His Phe Glu Phe Gln Leu Gly Arg
   65                  70                  75                  80
   atg agc ggc ctg gat cgc gag cgc aag aga atc caa ctg gcc gcc acc    288
   Met Ser Gly Leu Asp Arg Glu Arg Lys Arg Ile Gln Leu Ala Ala Thr
   85 90 95
   tat gac gag acc ggc gtc gag ctg ttg ccg gcc cgg gaa ctg ggc tac 336
   Tyr Asp Glu Thr Gly Val Glu Leu Leu Pro Ala Arg Glu Leu Gly Tyr
   100 105 110
   gac acc ctg gtg att gcc gtc ggc agc acc acc aat gac ttc ggc acc 384
   Asp Thr Leu Val Ile Ala Val Gly Ser Thr Thr Asn Asp Phe Gly Thr
   115 120 125
   gaa ggc gcg gcg cag cac tgc ctg ttc ctc gac acc cgc aaa cag gcc 432
   Glu Gly Ala Ala Gln His Cys Leu Phe Leu Asp Thr Arg Lys Gln Ala
   130 135 140
   gag cgc ttc cat cag caa ttg ctg cac cac tat ctg cgc gcc cac gcc 480
   Glu Arg Phe His Gln Gln Leu Leu His His Tyr Leu Arg Ala His Ala
   145 150 155 160
   ggg cag acc gat atc gtc gag cgc atc agc gtc gcc att gtc ggc gcc 528
   Gly Gln Thr Asp Ile Val Glu Arg Ile Ser Val Ala Ile Val Gly Ala
   165 170 175
   ggt gcg acc ggg gtc gaa ctg gcc gcc gag ctg cat aat gcc gcc cac 576
   Gly Ala Thr Gly Val Glu Leu Ala Ala Glu Leu His Asn Ala Ala His
   180 185 190
   gaa ctg cac gcc tac ggc ctg gac cgg atc aaa ccg gag aac atg cac 624
   Glu Leu His Ala Tyr Gly Leu Asp Arg Ile Lys Pro Glu Asn Met His
   195 200 205
   atc acc ctg atc gag gcc ggg cca cgc gtc ttg ccg gcc ctg ccg gag 672
   Ile Thr Leu Ile Glu Ala Gly Pro Arg Val Leu Pro Ala Leu Pro Glu
   210 215 220
   cgt atc ggc ggg ccg gtg cac aag acc ctg gaa aaa ctc ggg gtc aac 720
   Arg Ile Gly Gly Pro Val His Lys Thr Leu Glu Lys Leu Gly Val Asn
   225 230 235 240
   gtc atg acc aac gcc gcc gtc agc cag gtg acc gcc gac agc ctg att 768
   Val Met Thr Asn Ala Ala Val Ser Gln Val Thr Ala Asp Ser Leu Ile
   245 250 255
   acc gcg gac ggc aaa gtg atc gac gcg agc ctg aaa gtc tgg gcc gcc 816
   Thr Ala Asp Gly Lys Val Ile Asp Ala Ser Leu Lys Val Trp Ala Ala
   260 265 270
   ggg att cgc gcc ccg gac ttc ctc aag gac atc gac ggg ctg gag acc 864
   Gly Ile Arg Ala Pro Asp Phe Leu Lys Asp Ile Asp Gly Leu Glu Thr
   275 280 285
   aac cgg atc aac cag ttg cac gtg ctg ccc act ttg cag acc acc cgc 912
   Asn Arg Ile Asn Gln Leu His Val Leu Pro Thr Leu Gln Thr Thr Arg
   290 295 300
   gac gag aac atc ttc gcc ttc ggc gac tgc gcc gcc tgc ccg caa ccc 960
   Asp Glu Asn Ile Phe Ala Phe Gly Asp Cys Ala Ala Cys Pro Gln Pro
   305 310 315 320
   ggc agc gag cgc aac gtc cca cct cgc gcc cag gcc gca cac cag caa
   1008
   Gly Ser Glu Arg Asn Val Pro Pro Arg Ala Gln Ala Ala His Gln Gln
   325 330 335
   gcc tca ctg ctg gcc aaa tcc ttg aag ctg cgg atc gag ggc aag gcc
   1056
   Ala Ser Leu Leu Ala Lys Ser Leu Lys Leu Arg Ile Glu Gly Lys Ala
   340 345 350
   ctg ccg gaa tac aaa tac acc gac tac ggc tcg ctg atc tcg ctg tcg
   1104
   Leu Pro Glu Tyr Lys Tyr Thr Asp Tyr Gly Ser Leu Ile Ser Leu Ser
   355 360 365
   cgg ttc tcg gca gtg ggc aac ctg atg ggt aac ctg acc ggc agc gtg
   1152
   Arg Phe Ser Ala Val Gly Asn Leu Met Gly Asn Leu Thr Gly Ser Val
   370 375 380
   atg ctc gaa ggc tgg ctg gcg cgg atg ttc tat gtg tcg ctg tac cgc
   1200
   Met Leu Glu Gly Trp Leu Ala Arg Met Phe Tyr Val Ser Leu Tyr Arg
   385 390 395 400
   atg cac cag atg gcg ctg tac ggc atg ttc cgc acg gcc atg ttg atg
   1248
   Met His Gln Met Ala Leu Tyr Gly Met Phe Arg Thr Ala Met Leu Met
   405 410 415
   ctg ggt agc aag atc ggg cgt ggg acc gag cct cgg ctg aag ctg cac
   1296
   Leu Gly Ser Lys Ile Gly Arg Gly Thr Glu Pro Arg Leu Lys Leu His
   420 425 430
<210> 10
   <211> 432
   <212> PRT
   <213> Pseudomonas fluorescens
<400> 10
   Met Thr His Arg Ile Val Ile Val Gly Gly Gly Ala Gly Gly Leu Glu
   1 5 10 15
   Leu Ala Thr Arg Leu Gly Lys Thr Leu Gly Lys Arg Gly Thr Ala Ser
   20 25 30
   Val Met Leu Val Asp Ala Asn Leu Thr His Ile Trp Lys Pro Leu Leu
   35 40 45
   His Glu Val Ala Ala Gly Ser Leu Asn Ser Ser Glu Asp Glu Leu Asn
   50 55 60
   Tyr Val Ala Gln Ala Lys Trp Asn His Phe Glu Phe Gln Leu Gly Arg
   65 70 75 80
   Met Ser Gly Leu Asp Arg Glu Arg Lys Arg Ile Gln Leu Ala Ala Thr
   85 90 95
   Tyr Asp Glu Thr Gly Val Glu Leu Leu Pro Ala Arg Glu Leu Gly Tyr
   100 105 110
   Asp Thr Leu Val Ile Ala Val Gly Ser Thr Thr Asn Asp Phe Gly Thr
   115 120 125
   Glu Gly Ala Ala Gln His Cys Leu Phe Leu Asp Thr Arg Lys Gln Ala
   130 135 140
   Glu Arg Phe His Gln Gln Leu Leu His His Tyr Leu Arg Ala His Ala
   145 150 155 160
   Gly Gln Thr Asp Ile Val Glu Arg Ile Ser Val Ala Ile Val Gly Ala
   165 170 175
   Gly Ala Thr Gly Val Glu Leu Ala Ala Glu Leu His Asn Ala Ala His
   180 185 190
   Glu Leu His Ala Tyr Gly Leu Asp Arg Ile Lys Pro Glu Asn Met His
   195 200 205
   Ile Thr Leu Ile Glu Ala Gly Pro Arg Val Leu Pro Ala Leu Pro Glu
   210 215 220
   Arg Ile Gly Gly Pro Val His Lys Thr Leu Glu Lys Leu Gly Val Asn
   225 230 235 240
   Val Met Thr Asn Ala Ala Val Ser Gln Val Thr Ala Asp Ser Leu Ile
   245 250 255
   Thr Ala Asp Gly Lys Val Ile Asp Ala Ser Leu Lys Val Trp Ala Ala
   260 265 270
   Gly Ile Arg Ala Pro Asp Phe Leu Lys Asp Ile Asp Gly Leu Glu Thr
   275 280 285
   Asn Arg Ile Asn Gln Leu His Val Leu Pro Thr Leu Gln Thr Thr Arg
   290 295 300
   Asp Glu Asn Ile Phe Ala Phe Gly Asp Cys Ala Ala Cys Pro Gln Pro
   305 310 315 320
   Gly Ser Glu Arg Asn Val Pro Pro Arg Ala Gln Ala Ala His Gln Gln
   325 330 335
   Ala Ser Leu Leu Ala Lys Ser Leu Lys Leu Arg Ile Glu Gly Lys Ala
   340 345 350
   Leu Pro Glu Tyr Lys Tyr Thr Asp Tyr Gly Ser Leu Ile Ser Leu Ser
   355 360 365
   Arg Phe Ser Ala Val Gly Asn Leu Met Gly Asn Leu Thr Gly Ser Val
   370 375 380
   Met Leu Glu Gly Trp Leu Ala Arg Met Phe Tyr Val Ser Leu Tyr Arg
   385 390 395 400
   Met His Gln Met Ala Leu Tyr Gly Met Phe Arg Thr Ala Met Leu Met
   405 410 415
   Leu Gly Ser Lys Ile Gly Arg Gly Thr Glu Pro Arg Leu Lys Leu His
   420 425 430
<210> 11
   <211> 1296
   <212> DNA
   <213> Azotobacter vinelandii
<220>
   <221> CDS
   <222>
<400> 11
   atg act cat cgt atc gta atc gtc ggc ggt ggc gct ggc ggc gtg gaa    48
   Met Thr His Arg Ile Val Ile Val Gly Gly Gly Ala Gly Gly Val Glu
    1               5                  10                  15
   ctc gct acc cgc ctc ggc aag acc atg ggc agg aac ttc cag gcg aag    96
   Leu Ala Thr Arg Leu Gly Lys Thr Met Gly Arg Asn Phe Gln Ala Lys
               20                  25                  30
   atc acc ctg gtc gac gcc aac atg acc cac ctg tgg aaa ccg ctg ctg   144
   Ile Thr Leu Val Asp Ala Asn Met Thr His Leu Trp Lys Pro Leu Leu
   35 40 45
   cac gaa gtc gcc gcc ggc tcg ctg aac tcg acc ggc gac gaa ctg aac 192
   His Glu Val Ala Ala Gly Ser Leu Asn Ser Thr Gly Asp Glu Leu Asn
   50 55 60
   tat gtg gcc cag gcc aaa tgg aac aac ttc gag ttc cag tac ggc cgc 240
   Tyr Val Ala Gln Ala Lys Trp Asn Asn Phe Glu Phe Gln Tyr Gly Arg
   65 70 75 80
   atg tgc ggt ctg gac cgg gcc aac aag cgt atc cgc ctg gcg gcc cag 288
   Met Cys Gly Leu Asp Arg Ala Asn Lys Arg Ile Arg Leu Ala Ala Gln
   85 90 95
   ccg gcc cag gaa gat cgc gcg ccc ctg ccc gag cgc gaa ctg gaa tac 336
   Pro Ala Gln Glu Asp Arg Ala Pro Leu Pro Glu Arg Glu Leu Glu Tyr
   100 105 110
   gac acc ctg gtc ctt tcc gtc ggc agc acc acc aat gac ttc ggc acc 384
   Asp Thr Leu Val Leu Ser Val Gly Ser Thr Thr Asn Asp Phe Gly Thr
   115 120 125
   ccg ggc gcc gcc gag aac tgt att ttc ctg gaa ggc cgc gac cag gcc 432
   Pro Gly Ala Ala Glu Asn Cys Ile Phe Leu Glu Gly Arg Asp Gln Ala
   130 135 140
   gag cgt ttc cgc cgt ccg ctg ctc agc cac tac ttg cgt gcc cac gcc 480
   Glu Arg Phe Arg Arg Pro Leu Leu Ser His Tyr Leu Arg Ala His Ala
   145 150 155 160
   agc aat gac gac ggc cat cag gtc aag gtc gcc atc gtc ggc gcc ggt 528
   Ser Asn Asp Asp Gly His Gln Val Lys Val Ala Ile Val Gly Ala Gly
   165 170 175
   gcc acc ggg gtc gaa ctg gcc gca gaa ctg cgc cac gcc tcc aag gaa 576
   Ala Thr Gly Val Glu Leu Ala Ala Glu Leu Arg His Ala Ser Lys Glu
   180 185 190
   ctg gtc gcc tat ggg ctg gag cgc att ccg ccg gag aac ctc agc atc 624
   Leu Val Ala Tyr Gly Leu Glu Arg Ile Pro Pro Glu Asn Leu Ser Ile
   195 200 205
   acg ctg atc gaa tcc agc ccg cgt gta ctc gcc gcc ctg ccc gaa cgc 672
   Thr Leu Ile Glu Ser Ser Pro Arg Val Leu Ala Ala Leu Pro Glu Arg
   210 215 220
   atc agc cgc tcc gcg cac gcc acc ctg gaa agc ctg ggc gtt cgc gtg 720
   Ile Ser Arg Ser Ala His Ala Thr Leu Glu Ser Leu Gly Val Arg Val
   225 230 235 240
   ctc gtc agc acc gcc gtc agc gag gtc acc gcg gaa ggc gtg aag acg 768
   Leu Val Ser Thr Ala Val Ser Glu Val Thr Ala Glu Gly Val Lys Thr
   245 250 255
   aag gac gac cag ttc atc ccc gcc gac ctc atg gtc tgg gcc gca ggc 816
   Lys Asp Asp Gln Phe Ile Pro Ala Asp Leu Met Val Trp Ala Ala Gly
   260 265 270
   gtc cgc gcg ccc gcc ttc ctc aag gag ctg gat ggt ctg gaa acc aat 864
   Val Arg Ala Pro Ala Phe Leu Lys Glu Leu Asp Gly Leu Glu Thr Asn
   275 280 285
   cgc atc aac cag ttg cag gtc cgc cag acc ctg cag act act ctg gac 912
   Arg Ile Asn Gln Leu Gln Val Arg Gln Thr Leu Gln Thr Thr Leu Asp
   290 295 300
   gac gat atc ttc gcc ttc ggc gat tgt gcc tcc tgc ccg cag ccg ggt 960
   Asp Asp Ile Phe Ala Phe Gly Asp Cys Ala Ser Cys Pro Gln Pro Gly
   305 310 315 320
   acc gac cgc ccc gtt ccg ccg cgc gcc cag gcc gct cac cag cag gcc
   1008
   Thr Asp Arg Pro Val Pro Pro Arg Ala Gln Ala Ala His Gln Gln Ala
   325 330 335
   agc ctg ctg gcc aag tcg ctc cac cgc aag ctg cag gaa gac agc ctc
   1056
   Ser Leu Leu Ala Lys Ser Leu His Arg Lys Leu Gln Glu Asp Ser Leu
   340 345 350
   tgc tgg agt atc gct aca gcg aac cac ggc tcg ctg atc tcc ctc tcg
   1104
   Cys Trp Ser Ile Ala Thr Ala Asn His Gly Ser Leu Ile Ser Leu Ser
   355 360 365
   agc ttc tcg gcg atc ggt aac ctg atg ggc aac ctg acc ggc aac gtg
   1152
   Ser Phe Ser Ala Ile Gly Asn Leu Met Gly Asn Leu Thr Gly Asn Val
   370 375 380
   acc ttg gaa ggc tgg ctg gcc cgc aag ttc tac att tcc ctg tac cgc
   1200
   Thr Leu Glu Gly Trp Leu Ala Arg Lys Phe Tyr Ile Ser Leu Tyr Arg
   385 390 395 400
   atg cac cag atg gcg ctc tac ggt acc ttc cgc acc ctg atg atg atg
   1248
   Met His Gln Met Ala Leu Tyr Gly Thr Phe Arg Thr Leu Met Met Met
   405 410 415
   ctg ggc gac cgc ttc cgc agc agc acc gaa ccc cgc ctc aag ctt cac 1296
   Leu Gly Asp Arg Phe Arg Ser Ser Thr Glu Pro Arg Leu Lys Leu His
   420 425 430
<210> 12
   <211> 432
   <212> PRT
   <213> Azotobacter vinelandii
<400> 12
   Met Thr His Arg Ile Val Ile Val Gly Gly Gly Ala Gly Gly Val Glu
   1               5                   10                  15
   Leu Ala Thr Arg Leu Gly Lys Thr Met Gly Arg Asn Phe Gln Ala Lys
               20                  25                  30
   Ile Thr Leu Val Asp Ala Asn Met Thr His Leu Trp Lys Pro Leu Leu
           35                  40                  45
   His Glu Val Ala Ala Gly Ser Leu Asn Ser Thr Gly Asp Glu Leu Asn
       50                  55                  60
   Tyr Val Ala Gln Ala Lys Trp Asn Asn Phe Glu Phe Gln Tyr Gly Arg
   65                  70                  75                  80
   Met Cys Gly Leu Asp Arg Ala Asn Lys Arg Ile Arg Leu Ala Ala Gln
                   85                  90                  95
   Pro Ala Gln Glu Asp Arg Ala Pro Leu Pro Glu Arg Glu Leu Glu Tyr
               100                 105                 110
   Asp Thr Leu Val Leu Ser Val Gly Ser Thr Thr Asn Asp Phe Gly Thr
           115                 120                 125
   Pro Gly Ala Ala Glu Asn Cys Ile Phe Leu Glu Gly Arg Asp Gln Ala
       130                 135                 140
   Glu Arg Phe Arg Arg Pro Leu Leu Ser His Tyr Leu Arg Ala His Ala
   145                 150                 155                 160
   Ser Asn Asp Asp Gly His Gln Val Lys Val Ala Ile Val Gly Ala Gly
                   165                 170                 175
   Ala Thr Gly Val Glu Leu Ala Ala Glu Leu Arg His Ala Ser Lys Glu
               180                 185                 190
   Leu Val Ala Tyr Gly Leu Glu Arg Ile Pro Pro Glu Asn Leu Ser Ile
           195                 200                 205
   Thr Leu Ile Glu Ser Ser Pro Arg Val Leu Ala Ala Leu Pro Glu Arg
   210 215 220
   Ile Ser Arg Ser Ala His Ala Thr Leu Glu Ser Leu Gly Val Arg Val
   225 230 235 240
   Leu Val Ser Thr Ala Val Ser Glu Val Thr Ala Glu Gly Val Lys Thr
   245 250 255
   Lys Asp Asp Gln Phe Ile Pro Ala Asp Leu Met Val Trp Ala Ala Gly
   260 265 270
   Val Arg Ala Pro Ala Phe Leu Lys Glu Leu Asp Gly Leu Glu Thr Asn
   275 280 285
   Arg Ile Asn Gln Leu Gln Val Arg Gln Thr Leu Gln Thr Thr Leu Asp
   290 295 300
   Asp Asp Ile Phe Ala Phe Gly Asp Cys Ala Ser Cys Pro Gln Pro Gly
   305 310 315 320
   Thr Asp Arg Pro Val Pro Pro Arg Ala Gln Ala Ala His Gln Gln Ala
   325 330 335
   Ser Leu Leu Ala Lys Ser Leu His Arg Lys Leu Gln Glu Asp Ser Leu
   340 345 350
   Cys Trp Ser Ile Ala Thr Ala Asn His Gly Ser Leu Ile Ser Leu Ser
   355 360 365
   Ser Phe Ser Ala Ile Gly Asn Leu Met Gly Asn Leu Thr Gly Asn Val
   370 375 380
   Thr Leu Glu Gly Trp Leu Ala Arg Lys Phe Tyr Ile Ser Leu Tyr Arg
   385 390 395 400
   Met His Gln Met Ala Leu Tyr Gly Thr Phe Arg Thr Leu Met Met Met
   405 410 415
   Leu Gly Asp Arg Phe Arg Ser Ser Thr Glu Pro Arg Leu Lys Leu His
   420 425 430
<210> 13
   <211> 1302
   <212> DNA
   <213> Salmonella typhimurium LT2
<220>
   <221> CDS
   <222>
<400> 13 <210> 14

   <211> 434
   <212> PRT
   <213> Salmonella typhimurium LT2
<400> 14
<210> 15
   <211> 1908
   <212> DNA
   <213> Lactobacillus plantarum WCFS1
<220>
   <221> CDS
   <222>
<400> 15
   atg gca aag aaa aat att gtc gtt gtc ggt gcg ggg ttt gct ggt gtt 48
   Met Ala Lys Lys Asn Ile Val Val Val Gly Ala Gly Phe Ala Gly Val
   1 5 10 15
   tac gca acc aag aaa ctg tct aag cat ttc aaa aaa aat gca gac gtc 96
   Tyr Ala Thr Lys Lys Leu Ser Lys His Phe Lys Lys Asn Ala Asp Val
   20 25 30
   gag att acg ttg att gac cgg cat tca tac ttc acg tat atg act gaa 144
   Glu Ile Thr Leu Ile Asp Arg His Ser Tyr Phe Thr Tyr Met Thr Glu
   35 40 45
   tta cat gaa gtt gct acc gaa cgg gtg gaa cct gag cat atc caa tat 192
   Leu His Glu Val Ala Thr Glu Arg Val Glu Pro Glu His Ile Gln Tyr
   50 55 60
   gat ttg caa cgg ttg ttc gca cgg cga aaa aac gtt cgt ctc gtg acc 240
   Asp Leu Gln Arg Leu Phe Ala Arg Arg Lys Asn Val Arg Leu Val Thr
   65 70 75 80
   gat acc gtg acg ggc atc gac aaa aag gca caa aca gtt act acc gaa 288
   Asp Thr Val Thr Gly Ile Asp Lys Lys Ala Gln Thr Val Thr Thr Glu
   85 90 95
   cac gga agt tat caa tat gat caa ctt tta att agt ttg ggt ggg gaa 336
   His Gly Ser Tyr Gln Tyr Asp Gln Leu Leu Ile Ser Leu Gly Gly Glu
               100                 105                 110
   tcc aat gac ttt ggg act ccc ggt gtt aag gaa cac ggc ttc gaa ttg     384
   Ser Asn Asp Phe Gly Thr Pro Gly Val Lys Glu His Gly Phe Glu Leu
           115                 120                 125
   tgg tcc ttc gaa caa gcg atg gca ttg cgc gct cac tta tct gca att     432
   Trp Ser Phe Glu Gln Ala Met Ala Leu Arg Ala His Leu Ser Ala Ile
       130                 135                 140
   att cgg cgg ggg gcg gcg gag ctc gac cct gct aag cgc aaa gcc atg     480
   Ile Arg Arg Gly Ala Ala Glu Leu Asp Pro Ala Lys Arg Lys Ala Met
   145                 150                 155                 160
   ttg acc ttt aca gtc tgt ggt tct ggt ttt act ggt tct gaa ctg att     528
   Leu Thr Phe Thr Val Cys Gly Ser Gly Phe Thr Gly Ser Glu Leu Ile
                   165                 170                 175
   ggt gaa tta atc gaa tat cgt gat gtt ttg gct cga gac aac aag ctc     576
   Gly Glu Leu Ile Glu Tyr Arg Asp Val Leu Ala Arg Asp Asn Lys Leu
               180                 185                 190
   gat cca agt gaa atc acg ctc caa ttg gtc gaa gca gcg ccg act att     624
   Asp Pro Ser Glu Ile Thr Leu Gln Leu Val Glu Ala Ala Pro Thr Ile
           195                 200                 205
   att aac atg ctc aac cgg acg caa gcc ggt aag gcc gct aag tac atg     672
   Ile Asn Met Leu Asn Arg Thr Gln Ala Gly Lys Ala Ala Lys Tyr Met
       210                 215                 220
   gaa aaa cat ggt gtc aaa atc atg acg aac tcc atg att acc gaa gtc     720
   Glu Lys His Gly Val Lys Ile Met Thr Asn Ser Met Ile Thr Glu Val
   225                 230                 235                 240
   tgt gaa gac cat gtt aac tta aaa ggc aag gat cca att cca acc tac     768
   Cys Glu Asp His Val Asn Leu Lys Gly Lys Asp Pro Ile Pro Thr Tyr
                   245                 250                 255
   acg tta atc tgg aca gcc ggt gtt cgt gct aat agt atc gtt aaa aag     816
   Thr Leu Ile Trp Thr Ala Gly Val Arg Ala Asn Ser Ile Val Lys Lys
               260                 265                 270
   ttc ggc att gaa act aac ccc cgc ggt ggt cgc ttg atg gcc aat gaa     864
   Phe Gly Ile Glu Thr Asn Pro Arg Gly Gly Arg Leu Met Ala Asn Glu
           275                 280                 285
   ttc atg caa gct aag gat tgt aac aat atc ttc tta gcc ggt gat tca     912
   Phe Met Gln Ala Lys Asp Cys Asn Asn Ile Phe Leu Ala Gly Asp Ser
       290                 295                 300
   acc agc tac caa gaa cct gac caa cca cgg cca gtc cca caa atc gtt 960
   Thr Ser Tyr Gln Glu Pro Asp Gln Pro Arg Pro Val Pro Gln Ile Val
   305 310 315 320
   caa ggg gct gaa gaa acc gca gct aag gcc gtc gaa ggt att att aag
   1008
   Gln Gly Ala Glu Glu Thr Ala Ala Lys Ala Val Glu Gly Ile Ile Lys
   325 330 335
   aac gtt gac cag act gac gtt acg atc aag cca ttt aag ggc gct tat
   1056
   Asn Val Asp Gln Thr Asp Val Thr Ile Lys Pro Phe Lys Gly Ala Tyr
   340 345 350
   caa gca tcc gtc gac tca att ggt tcc aaa tat gcc gtt gca caa gtt
   1104
   Gln Ala Ser Val Asp Ser Ile Gly Ser Lys Tyr Ala Val Ala Gln Val
   355 360 365
   tta gag aag tgg aac gtc tct ggt ttt att gcc gtg ctt tta aaa cac
   1152
   Leu Glu Lys Trp Asn Val Ser Gly Phe Ile Ala Val Leu Leu Lys His
   370 375 380
   gcc atc aac tgg atg tac tac gtt cag att ttc tca ggt tac tac cta
   1200
   Ala Ile Asn Trp Met Tyr Tyr Val Gln Ile Phe Ser Gly Tyr Tyr Leu
   385 390 395 400
   ttc cag tac ttc atg cac gaa ttc ttc cgg act cgt aat aac cgt aac
   1248
   Phe Gln Tyr Phe Met His Glu Phe Phe Arg Thr Arg Asn Asn Arg Asn
   405 410 415
   gtc ttc cgc ggt tgg gtc tca cgg gct ggt aac gta ctc tgg agt gtg
   1296
   Val Phe Arg Gly Trp Val Ser Arg Ala Gly Asn Val Leu Trp Ser Val
   420 425 430
   cca ctg cgg ttc ttc tat ggt gcc atg tgg tta tgg gac tgc tgg act
   1344
   Pro Leu Arg Phe Phe Tyr Gly Ala Met Trp Leu Trp Asp Cys Trp Thr
   435 440 445
   aaa gtt cag gga tct gaa tcc tgg ttc act gac aag tta cgg tta cca
   1392
   Lys Val Gln Gly Ser Glu Ser Trp Phe Thr Asp Lys Leu Arg Leu Pro
   450 455 460
   ttc gaa tgg att acc gtg gcc gca acc agt ggt gcc tct caa gca act
   1440
   Phe Glu Trp Ile Thr Val Ala Ala Thr Ser Gly Ala Ser Gln Ala Thr
   465 470 475 480
   aaa gcc gct gca acc agt ggt gct tct gaa gct gcc acg tca acc gtt
   1488
   Lys Ala Ala Ala Thr Ser Gly Ala Ser Glu Ala Ala Thr Ser Thr Val
   485 490 495
   aaa gcc gct aag ggt gtc ttc agt ctt tca tac atg tac ggt aaa gaa
   1536
   Lys Ala Ala Lys Gly Val Phe Ser Leu Ser Tyr Met Tyr Gly Lys Glu
   500 505 510
   ccc ctg atg gtc ttt gac aaa atg cca cat tgg ttc gaa tca att acc
   1584
   Pro Leu Met Val Phe Asp Lys Met Pro His Trp Phe Glu Ser Ile Thr
   515 520 525
   aag gtc ttc att ccg aac atg caa atg gcc ctc ttc ttc cag aaa ttc
   1632
   Lys Val Phe Ile Pro Asn Met Gln Met Ala Leu Phe Phe Gln Lys Phe
   530 535 540
   atg act tgt gtt gaa atc gtc att gca tta tgt att ttc ttc gga ctc
   1680
   Met Thr Cys Val Glu Ile Val Ile Ala Leu Cys Ile Phe Phe Gly Leu
   545 550 555 560
   ttt act tgg ttt gct aac gca gtc acc att ggc tta gtt gtc gtc ttc
   1728
   Phe Thr Trp Phe Ala Asn Ala Val Thr Ile Gly Leu Val Val Val Phe
   565 570 575
   tgc tta tct ggc atg ttc tac tgg gtc aac att tgg atg atc ttt gtt
   1776
   Cys Leu Ser Gly Met Phe Tyr Trp Val Asn Ile Trp Met Ile Phe Val
   580 585 590
   gcc ctc gcc tta atg aac ggt tcc gga cgg aca ttt ggg tta gac tac
   1824
   Ala Leu Ala Leu Met Asn Gly Ser Gly Arg Thr Phe Gly Leu Asp Tyr
   595 600 605
   tgg gtc gtt ccg tgg atg caa aaa cac ctt gga cac tgg tgg tac ggc
   1872
   Trp Val Val Pro Trp Met Gln Lys His Leu Gly His Trp Trp Tyr Gly
   610 615 620
   aac gtt cgt tct cat tac gac ggt gtt aaa acc cgc                1908
   Asn Val Arg Ser His Tyr Asp Gly Val Lys Thr Arg
   625                 630                 635
<210> 16
   <211> 636
   <212> PRT
   <213> Lactobacillus plantarum WCFS1
<400> 16
   Met Ala Lys Lys Asn Ile Val Val Val Gly Ala Gly Phe Ala Gly Val
   1 5 10 15
   Tyr Ala Thr Lys Lys Leu Ser Lys His Phe Lys Lys Asn Ala Asp Val
   20 25 30
   Glu Ile Thr Leu Ile Asp Arg His Ser Tyr Phe Thr Tyr Met Thr Glu
   35 40 45
   Leu His Glu Val Ala Thr Glu Arg Val Glu Pro Glu His Ile Gln Tyr
   50 55 60
   Asp Leu Gln Arg Leu Phe Ala Arg Arg Lys Asn Val Arg Leu Val Thr
   65 70 75 80
   Asp Thr Val Thr Gly Ile Asp Lys Lys Ala Gln Thr Val Thr Thr Glu
   85 90 95
   His Gly Ser Tyr Gln Tyr Asp Gln Leu Leu Ile Ser Leu Gly Gly Glu
   100 105 110
   Ser Asn Asp Phe Gly Thr Pro Gly Val Lys Glu His Gly Phe Glu Leu
   115 120 125
   Trp Ser Phe Glu Gln Ala Met Ala Leu Arg Ala His Leu Ser Ala Ile
   130 135 140
   Ile Arg Arg Gly Ala Ala Glu Leu Asp Pro Ala Lys Arg Lys Ala Met
   145 150 155 160
   Leu Thr Phe Thr Val Cys Gly Ser Gly Phe Thr Gly Ser Glu Leu Ile
   165 170 175
   Gly Glu Leu Ile Glu Tyr Arg Asp Val Leu Ala Arg Asp Asn Lys Leu
   180 185 190
   Asp Pro Ser Glu Ile Thr Leu Gln Leu Val Glu Ala Ala Pro Thr Ile
   195 200 205
   Ile Asn Met Leu Asn Arg Thr Gln Ala Gly Lys Ala Ala Lys Tyr Met
   210 215 220
   Glu Lys His Gly Val Lys Ile Met Thr Asn Ser Met Ile Thr Glu Val
   225 230 235 240
   Cys Glu Asp His Val Asn Leu Lys Gly Lys Asp Pro Ile Pro Thr Tyr
   245 250 255
   Thr Leu Ile Trp Thr Ala Gly Val Arg Ala Asn Ser Ile Val Lys Lys
   260 265 270
   Phe Gly Ile Glu Thr Asn Pro Arg Gly Gly Arg Leu Met Ala Asn Glu
   275 280 285
   Phe Met Gln Ala Lys Asp Cys Asn Asn Ile Phe Leu Ala Gly Asp Ser
   290 295 300
   Thr Ser Tyr Gln Glu Pro Asp Gln Pro Arg Pro Val Pro Gln Ile Val
   305 310 315 320
   Gln Gly Ala Glu Glu Thr Ala Ala Lys Ala Val Glu Gly Ile Ile Lys
   325 330 335
   Asn Val Asp Gln Thr Asp Val Thr Ile Lys Pro Phe Lys Gly Ala Tyr
   340 345 350
   Gln Ala Ser Val Asp Ser Ile Gly Ser Lys Tyr Ala Val Ala Gln Val
   355 360 365
   Leu Glu Lys Trp Asn Val Ser Gly Phe Ile Ala Val Leu Leu Lys His
   370 375 380
   Ala Ile Asn Trp Met Tyr Tyr Val Gln Ile Phe Ser Gly Tyr Tyr Leu
   385 390 395 400
   Phe Gln Tyr Phe Met His Glu Phe Phe Arg Thr Arg Asn Asn Arg Asn
   405 410 415
   Val Phe Arg Gly Trp Val Ser Arg Ala Gly Asn Val Leu Trp Ser Val
   420 425 430
   Pro Leu Arg Phe Phe Tyr Gly Ala Met Trp Leu Trp Asp Cys Trp Thr
   435 440 445
   Lys Val Gln Gly Ser Glu Ser Trp Phe Thr Asp Lys Leu Arg Leu Pro
   450 455 460
   Phe Glu Trp Ile Thr Val Ala Ala Thr Ser Gly Ala Ser Gln Ala Thr
   465 470 475 480
   Lys Ala Ala Ala Thr Ser Gly Ala Ser Glu Ala Ala Thr Ser Thr Val
   485 490 495
   Lys Ala Ala Lys Gly Val Phe Ser Leu Ser Tyr Met Tyr Gly Lys Glu
   500 505 510
   Pro Leu Met Val Phe Asp Lys Met Pro His Trp Phe Glu Ser Ile Thr
   515 520 525
   Lys Val Phe Ile Pro Asn Met Gln Met Ala Leu Phe Phe Gln Lys Phe
   530 535 540
   Met Thr Cys Val Glu Ile Val Ile Ala Leu Cys Ile Phe Phe Gly Leu
   545 550 555 560
   Phe Thr Trp Phe Ala Asn Ala Val Thr Ile Gly Leu Val Val Val Phe
   565 570 575
   Cys Leu Ser Gly Met Phe Tyr Trp Val Asn Ile Trp Met Ile Phe Val
   580 585 590
   Ala Leu Ala Leu Met Asn Gly Ser Gly Arg Thr Phe Gly Leu Asp Tyr
   595 600 605
   Trp Val Val Pro Trp Met Gln Lys His Leu Gly His Trp Trp Tyr Gly
   610 615 620
   Asn Val Arg Ser His Tyr Asp Gly Val Lys Thr Arg
   625 630 635